# EUROPEAN PATENT APPLICATION

(11) **EP 1 020 434 A1**
(43) Date of publication of application: **19.07.2000**
(21) Application number: 98938906.9
(22) Date of filing: 20.08.1998
(51) Int. Cl.: C07C 257/18, C07C 271/44, C07C 255/58, C07C 311/08, C07C 307/10, C07D 211/26, C07D 233/64, C07D 211/46, C07D 401/12, A61K 31/165, A61K 31/445, A61K 31/415, A61K 31/505, A61K 31/245

(54) **3-AMIDINOANILINE DERIVATIVES, ACTIVATED BLOOD COAGULATION FACTOR X INHIBITORS, AND INTERMEDIATES FOR PRODUCING BOTH**

(30) Priority: 27.08.1997 JP 27185397
(71) Applicant: Kissei Pharmaceutical Co., Ltd., Matsumoto-shi Nagano 399-8710 (JP)
(72) Inventor: AKAHANE, Satoshi, Matsumoto-shi, Nagano 399-0033 (JP); UCHIDA, Masahiko, Minamiazumi-gun, Nagano 399-8302 (JP); ISAWA, Hideotoshi, Kopo Hanamizuki 201, Minamiazumi-gun, Nagano 399-8205 (JP); KIKUCHI, Norihiko, Matsumoto-shi, Nagano 390-1242 (JP); OZAWA, Tomonaga, Matsumoto-shi, Nagano 399-0005 (JP); KOBAYASHI, Hiroaki, Matsumoto-shi, Nagano 399-0011 (JP); KAI, Yuichiro, Kissei Daisanseiyu-ryo, Minamiazumi-gun, Nagano 399-8304 (JP); AKAHANE, Kenji, Minamiazumi-gun, Nagano 399-8205 (JP)
(74) Representative: Baverstock, Michael George Douglas
(86) International application number: JP9803685
(87) International publication number: WO9910316

(57) **Abstract**

The present invention relates to a 3-amidinoaniline derivative represented by the general formula: 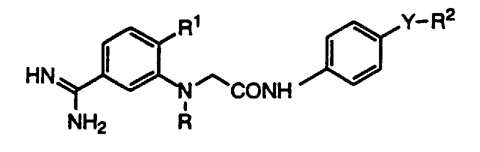 wherein R represents a hydrogen atom, a lower alkyl group, a lower alkenyl group etc.; R¹ represents a hydrogen atom, a hydroxy group, a lower alkyl group etc.; Y represents a single bond or an oxygen atom; and R² represents a lower alkyl group or a group represented by the general formula: 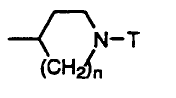 wherein n represents 1 or 2; and T represents a hydrogen atom or a group represented by the general formula:

-C(=NH)-W

wherein W represents a lower alkyl group; or a pharmaceutically acceptable salt thereof, which has a potent and selective activated blood coagulation factor X inhibitory activity, and an intermediate for producing both.

## Description

### Technical Field

The present invention relates to novel 3-amidinoaniline derivatives or pharmaceutically acceptable salts thereof which are useful as medicaments.

### Background Art

Anticoagulation therapy has been extensively performed for the prevention and treatment of thromboembolic diseases caused by accelerating blood clotting, and drugs such as heparin and warfarin potassium have been frequently used as anticoagulant agents at present.

However, it is known that heparin is apt to cause a tendency to bleed since it has antithrombin and activated blood coagulation factor X inhibitory activities.

Warfarin potassium is an anticoagulant which controls biosynthesis of vitamin K-dependent coagulation factor, and it is difficult to control the anticoagulation capacity due to its action mechanism when this drug is used in the prevention and treatment of thromboembolic diseases. Therefore, this drug is extremely difficult to use clinically.

In recent years, selective thrombin inhibitors have been developed and have been used clinically. However, since thrombin plays a close part in the conversion of fibrinogen into fibrin in blood coagulation cascade reactions and platelet activation and aggregation, thrombin inhibitors also have similar problems to those of heparin from the safety point of view such as a tendency to bleed and it has been reported that their effects are not necessarily enough.

On the other hand, activated blood coagulation factor X, which acts at the junction of the extrinsic and intrinsic blood coagulation cascade reactions, locates upstream to thrombin, so that coagulation inhibition is more efficient than that of thrombin inhibitors and therefore activated blood coagulation factor X inhibitors attract public attention as drugs having a possibility of inihibiting the coagulation system effectively.

Furthermore, with the changes in European and American life styles and the increase in aged population in recent years, the incidence of thromboembolic diseases such as myocardial infarction and arteriovenous obstruction will continue to increase, and therefore, demands for the development of more effective anticoagulants are great and the social importance of their treatment has been increasing more and more.

### Disclosure of Invention

The present inventors have extensively studied in order to find novel compounds having excellent activated blood coagulation factor X inhibitory activity. As a result, it has been found unexpectedly that certain 3-amidinoaniline derivatives have a potent and selective activated blood coagulation factor X inhibitory activity, thereby forming the basis of the present invention.

Accordingly, the present invention relates to a 3-amidinoaniline derivative represented by the general formula: 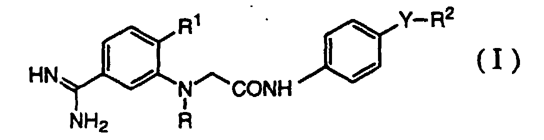 wherein R represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carbamoyl-substituted lower alkoxy group, a carboxy group, a lower alkoxycarbonyl group, a lower alkylsulfonyl group, a sulfamoyl group and a halogen atom, a group represented by the general formula: 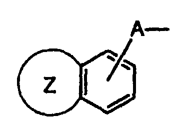 wherein A represents a lower alkylene group; and the ring Z fused with the benzene ring represents a 6-membered aromatic heterocyclic group which contains one or two nitrogen atoms in the ring or a group represented by the general formula: 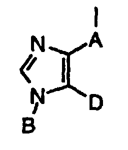 wherein A represents a lower alkylene group; B represents a hydrogen atom, a lower alkyl group, a carboxy-substituted lower alkyl group, a lower alkoxycarbonyl-substituted lower alkyl group, a carbamoyl-substituted lower alkyl group or an aryl-substituted lower alkyl group which may have a hydroxy group or a lower alkyl group as a substituent; and D represents a hydrogen atom or a lower alkyl group ; R¹ represents a hydrogen atom, a hydroxy group, a lower alkyl group, a hydroxy-substituted lower alkyl group, a lower alkoxy group which may have an aryl group as a substituent or a halogen atom; Y represents a single bond or an oxygen atom; and R² represents a lower alkyl group or a group represented by the general formula: 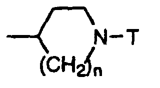 wherein n represents 1 or 2; and T represents a hydrogen atom or a group represented by the general formula:

-C(=NH)-W

wherein W represents a lower alkyl group or a pharmaceutically acceptable salt thereof.

The present invention also relates to a pharmaceutical composition comprising a 3-amidinoaniline derivative represented by the above general formula (I) or a pharmaceutically acceptable salt thereof.

The present invention additionally relates to an activated blood coagulation factor X inhibitor which comprises, as an active ingredient, a 3-amidinoaniline derivative represented by the above general formula (I) or a pharmaceutically acceptable salt thereof.

The present invention also relates to a method for the prevention or treatment of thromboembolic diseases which comprises administering a 3-amidinoaniline derivative represented by the above general formula (I) or a pharmaceutically acceptable salt thereof.

The present invention relates as well to a use of a 3-amidinoaniline derivative represented by the above general formula (I) or a pharmaceutically acceptable salt thereof for the manufacture of a pharmaceutical composition for the prevention or treatment of thromboembolic diseases.

The present invention also relates to a process for the manufacture of a pharmaceutical composition for the prevention or treatment of thromboembolic diseases, characterized in the use, as an essential constituent of said pharmaceutical composition, of a 3-amidinoaniline derivative represented by the above general formula (I) or a pharmaceutically acceptable salt thereof.

The present invention additionally relates to a 3-cyanoaniline derivative represented by the general formula: 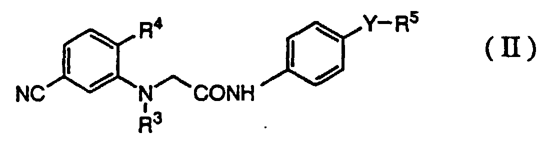 wherein R³ represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group which may have a protective group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxy-carbonyl-substituted lower alkenyl group, a carboxy group, a lower alkoxycarbonyl group, a lower alkylsulfonyl group, a sulfamoyl group and a halogen atom, a group represented by the general formula: 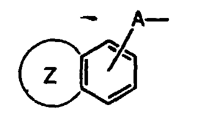 wherein A represents a lower alkylene group; and the ring Z fused with the benzene ring represents a 6-membered aromatic heterocyclic group which contains one or two nitrogen atoms in the ring or a group represented by the general formula: 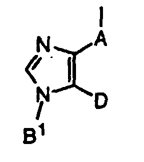 wherein A represents a lower alkylene group; B¹ represents a hydrogen atom, an amino-protective group, a lower alkyl group, a carboxy-substituted lower alkyl group, a lower alkoxy-carbonyl-substituted lower alkyl group or an aryl-substituted lower alkyl group which may have a hydroxy group or a lower alkyl group as a substituent; and D represents a hydrogen atom or a lower alkyl group ; R⁴ represents a hydrogen atom, a lower alkyl group, a protected hydroxy-substituted lower alkyl group, a lower alkoxy group which may have an aryl group as a substituent or a halogen atom; y represents a single bond or an oxygen atom; and R⁵ represents a lower alkyl group or a group represented by the general formula: 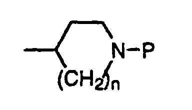 wherein n represents 1 or 2; and P represents a hydrogen atom or an amino-protective group or a salt thereof.

In the compounds represented by the above general formula (I) of the present invention, the term "lower alkyl group" means a straight- or branched-chain alkyl group having 1 to 6 carbon atoms such as a methyl group, an ethyl group, a propyl group, an isopropyl group, a butyl group, an isobutyl group, a sec-butyl group, a pentyl group, an isopentyl group, a neo-pentyl group, a 1-methylbutyl group, a 2-methylbutyl group or a hexyl group with the proviso that, when the substituent R is a hydroxy-substituted lower alkyl group, said hydroxy group does not exist at the α-position, and the term "lower alkenyl group" means a straight- or branched-chain alkenyl group having 2 to 6 carbon atoms such as a vinyl group, an allyl group, a 1-propenyl group, a 1-butenyl group, a 2-butenyl group, a 3-butenyl group, a 2-methylallyl group or a 3-methyl-2-butenyl group with the proviso that the substituent R does not have a double bond at the 1-position.

The term "lower alkoxy group" means a straight- or branched-chain alkoxy group having 1 to 6 carbon atoms such as a methoxy group, an ethoxy group, a propoxy group, an isopropoxy group, a butoxy group, an isobutoxy group, a sec-butoxy group, a tert-butoxy group, a pentyloxy group, an isopentyloxy group, a neo-pentyloxy group, a tert-pentyloxy group or a hexyloxy group, the term "lower alkoxy-substituted lower alkyl group" means the above alkyl group having the above lower alkoxy group at a position except for the α-position, and the term "lower alkoxycarbonyl group" means a straight- or branched-chain alkoxycarbonyl group having 2 to 7 carbon atoms such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a neo-pentyloxycarbonyl group, a tert-pentyloxycarbonyl group or a hexyloxycarbonyl group.

The term "aryl group" means an aromatic hydrocarbon group such as a phenyl group or a naphthyl group, and the term "cycloalkyl group" means a 3 to 7-membered cyclic alkyl group.

The term "halogen atom" includes a fluorine atom, a chlorine atom, a bromine atom and an iodine atom, and the term "lower alkylsulfonyl group" means a straight- or branched-chain alkylsulfonyl group having 1 to 6 carbon atoms such as a methanesulfonyl group or an ethanesulfonyl group.

The term "lower alkylene group" means a straight- or branched-chain alkylene group having 1 to 6 carbon atoms such as a methylene group, an ethylene group, a trimethylene group, or a propylene group.

The term "6-membered aromatic heterocyclic group which is fused with the benzene and contains one or two nitrogen atoms in the ring" means a bicyclic aromatic heterocyclic group such as a 5-quinolyl group, a 6-quinolyl group, a 7-quinolyl group, an 8-quinolyl group, a 5-isoquinolyl group, a 6-isoquinolyl group, a 7-isoquinolyl group, an 8-isoquinolyl group, a 5-phthalazinyl group, a 6-phthalazinyl group, a 5-quinoxalinyl group, a 6-quinoxalinyl group, a 5-quinazolinyl group, a 6-quinazolinyl group, a 7-quinazolinyl group, an 8-quinazolinyl group, a 5-cinnolinyl group, a 6-cinnolinyl group, a 7-cinnolinyl group, or an 8-cinnolinyl group.

The compounds represented by the general formula (I) of the present invention can be prepared, for example, by converting a 3-cyanoaniline derivative represented by the general formula: 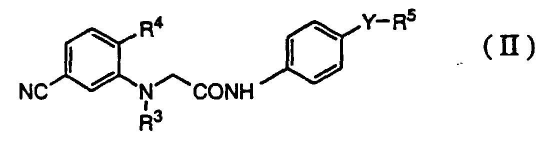 wherein R³ represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group which may have a protective group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carboxy group, a lower alkoxycarbonyl group, a lower alkylsulfonyl group, a sulfamoyl group and a halogen atom, a group represented by the general formula: 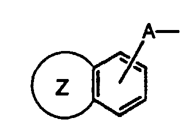 wherein A and the ring Z fused with the benzene ring have the same meanings as mentioned above or a group represented by the general formula: 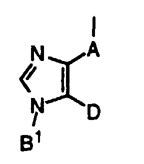 wherein B¹ represents a hydrogen atom, an amino-protective group, a lower alkyl group, a carboxy-substituted lower alkyl group, a lower alkoxycarbonyl-substituted lower alkyl group or an aryl-substituted lower alkyl group which may have a hydroxy group or a lower alkyl group as a substituent; and A and D have the same meanings as mentioned above ; R⁴ represents a hydrogen atom, a lower alkyl group, a protected hydroxy-substituted lower alkyl group, a lower alkoxy group which may have an aryl group as a substituent or a halogen atom; R⁵ represents a lower alkyl group or a group represented by the general formula: 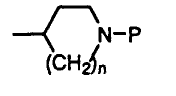 wherein P and n have the same meanings as mentioned above ; and Y has the same meaning as mentioned above into a 3-amidinoaniline derivative represented by the general formula: 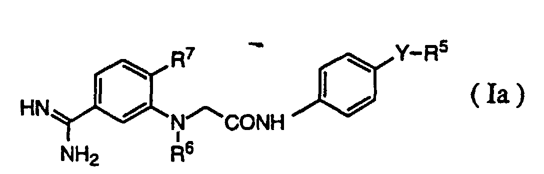 wherein R⁶ represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carbamoyl-substituted lower alkoxy group, a carboxy group, a lower alkoxycarbonyl group, a lower alkylsulfonyl group, a sulfamoyl group and a halogen atom, a group represented by the general formula: 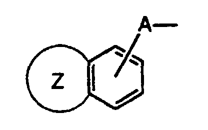 wherein A and the ring Z fused with the benzene ring have the same meanings as mentioned above or a group represented by the general formula: 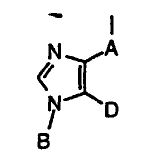 wherein A, B and D have the same meanings as mentioned above ; R⁷ represents a hydrogen atom, a lower alkyl group, a hydroxy-substituted lower alkyl group, a lower alkoxy group which may have an aryl group as a substituent or a halogen atom; and R⁵ and Y have the same meanings as mentioned above, if necessary, removing the amino-protective group, if desired, allowing the resulting compound to react with an alkyliminoether compound represented by the general formula: 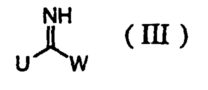 wherein U represents a lower alkoxy group; and W has the same meaning as mentioned above to prepare a compound represented by the general formula: 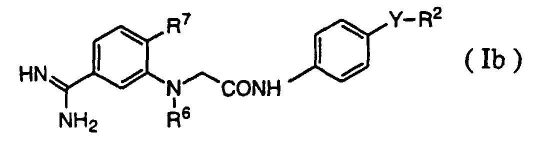 wherein R², R⁶, R⁷ and Y have the same meanings as mentioned above , and, if desired, subjecting suitably the resulting compound to debenzylation by hydrogenolysis, reduction of the double bond, hydrolysis of the ester group or esterification in the usual way.

The compounds having a functional group such as a hydroxy group, an amino group, a carboxyl group and the like can be allowed to react after introducing an appropriate protective group as occasion demands. As the protective group, the groups commonly used for the protection of said functional groups can be used with no particular limitation, and as examples of the protective groups, a carbamate group such as tert-butoxycarbonyl and benzyloxycarbonyl, an alkyl group such as benzyl, methyl and tert-butyl, a silyl group such as trimethylsilyl and tert-butyldimethylsilyl, an acetal group such as methoxymethyl and tetrahydropyranyl, and an acyl group such as acetyl, benzoyl and trifluoroacetyl are illustrative.

The reactions from a 3-cyanoaniline derivative represented by the above general formula (II) to an amidino derivative represented by the general formula (Ia) in the aforementioned production process are shown as the following scheme 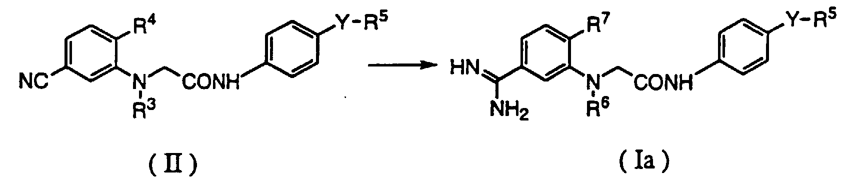 wherein R³, R⁴, R⁵, R⁶, R⁷ and Y have the same meanings as mentioned above.

### Method 1

A 3-cyanoaniline derivative represented by the above general formula (II) is allowed to react with an alcohol such as methanol or ethanol in the presence of a hydrogen halide such as hydrogen chloride or hydrogen bromide usually at from -20°C to room temperature, and then the resulting imidate compound is allowed to react with ammonia or an ammonium salt such as ammonium carbonate, ammonium chloride or ammonium acetate to obtain a 3-amidinoaniline derivative represented by the above general formula (Ia). As the solvent used, methanol, ethanol, tetrahydrofuran or dichloromethane are illustrative.

### Method 2

A 3-cyanoaniline derivative represented by the above general formula (II) is allowed to react with hydrogen sulfide in the presence of an organic base such as triethylamine or pyridine usually at from -20 °C to room temperature, the resulting thioamide compound is allowed to react with a lower alkyl halide compound such as methyl iodide or ethyl iodide to prepare a thioimidate compound, and then the resulting compound is allowed to react with ammonia or an ammonium salt such as ammonium carbonate, ammonium chloride or ammonium acetate to obtain a 3-amidinoaniline derivative represented by the above general formula (Ia). As the solvent used, methanol, ethanol, tetrahydrofuran or dichloromethane are illustrative.

When the amino-protective group is not eliminated during the aforementioned reactions, the protective group can be easily removed by treatment in the usual way.

When converting a 3-cyanoaniline derivative represented by the above general formula (II) into a 3-amidinoaniline derivative represented by the above general formula (Ia), the carboxyl group may be esterified by the alcohol used, and the lower alkoxycarbonyl group may be subjected to ester interchange with the alcohol used.

When the imidate compound or thioimidate compound is treated with ammonia, the lower alkoxycarbonyl group may be converted into a carbamoyl group in addition to the conversion into an amidino group.

The reaction of a cyclic amine compound represented by the general formula (Ia') with an alkyliminoether compound represented by the above general formula (III) in the aforementioned production process is shown as the following scheme 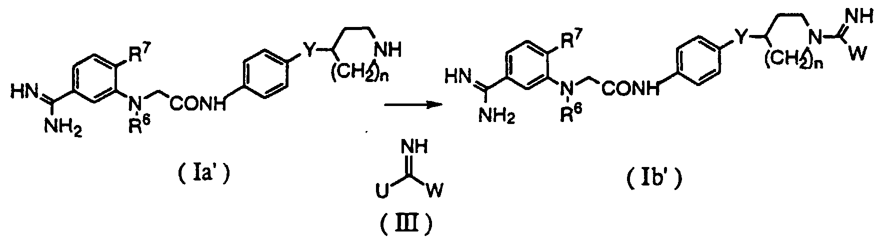 wherein R⁶, R⁷, Y, W and n have the same meanings as mentioned above.

A compound represented by the above general formula (Ia') is allowed to react with an alkyliminoether represented by the above general formula (III) in the presence of a base such as triethylamine or N-methylmorpholine usually at from -20 °C to 50 °C to obtain a compound represented by the above general formula (Ib'). As the solvent used, ethanol, methanol, dichloromethane, or N,N-dimethylformamide are illustrative.

For example, debenzylation by hydrogenolysis and reduction of the double bond can be carried out using a palladium catalyst such as palladium on carbon in a protic solvent such as an alcohol or water, an aprotic solvent such as tetrahydrofuran or ethyl acetate, or a mixture thereof in the presence of an acid catalyst such as hydrochloric acid as occasion demands, usually at from 0°C to 50°C under a hydrogen atmosphere and at atmospheric pressure.

For example, hydrolysis of the ester group can be carried out by treating the corresponding ester derivative in the presence of an acid such as hydrochloric acid, sulfuric acid or p-toluenesulfonic acid in a solvent containing water, usually at from 0 °C to 50 °C, or by subjecting the corresponding ester derivative not having an alkanimidoyl group to alkali hydrolysis using for example sodium hydroxide. Esterification or ester interchange can be carried out by allowing the corresponding carboxylic acid derivative or ester derivative to react with a desired alcohol in the presence of an acid such as hydrochloric acid, sulfuric acid or p-toluenesulfonic acid.

The nitrile compounds represented by the above general formula (II) which are used as starting materials in the aforementioned production process can be prepared, for example, by the following steps A-B. 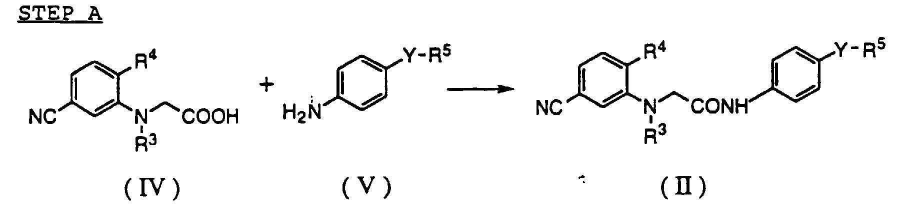 wherein R³,R⁴,R⁵ and Y have the same meanings as mentioned above.

### Step A

A carboxylic acid derivative represented by the above general formula (IV) is condensed with an aniline derivative represented by the above general formula (V) using a condensing agent such as dicyclohexylcarbodiimide, 1-ethyl-(3-dimethylaminopropyl)carbodiimide hydrochloride or diethyl cyanophosphate, if necessary in the presence of an agent for making an activated ester such as 1-hydroxybenzotriazole in an aprotic solvent such as acetonitrile or N,N-dimethylformamide, usually at from 0 °C to room temperature and, if desired, removal of the protective group to obtain a 3-cyanoaniline compound represented by the above general formula (II). 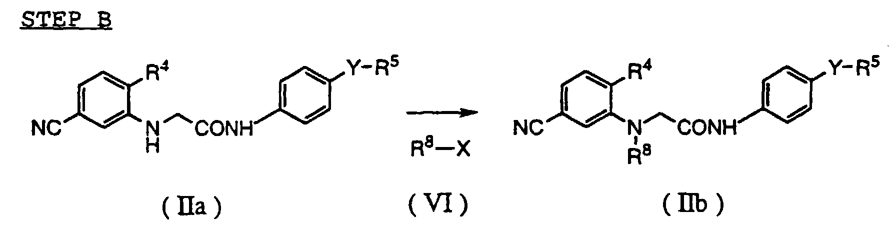 wherein R⁸ represents a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group which may have a protective group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carboxy group, a lower alkoxycarbonyl group, a lower alkylsulfonyl group, a sulfamoyl group and a halogen atom, a group represented by the general formula: 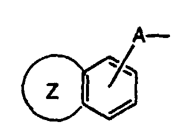 wherein A and the ring Z fused with the benzene ring have the same meanings as mentioned above or a group represented by the general formula: 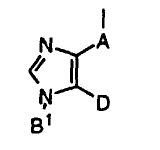 wherein A, B¹ and D have the same meanings as mentioned above ; X represents a halogen atom; and R⁴, R⁵ and Y have the same meanings as mentioned above,

### Step B

A 3-cyanoaniline derivative represented by the above general formula (IIa) obtained by the aforementioned Step A is allowed to react with a halogeno compound represented by the above general formula (VI) in the presence of an organic base such as N-ethyldiisopropylamine or triethylamine, or an inorganic base such as potassium carbonate in a polar solvent such as an alcohol e.g., 2-butanol; N,N-dimethylformamide or dimethylsulfoxide, or a mixture thereof usually at from 0 °C to 100 °C, and, if desired, removed the protective group to obtain a 3-cyanoaniline compound represented by the above general formula (IIb).

In the aforementioned method for preparing 3-cyanoaniline derivatives represented by the above general formulae (II) and (IIb), reactions such as deprotection including debenzylation by hydrogenolysis, reduction of the double bond, N, O or C-alkylation can be carried out by reacting suitably in the usual way.

The carboxylic acid derivatives represented by the above general formula (IV) which are used in the aforementioned Step A can be prepared, for example, by the following steps. 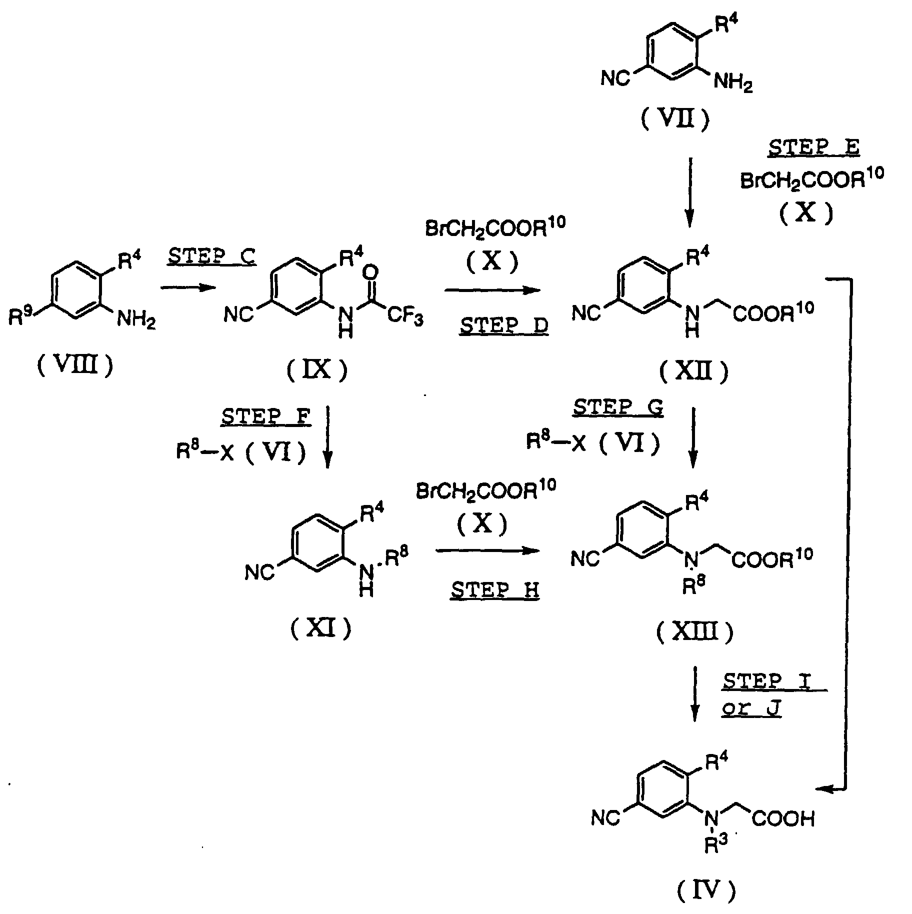 wherein R⁹ represents a cyano group or a carbamoyl group; R¹⁰ represents a lower alkyl group or a benzyl group; and R³ ,R⁴, R⁸ and X have the same meanings as mentioned above

### Step C

An aniline derivative represented by the above general formula (VIII) is allowed to react with trifluoroacetic anhydride in the presence of an organic base such as triethylamine or pyridine in an inert solvent such as dichloromethane, a mixture thereof, or without solvent usually at from 0 °C to room temperature.

### Step D

An amide derivative represented by the above general formula (IX) is allowed to react with a bromoacetic acid derivative represented by the above general formula (X) in the presence of a base such as sodium hydride or potassium tert-butoxide in a polar solvent such as N,N-dimethylformamide or tetrahydrofuran, or a mixture thereof, usually at from room temperature to 100 °C.

### Step E

An aniline derivative represented by the above general formula (VII) is allowed to react with a bromoacetic acid derivative represented by the above general formula (X) in the presence of a base such as potassium carbonate in a solvent such as N,N-dimethylformamide or dimethylsulfoxide, or a mixture thereof, usually at from room temperature to 100 °C after adding an activating agent such as sodium iodide as appropriate.

### Step F

An amide derivative represented by the above general formula (IX) is allowed to react with a halogeno compound represented by the above general formula (VI) in the presence of a base such as potassium hydroxide in a polar solvent such as acetone, or a mixture thereof usually at from room temperature to 80 °C.

### Step G

A glycine ester derivative represented by the above general formula (XII) is allowed to react with a halogeno compound represented by the above general formula (VI) in the presence of an organic base such as N-ethyldiisopropylamine or triethylamine in a polar solvent such as an alcohol e.g., ethanol, 2-butanol; or N,N-dimethylformamide, or a mixture thereof, usually at from room temperature to 100 °C, or in the presence of an inorganic base such as sodium hydride in an aprotic and polar solvent such as N,N-dimethylformamide usually at from 0 °C to 100 °C.

### Step H

A compound represented by the above general formula (XI) is allowed to react with a bromoacetic acid derivative represented by the above general formula (X) in the presence of a base such as potassium carbonate in a polar solvent such as dimethylsulfoxide or N,N-dimethylformamide, or a mixture thereof usually at from room temperature to 100 °C.

### Step I

A glycine ester derivative represented by the above general formula (XII) or (XIII) is treated with alkali hydroxide such as sodium hydroxide or potassium hydroxide in an alcohol, water or an alcohol containing water usually at from 0 °C to 50 °C.

### Step J

A glycine benzyl ester derivative represented by the above general formula (XII) or (XIII) is allowed to react in the presence of a palladium catalyst such as palladium on carbon in a protic solvent such as an alcohol or water, an aprotic solvent such as tetrahydrofuran or ethyl acetate, or a mixture thereof usually at from room temperature to 50 °C under a hydrogen atmosphere at atmospheric pressure after adding an acid catalyst such as, suitably hydrochloric acid, as appropriate.

The compounds of the present invention obtained by the aforementioned production process can be easily isolated and purified by conventional separation means such as fractional recrystallization, precipitation, purification using column chromatography and solvent extraction.

The 3-amidinoaniline derivatives represented by the above general formula (I) of the present invention can be converted into their pharmaceutically acceptable salts in the usual way. Examples of the such salts include acid addition salts with mineral acids (e.g., hydrochloric acid, hydrobromic acid, hydroiodic acid, sulfuric acid, nitric acid and phosphoric acid) acid addition salts with organic acids (e.g., formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid and aspartic acid, salts with inorganic bases such as a sodium salt and a potassium salt, and salts with organic amines (e.g., morpholine, piperidine or lysine).

In addition, the compounds represented by the above general formula (I) of the present invention also include its hydrates and solvates with pharmaceutically acceptable solvents, e.g. ethanol.

Of the compounds represented by the above general formula (I) of the present invention, the compounds having an unsaturated bond exist in two geometrical isomer forms. Either the cis (Z) isomer or trans (E) isomer can be employed in the present invention.

Of the compounds represented by the above general formula (I) of the present invention, compounds having an asymmetric carbon atom exist in the two optical isomer forms (*R*) configuration and (*S*) configuration. Either of the isomers or a mixture thereof can be employed in the present invention.

Of the compounds represented by the above general formula (I) of the present invention, compounds wherein tautomers exist can be employed as either one of the tautomers or a mixture thereof in the present invention.

The compounds represented by the above general formula (I) of the present invention are compounds having a potent inhibitory activity on activated blood coagulation factor X and anti-coagulation activity. The compounds represented by the above general formula (I) of the present invention also have an extremely weak inhibitory activity on thrombin and therefore are highly selective activated blood coagulation factor X inhibitors.

The compounds represented by the above general formula (I) of the present invention are selective activated blood coagulation factor X inhibitors. In consequence, the compounds of the present compounds are extremely useful as agents for the prevention or treatment of cerebral infarction, cerebral thrombosis, cerebral embolism, transient cerebral ischemic attack (TIA), subarachnoid hemorrhage, myocardial infarction, unstable angina, pulmonary thrombosis, pulmonary embolism, Berger disease, peripheral arterial obstruction, deep venous thrombosis, disseminated intravascular coagulation syndrome, thrombus formation after artificial blood vessel operation or after artificial valve replacement, restenosis and reocculusion after coronary intervention such as percutaneous transluminal coronary angioplasty (PTCA) or percutaneous transluminal coronary recanalization (PTCR) surgery, and thrombus formation at the time of extracorporeal circulation, agents for the prevention of blood coagulation at the time of insertion of a blood vessel catheter, and agents for the prevention or treatment of influenza virus infection based on their activity in inhibiting growth of the influenza virus.

The compounds represented by the above general formula (I) of the present invention are very safe compounds. For example, in acute toxicity test using rats, no death was observed after an intravenous administration of 2-[N-(3-amidinophenyl)-N-(3-carboxymethyloxybenzyl)amino]-N-[4-[1-(1-iminoethyl)piperidin-4-yl]phenyl]acetamide dihydrochloride at a dose of 30 mg/kg.

When the 3-amidinoaniline derivatives represented by the above general formula (I) of the present invention and pharmaceutically acceptable salts thereof are employed in practical treatment, they are administered orally or parenterally in the form of appropriate pharmaceutical compositions such as tablets, powders, fine granules, granules, capsules, injections, solutions, adhesive preparations, ointments or suppositories. These pharmaceutical compositions can be formulated in accordance with conventional methods using conventional pharmaceutical carriers, excipients and other additives.

The dosage is appropriately decided depending on the sex, age, body weight, and extent of symptoms of each patient to be treated, which is approximately within the range of from 1 to 5,000 mg per day per adult human in the case of oral administration and approximately within the range of from 0.01 to 500 mg per day per adult human in the case of parenteral administration, and the daily dose can be divided into more than one dose per day.

### Industrial Applicability

The compounds of the present invention have a potent and selective inhibitory activity on activated blood coagulation factor X, and are therefore extremely suitable for the prevention or treatment of thromboembolic diseases.

### Best Mode for Carrying Out the Invention

The present invention is further illustrated in more detail by way of the following Reference Examples, Examples and Test Examples. The present invention is not limited thereto.

### Reference Example 1

### 1-tert-Butoxycarbonyl-4-hydroxypiperidine

4-Hydroxypiperidine (10.0 g) was dissolved in 100 ml of 2 N aqueous sodium hydroxide solution, 27.3 ml of di-tert-butyl dicarbonate was added to the solution, and the mixture was stirred for 6 hours. Water was added to the reaction mixture, and the mixture was extracted with diethyl ether. The organic layer was washed successively with 10 % aqueous citric acid solution and brine, and dried over anhydrous magnesium sulfate. The solvent was removed under reduced pressure to give 19.1 g of 1-tert-butoxycarbonyl-4-hydroxypiperidine.
¹H-NMR(CDCl₃) δ ppm:
   1.35-1.70 (12H, m), 1.80-1.90 (2H, m), 2.95-3.10 (2H, m), 3.75-3.95 (3H, m)

### Reference Example 2

### 4-(1-tert-Butoxycarbonylpiperidin-4-yloxy)nitrobenzene

Sodium hydride (4.5 g, 60 % in oil) was suspended in dry hexane, and the supernatant was decanted. A solution of 31.14 g of 1-tert-butoxycarbonyl-4-hydroxyperidine in 500 ml of dimethylsulfoxide was added to the resulting residue under ice-cooling, and the mixture was stirred under an argon atmosphere at room temperature for 30 minutes. To the reaction mixture was added 24.41 g of 4-chloronitrobenzene portionwise, and the mixture was stirred at 60 °C for 4 hours. Water was added to the reaction mixture, and the resulting precipitates were collected by filtration and washed with hexane and diethyl ether to give 30.70 g of 4-(1-tert-butoxycarbonylpiperidin-4-yloxy)nitrobenzene.
¹H-NMR(CDCl₃) δ ppm:
   1.46 (9H, s), 1.72-1.86 (2H, m), 1.90-2.03 (2H, m), 3.32-3.45 (2H, m), 3.63-3.76 (2H, m), 4.55-4.65 (1H, m), 6.90-7.00 (2H, m), 8.13-8.24 (2H, m)

### Reference Example 3

### 4-(1-tert-Butoxycarbonylpiperidin-4-yloxy)aniline

4-(1-tert-Butoxycarbonylpiperidin-4-yloxy)nitrobenzene (11.87 g) was dissolved in a mixture of 150 ml of ethanol and 150 ml of tetrahydrofuran, and 780 mg of 10 % palladium on carbon was added to the solution. The mixture was stirred at atmospheric pressure under a hydrogen atmosphere at room temperature for 5 hours. The insoluble material was removed by filtration and the filtrate was concentrated under reduced pressure to give 9.75 g of 4-(1-tert-butoxycarbonylpiperidin-4-yloxy)aniline.
¹H-NMR(CDCl₃) δ ppm:
   1.46 (9H, s), 1.60-1.77 (2H, m), 1.80-1.95 (2H, m), 3.20-3.80 (6H, m), 4.21-4.30 (1H, m), 6.58-6.67 (2H, m), 6.72-6.80 (2H, m)

### Reference Example 4

### 4-Phenylpiperidine hydrochloride

1,2,5,6-Tetrahydro-4-phenylpyridine hydrochloride (125 g) and 12.0 g of 10 % palladium on carbon were suspended in 1 L of methanol, and the suspension was stirred at atmospheric pressure under a hydrogen atmosphere at room temperature for 5 hours. The insoluble material was removed through Celite®, and the filtrate was concentrated under reduced pressure. The resulting residue was suspended in ethyl acetate and a small amount of ethanol, and the insoluble material was collected by filtration to give 60.3 g of 4-phenylpiperidine hydrochloride.
¹H-NMR(DMSO-d₆) δ ppm:
   1.79-2.01 (4H, m), 2.73-3.08 (3H, m), 3.20-3.45 (2H, m), 7.10-7.40 (5H, m), 9.18 (2H, br-s)

### Reference Example 5

### 4-Phenyl-1-trifluoroacetylpiperidine

To 50 ml of dichloromethane were added 10.0 g of 4-phenylpiperidine hydrochloride and 25 ml of triethylamine, and 8.5 ml of trifluoroacetic anhydride was added dropwise to the mixture under ice-cooling. After stirring at room temperature for 2 hours, the reaction mixture was concentrated under reduced pressure. The resulting residue was suspended in diethyl ether, the insoluble material was removed through Celite®, and the filtrate was washed successively with water, dilute hydrochloric acid, a saturated aqueous sodium hydrogen carbonate solution and brine. The organic layer was dried over anhydrous magnesium sulfate and concentrated under reduced pressure to give 12.45 g of 4-phenyl-1-trifluoroacetylpiperidine.
¹H-NMR(CDCl₃) δ ppm:
   1.63-1.80 (2H, m), 1.90-2.07 (2H, m), 2.74-2.93 (2H, m), 3.17-3.32 (1H, m), 4.06-4.21 (1H, m), 4.62-4.78 (1H, m), 7.11-7.40 (5H, m)

### Reference Example 6

### 4-(4-Nitrophenyl)-1-trifluoroacetylpiperidine

4-Phenyl-1-trifluoroacetylpiperidine (123.7 g) was dissolved in 600 ml of trifluoroacetic acid, and 82.0 g of sodium nitrate was added to the solution under ice-cooling. The mixture was stirred at room temperature for 14 hours, and the insoluble material was removed through Celite®, and the filtrate was concentrated under reduced pressure. Ethyl acetate was added to the residue, and the mixture was washed successively with water, a saturated aqueous sodium hydrogen carbonate solution and brine. The organic layer was dried over anhydrous magnesium sulfate, and the solvent was removed under reduced pressure. The resulting residue was suspended in diethyl ether, and the insoluble material was collected by filtration to give 90.4 g of 4-(4-nitrophenyl)-1-trifluoroacetylpiperidine.
¹H-NMR(CDCl₃) δ ppm:
   1.66-1.82 (2H, m), 1.95-2.10 (2H, m), 2.81-3.04 (2H, m), 3.20-3.34 (1H, m), 4.12-4.25 (1H, m), 4.68-4.80 (1H, m), 7.38 (2H, d, J=8.7Hz), 8.20 (2H, d, J=8.7Hz)

### Reference Example 7

### 4-(4-Aminophenyl)-1-trifluoroacetylpiperidine

4-(4-Nitrophenyl)-1-trifluoroacetylpiperidine (70.5 g), 10.0 g of 10 % palladium on carbon and 100 ml of 2 N hydrochloric acid were suspended in 50 ml of methanol, and the suspension was allowed to react at atmospheric pressure under a hydrogen atmosphere at room temperature for 8 hours. The insoluble material was removed through Celite®, and the filtrate was concentrated under reduced pressure. The resulting residue was dissolved in water, the solution was well alkalized with a saturated aqueous sodium hydrogen carbonate solution, and the precipitates were collected by filtration. The obtained material was dissolved in dichloromethane, and the solution was washed with brine and dried over anhydrous magnesium sulfate. The solvent was removed under reduced pressure to give 60.7 g of 4-(4-aminophenyl)-1-trifluoroacetylpiperidine.
¹H-NMR(CDCl₃) δ ppm:
   1.50-1.75 (2H, m), 1.87-2.00 (2H, m), 2.62-2.76 (1H, m), 2.77-2.91 (1H, m), 3.15-3.28 (1H, m), 3.61 (2H, br-s), 4.05-4.18 (1H, m), 4.61-4.74 (1H, m), 6.65 (2H, d, J=8.4Hz), 6.98 (2H, d, J=8.4Hz)

### Reference Example 8

### Ethyl 3-sulfobenzoate

Sodium 3-sulfobenzoate (40.0 g) was suspended in 400 ml of ethanol, 40 ml of a saturated hydrogen chloride ethanol solution was added to the suspension, and the mixture was heated under reflux for 18 hours. The reaction mixture was filtered, and the filtrate was concentrated under reduced pressure. Dichloromethane was added to the resulting residue, and the insoluble material was removed by filtration. The filtrate was concentrated under reduced pressure to give 40.9 g of ethyl 3-sulfobenzoate.
¹H-NMR(DMSO-d₆) δ ppm:
   1.33 (3H, t, J=7.1Hz), 4.32 (2H, q, J=7.1Hz), 7.43-7.55 (1H, m), 7.60-8.25 (4H, m)

### Reference Example 9

### 3-Ethoxycarbonylbenzenesulfonyl chloride

Ethyl 3-sulfobenzoate (40.9 g) was dissolved in 200 ml of thionyl chloride, and 1.0 ml of pyridine and 0.2 ml of *N,N-*dimethylformamide were added to the solution, and the mixture was heated under reflux for 9 hours. The reaction mixture was concentrated under reduced pressure. Diethyl ether was added to the resulting residue, and the mixture was washed successively with water, a saturated aqueous sodium hydrogen carbonate solution and brine. The organic layer was dried over anhydrous magnesium sulfate, and the solvent was removed under reduced pressure to give 42.7 g of 3-ethoxycarbonylbenzenesulfonyl chloride.
¹H-NMR(CDCl₃) δ ppm:
   1.44 (3H, t, J=7.1Hz), 4.46 (2H, q, J=7.1Hz), 7.69-7.79 (1H, m), 8.18-8.28 (1H, m), 8.38-8.48 (1H, m), 8.66-8.75 (1H, m)

### Reference Example 10

### Ethyl 3-sulfamoylbenzoate

3-Ethoxycarbonylbenzenesulfonyl chloride (10.0 g) was dissolved in 20 ml of diethyl ether, 50 ml of 28 % aqueous ammonia solution was added to the solution under ice-cooling, and the mixture was stirred at room temperature for 30 minutes. The precipitates were collected by filtration and washed with diethyl ether to give 7.87 g of ethyl 3-sulfamoylbenzoate.
¹H-NMR(DMSO-d₆) δ ppm:
   1.34 (3H, t, J=7.1Hz), 4.37 (2H, q, J=7.1Hz), 7.39 (2H, br), 7.79-7.89 (1H, m), 8.03-8.12 (1H, m), 8.13-8.20 (1H, m), 8.36-8.42 (1H, m)

### Reference Example 11

### 3-Ethoxycarbonylbenzenesulfinic acid

Zinc powder (4.0 g) was suspended in 30 ml of hot water (70 °C), 6.5 g of 3-ethoxycarbonylbenzenesulfonyl chloride was added dropwise to the suspension. After the generation of heat ceased, the reaction mixture was stirred at room temperature for 30 minutes. To the reaction mixture was added 15 ml of 2 N aqueous sodium hydroxide solution, and sodium carbonate was added to the mixture until it was well alkalized. The insoluble material was removed through Celite®, and the filtrate was acidified with concentrated hydrochloric acid and extracted with ethyl acetate. The organic layer was washed with brine and dried over anhydrous magnesium sulfate. The solvent was removed under reduced pressure to give 4.9 g of 3-ethoxycarbonylbenzenesulfinic acid.
¹H-NMR(CDCl₃) δ ppm:
   1.41 (3H, t, J=7.1Hz), 4.41 (2H, q, J=7.1Hz), 7.55-7.66 (1H, m), 7.76 (2H, br-s), 7.87-7.96 (1H, m), 8.17-8.25 (1H, m), 8.31-8.38 (1H, m)

### Reference Example 12

### Ethyl 3-methanesulfonylbenzoate

3-Ethoxycarbonylbenzensulfinic acid (1.06 g) was dissolved in 5 ml of ethanol, 1.01 ml of 5 N aqueous sodium hydroxide solution and 0.62 ml of iodomethane were added successively to the solution under ice-cooling, and the mixture was stirred at room temperature for 5 hours. Water was added to the reaction mixture, and the mixture was extracted with ethyl acetate. The organic layer was washed successively with a saturated aqueous sodium hydrogen carbonate solution and brine, and dried over anhydrous magnesium sulfate. The solvent was removed under reduced pressure to give 522 mg of ethyl 3-methanesulfonylbenzoate.
¹H-NMR(CDCl₃) δ ppm:
   1.43 (3H, t, J=7.1Hz), 3.10 (3H, s), 4.44 (2H, q, J=7.1Hz), 7.64-7.73 (1H, m), 8.10-8.20 (1H, m), 8.30-8.38 (1H, m), 8.56-8.64 (1H, m)

### Reference Example 13

### 3-Methanesulfonylbenzyl alcohol

Ethyl 3-methanesulfonylbenzoate (420 mg) was dissolved in 4 ml of tetrahydrofuran, 120 mg of lithium borohydride was added to the solution, and the mixture was heated under reflux for 20 hours under an argon atmosphere. Water was added to the reaction mixture, and the mixture was extracted with ethyl acetate. The organic layer was washed successively with a saturated aqueous sodium hydrogen carbonate solution and brine, and dried over anhydrous magnesium sulfate. The solvent was removed under reduced pressure to give 283 mg of 3-methanesulfonylbenzyl alcohol.
¹H-NMR(CDCl₃) δ ppm:
   1.91 (1H, t, J=5.8Hz), 3.07 (3H, s), 4.82 (2H, d, J=5.8Hz), 7.57 (1H, t, J=7.7Hz), 7.67 (1H, d, J=7.7Hz), 7.87 (1H, d, J=7.7Hz), 7.97 (1H, s)

### Reference Example 14

The following compound was prepared using the same procedure as Reference Example 13.

### 3-Sulfamoylbenzyl alcohol

¹H-NMR(DMSO-d₆) δ ppm:
   4.57 (2H, s), 5.30-5.60 (1H, br), 7.25-7.40 (2H, m), 7.45-7.55 (2H, m), 7.64-7.75 (1H, m), 7.77-7.85 (1H, m)

### Reference Example 15

### Methyl 2-(3-formylphenyloxy)acetate

3-Hydroxybenzaldehyde (10.0 g) was dissolved in 50 ml of dimethylsulfoxide, 13.0 g of potassium carbonate was added to the solution, and 8.14 ml of methyl bromoacetate was added dropwise. The mixture was stirred at room temperature for 4 hours, water was added to the reaction mixture, and the mixture was extracted with diethyl ether. The organic layer was washed successively with water, a saturated aqueous sodium hydrogen carbonate solution and brine, and dried over anhydrous magnesium sulfate. The solvent was removed under reduced pressure to give 16.3 g of methyl 2-(3-formylphenyloxy)acetate.
¹H-NMR(CDCl₃) δ ppm:
   3.82 (3H, s), 4.71 (2H, s), 7.20-7.41 (1H, m), 7.35-7.41 (1H, m), 7.44-7.56 (2H, m), 9.97 (1H, s)

### Reference Example 16

The following compounds were prepared using the same procedure as Reference Example 15.

### 3-Methoxymethyloxybenzaldehyde

¹H-NMR(CDCl₃) δ ppm:
   3.49 (3H, s), 5.23 (2H, s), 7.26-7.34 (1H, m), 7.42-7.58 (3H, m), 9.98 (1H, s)

### 4-Benzyloxy-3-nitrobenzonitrile

¹H-NMR(CDCl₃) δ ppm:
   5.32 (2H, s), 7.21 (1H, d, J=8.8Hz), 7.32-7.48 (5H, m), 7.76 (1H, dd, J=8.8Hz, 2.1Hz), 8.15 (1H, d, J=2.1Hz)

### Reference Example 17

### 4-tert-Butyldimethylsilyloxybenzaldehyde

4-Hydroxybenzaldehyde (2.0 g) was dissolved in 20 ml of *N,N*-dimethylformamide, and 2.71 g of tert-butyldimethylsilyl chloride and 1.23 g of imidazole were added to the solution. The mixture was allowed to react under an argon atmosphere at room temperature for 3 hours. Water was added to the reaction mixture, and the mixture was extracted with diethyl ether. The organic layer was washed successively with 1 N aqueous sodium hydroxide solution, water and brine, and dried over anhydrous magnesium sulfate. The solvent was removed under reduced pressure, and the resulting residue was purified by silica gel column chromatography (eluent: ethyl acetate-hexane) to give 3.35 g of 4-tert-butyldimethylsilyloxybenzaldehyde.
¹H-NMR(CDCl₃) δ ppm:
   0.25 (6H, s), 1.00 (9H, s), 6.95 (2H, d, J=8.6Hz), 7.79 (2H, d, J=8.6Hz), 9.89 (1H, s)

### Reference Example 18

### Methyl 2-(3-hydroxymethylphenyloxy)acetate

Methyl 2-(3-formylphenyloxy)acetate 10.0 g was dissolved in 50 ml of tetrahydrofuran, and 1.01 g of sodium borohydride was added to the solution under ice-cooling. To the mixture was added dropwise 10 ml of methanol, and the resulting mixture was stirred under an argon atmosphere at room temperature for 2 hours. Water and 30 ml of 2 N hydrochloric acid were added to the reaction mixture, and the mixture was extracted with diethyl ether. The organic layer was washed with water and brine, and dried over anhydrous magnesium sulfate. The solvent was removed under reduced pressure, and the resulting residue was purified by silica gel column chromatography (eluent: ethyl acetate-hexane) to give 4.37 g of methyl 2-(3-hydroxymethylphenyloxy)acetate.
¹H-NMR(CDCl₃) δ ppm:
   1.69 (1H, t, J=6.1Hz), 3.81 (3H, s), 4.61-4.72 (4H, m), 6.83 (1H, dd, J=8.3Hz, 2.6Hz), 6.92-7.03 (2H, m), 7.23-7.32 (1H, m)

### Reference Example 19

The following compounds were prepared using the same procedure as Reference Example 18.

### 4-tert-Butyldimethylsilyloxybenzyl alcohol

¹H-NMR(CDCl₃) δ ppm:
   0.19 (6H, s), 0.98 (9H, s), 1.54 (1H, t, J=5.8Hz), 4.61(2H, d, J=5.8Hz), 6.83 2H, d, J=8.4Hz), 7.23 (2H, d, J=8.4Hz)

### 3-Methoxymethyloxbenzyl alcohol

¹H-NMR(CDCl₃) δ ppm:
   1.67-1.80 (1H, m), 3.48 (3H, s), 4.67 (2H, d, J=6.0Hz), 5.18 (2H, s), 6.93-7.10 (3H, m), 7.23-7.32 (1H, m)

### Reference Example 20

### 3-Benzyloxybenzyl alcohol

3-Hydroxybenzaldehyde (25.0 g) and 28.3 g of potassium carbonate were added to 150 ml of acetonitrile, then 26.8 ml of benzyl bromide was added dropwise, and the mixture was stirred at 60 °C for 4 hours. Dilute hydrochloric acid was added to the reaction mixture, and the mixture was extracted with diethyl ether. The organic layer was washed successively with a saturated aqueous sodium hydrogen carbonate solution and brine, and dried over anhydrous magnesium sulfate. The solvent was removed under reduced pressure, and the resulting residue was washed with hexane and then dissolved in 150 ml of methanol. To the solution was added portionwise 3.3 g of sodium borohydride under ice-cooling, and the mixture was stirred under an argon atmosphere at room temperature for 10 minutes. The reaction mixture was acidified with dilute hydrochloric acid, and the mixture was extracted with diethyl ether. The organic layer was washed successively with a saturated aqueous sodium hydrogen carbonate solution and brine, and dried over anhydrous magnesium sulfate. The solvent was removed under reduced pressure to give 32.0 g of 3-benzyloxybenzyl alcohol.
¹H-NMR(CDCl₃) δ ppm:
   4.67 (2H, s), 5.08 (2H, s), 6.87-7.05 (3H, m), 7.26-7.45 (6H, m)

### Reference Example 21

### Methyl 2-(3-bromomethylphentloxy) acetate

Methyl 2-(3-hydroxymethylphenyloxy)acetate (4.20 g) and 6.18 g of triphenylphosphine were dissolved in 30 ml of dichloromethane, and 7.81 g of carbon tetrabromide was added portionwise to the solution under ice-cooling. After being allowed to react under an argon atmosphere at room temperature for 15 minutes, the reaction mixture was concentrated under reduced pressure. The resulting residue was purified by silica gel column chromatography (eluent: ethyl acetate-hexane) to give 5.5 g of methyl 2-(3-bromomethylphenyloxy) acetate.
¹H-NMR(CDCl₃) δ ppm:
   3.81 (3H, s), 4.45 (2H, s), 4.65 (2H, s), 6.81-6.88 (1H, m), 6.93-6.98 (1H, m), 7.00-7.06 (1H, m), 7.23-7.30 (1H, m)

### Reference Example 22

The following compounds were prepared using the same procedure as Reference Example 21.

### 3-Methoxymethyloxybenzyl bromide

¹H-NMR(CDCl₃) δ ppm:
   3.48 (3H, s), 4.47 (2H, s), 5.18 (2H, s), 6.95-7.10 (3H, m), 7.20-7.30 (1H, m)

### 4-tert-Butyldimethylsilyloxybenzyl bromide

¹H-NMR(CDCl₃) δ ppm:
   0.20 (6H, s), 0.98 (9H, s), 4.49 (2H, s), 6.79 (2H, d, J=8.5Hz), 7.23-7.30 (2H, m)

### 3-Sulfamoylbenzyl bromide

¹H-NMR(CDCl₃) δ ppm:
   4.51 (2H, s), 4.79 (2H, br-s), 7.52 (1H, t, J=7.8Hz), 7.58-7.66 (1H, m), 7.83-7.90 (1H, m), 7.96 (1H, t, J=1.8Hz)

### 3-Methanesulfonylbenzyl bromide

¹H-NMR(CDCl₃) δ ppm:
   3.07 (3H, s), 4.53 (2H, s), 7.57 (1H, t, J=7.8Hz), 7.66-7.72 (1H, m), 7.85-7.92 (1H, m), 7.95-8.01 (1H, m)

### 3-Benzyloxybenzyl bromide

¹H-NMR(CDCl₃) δ ppm:
   4.45 (2H, s), 5.05 (2H, s), 6.89 (1H, dd, J=8.3Hz, 2.5Hz), 6.95-7.04 (2H, m), 7.20-7.47 (6H, m)

### Reference Example 23

### 4-Benzyloxy-1-bromo-2-butene

4-Benzyloxy-2-buten-1-ol (950 mg) and 2.12 g of carbon tetrabromide were dissolved in 80 ml of dichloromethane, and 1.68 g of triphenylphosphine was added portionwise to the solution. The mixture was stirred under an argon atmosphere at room temperature for 1 hour, the reaction mixture was concentrated under reduced pressure. The resulting residue was purified by silica gel column chromatography (eluent: ethyl acetate-hexane) to give 980 mg of 4-benzyloxy-1-bromo-2-butene.
¹H-NMR(CDCl₃) δ ppm:
   3.99 (2H, d, J=8.3Hz), 4.15 (2H, dd, J=6.3Hz, 1.5Hz), 4.54 (2H, s), 5.70-5.82 (1H, m), 5.85-5.96 (1H, m), 7.20-7.40 (5H, m)

### Reference Example 24

### 4-Cyano-2-nitrobenzyl acetate

4-Cyanobenzyl acetate (5.0 g) was dissolved in 100 ml of acetonitrile. To the solution was added dropwise a solution of 3.1 g of nitronium tetrafluoroborate (85 %) in 30 ml of acetonitrile under an argon atmosphere under ice-cooling, and the mixture was allowed to react at room temperature for 3 hours. The reaction mixture was concentrated under reduced pressure, water was added to the residue, and the mixture was extracted with ethyl acetate. The organic layer was washed with water and brine, and dried over anhydrous magnesium sulfate. The solvent was removed under reduced pressure, and the resulting residue was suspended in diethyl ether and a small amount of dichloromethane. The insoluble material was collected by filtration and washed with diisopropyl ether to give 0.95 g of 4-cyano-2-nitrobenzyl acetate.
¹H-NMR(CDCl₃) δ ppm:
   2.21 (3H, s), 5.57 (2H, s), 7.79 (1H, d, J=8.1Hz), 7.94(1H, dd, J=8.1Hz, 1.7Hz), 8.42 (1H, d, J=1.7Hz)

### Reference Example 25

### 4-Hydroxymethyl-3-nitrobenzonitrile

4-Cyano-2-nitrobenzyl acetate (2.0 g) was suspended in 20 ml of ethanol, 4.8 ml of 2 N aqueous sodium hydroxide solution was added dropwise to the suspension, and the mixture was allowed to react at room temperature for 50 minutes. Water was added to the reaction mixture, and the mixture was extracted with diethyl ether. The organic layer was washed with water and brine, and dried over anhydrous magnesium sulfate. The solvent was removed under reduced pressure, and the resulting residue was purified by silica gel column chromatography (eluent: methanol-diethyl ether-dichloromethane) to give 342 mg of 4-hydroxymethyl-3-nitrobenzonitrile.
¹H-NMR(CDCl₃) δ ppm:
   2.20-2.48 (1H, br), 5.14 (2H, s), 7.97 (1H, dd, J=8.1Hz, 1.6Hz), 8.07 (1H, d, J=8.1Hz), 8.42 (1H, d, J=1.6Hz)

### Reference Example 26

### 4-tert-Butyldimethylsilyloxymethyl-3-nitrobenzonitrile

4-Hydroxymethl-3-nitrobenzonitrile (320 mg) was dissolved in 3 ml of *N,N*-dimethylformamide, 300 mg of tert-butyldimethylsilyl chloride and 150 mg of imidazole were added to the solution, and the mixture was allowed to react under an argon atmosphere at room temperature for 40 minutes. Water was added to the reaction mixture, and the precipitates were collected and washed with water. The obtained material was dissolved in diethyl ether, and the solution was dried over anhydrous magnesium sulfate. The solvent was removed under reduced pressure to give 512 mg of 4-tert-butyldimethylsilyloxy-methyl-3-nitrobenzonitrile.
¹H-NMR(CDCl₃) δ ppm:
   0.16 (6H, s), 0.97 (9H, s), 5.15 (2H, s), 7.95 (1H, dd, J=8.2Hz, 1.6Hz), 8.12 (1H, d, J=8.2Hz), 8.41 (1H, d, J=1.6Hz)

### Reference Example 27

### 3-Amino-4-tert-butyldimethylsilyloxymethylbenzonitrile

4-tert-Butyldimethylsilyloxymethyl-3-nitrobenzonitrile (500 mg) was suspended in ethanol, and 770 mg of tin(II) chloride dihydrate was added to the suspension. To the mixture was added portionwise 130 mg of sodium borohydride under ice-cooling, and the mixture was allowed to react under an argon atmosphere at room temperature for 2 hours. Water was added to the reaction mixture, and the insoluble material was collected by filtration and dissolved in diethyl ether. The solution was washed successively with a saturated aqueous sodium hydrogen carbonate solution, water and brine. The organic layer was dried over anhydrous magnesium sulfate, and the solvent was removed under reduced pressure. The resulting residue was dissolved in 4 ml of acetic acid, 910 mg of zinc powder was added to the solution, and the mixture was allowed to react at room temperature for 18 hours. Water was added to the reaction mixture, and the mixture was alkalized with a saturated aqueous sodium hydrogen carbonate solution. The precipitates were collected by filtration and dissolved in ethyl acetate. The solution was washed successively with water, a saturated aqueous ammonium chloride solution and brine. The organic layer was dried over anhydrous magnesium sulfate, and the solvent was removed under reduced pressure to give 377 mg of 3-amino-4-tert-butyldimethylsilyloxymethylbenzonitrile.
¹H-NMR(CDCl₃) δ ppm:
   0.08 (6H, s), 0.90 (9H, s), 4.34-4.46 (2H, br), 4.68 (2H, s), 6.89 (1H, d, J=1.4Hz), 6.97 (1H, dd, J=7.6Hz, 1.5Hz), 7.11 (1H, d, J=7.6Hz)

### Reference Example 28

### 2-(2-tert-Butyldimethylsilyloxymethyl-5-cyanophenylamino)acetic acid

3-Amino-4-tert-butyldimethylsilyloxymethylbenzonitrile (300 mg) was dissolved in 3 ml of dimethylsulfoxide. To the solution were added 320 mg of potassium carbonate, 210 mg of sodium iodide and 0.19 ml of ethyl bromoacetate, and the mixture was allowed to react at 80 °C for 7 hours. To the reaction mixture was added water, and the mixture was extracted with diethyl ether. The organic layer was washed with water and brine, and dried over anhydrous magnesium sulfate. The solvent was removed under reduced pressure, and the resulting residue was purified by silica gel column chromatography (eluent: ethyl acetate-hexane). The resulting ester compound was dissolved in 4 ml of ethanol. To the solution was added 0.46 ml of 2 N aqueous sodium hydroxide solution, and the mixture was allowed to react at room temperature for 2 hours. The reaction mixture was concentrated under reduced pressure, water was added to the residue, and the mixture was washed with diethyl ether. After the aqueous layer was acidified with dilute hydrochloric acid, the mixture was extracted with diethyl ether, and the organic layer was washed with water and brine, and dried over anhydrous magnesium sulfate. The solvent was removed under reduced pressure to give 241 mg of 2-(2-tert-butyldimethylsilyloxymethyl-5-cyanophenylamino)acetic acid.
¹H-NMR(CDCl₃) δ ppm:
   0.87 (6H, s), 0.90 (9H, s), 4.01 (2H, s), 4.72 (2H, s), 5.69-5.85 (1H, br), 6.71 (1H, d, J=1.5Hz), 7.01 (1H, dd, J=7.6Hz, 1.5Hz), 7.13 (1H, d, J=7.6Hz)
MS(FAB, m/z) : 321(M+H)

### Reference Example 29

### 4-Chloro-3-nitrobenzamide

4-Chloro-3-nitrobenzoic acid (4.72 g) and 5.12 ml of thionyl chloride were added to 3 ml of toluene, and the mixture was stirred at 90 °C for 2 hours. After the reaction mixture was concentrated under reduced pressure, 15 ml of 25 % aqueous ammonia solution was added to the residue under ice-cooling, and the mixture was stirred for 15 minutes. The precipitates were collected by filtration and washed with water to give 4.67 g of 4-chloro-3-nitrobenzamide.
¹H-NMR(DMSO-d₆) δ ppm:
   7.77 (1H, br-s), 7.90 (1H, d, J=8.5Hz), 8.16 (1H, dd, J=8.5Hz, 2.1Hz), 8.30 (1H, br-s), 8.51 (1H, d, J=2.1Hz)

### Reference Example 30

The following compound was prepared using the same procedure as Reference Example 29.

### 4-Fluoro-3-nitrobenzamide

¹H-NMR(DMSO-d₆) δ ppm:
   7.65-7.85 (2H, m), 8.25-8.40 (2H, m), 8.60-8.70 (1H, m)

### Reference Example 31

### 3-Amino-4-chlorobenzamide

4-Chloro-3-nitrobenzamide (3.41 g) and 30 mg of platinum(IV) oxide were added to 80 ml of acetic acid, and the mixture was stirred at atmospheric pressure under a hydrogen atmosphere at room temperature for 2 hours. The insoluble material was removed by filtration, and the filtrate was concentrated under reduced pressure. A saturated aqueous sodium hydrogen carbonate solution was added to the residue, and the resulting precipitates were collected by filtration and washed with brine to give 2.21 g of 3-amino-4-chlorobenzamide.

¹H-NMR(DMSO-d₆) δ ppm: 5.48 (2H, s), 7.00 (1H, dd, J=8.2Hz, 2.1Hz), 7.15-7.32 (3H, m), 7.81 (1H, br-s)

### Reference Example 32

### 4-Methyl-3-trifluoroacetylaminobenzonitrile

3-Amino-4-methylbenzamide (2.87 g) was dissolved in 15 ml of pyridine, 8.1 ml of trifluoroacetic anhydride was added dropwise to the solution under ice-cooling, and the mixture was stirred for 30 minutes. The reaction mixture was acidified with dilute hydrochloric acid, and the precipitates were collected by filtration and washed with water to give 2.90 g of 4-methyl-3-trifluoroacetylaminobenzonitrile.
¹H-NMR(CDCl₃) δ ppm:
   2.39 (3H, s), 7.38 (1H, d, J=7.9Hz), 7.49 (1H, dd, J=7.9Hz, 1.6Hz), 7.86 (1H, br-s), 8.18 (1H, d, J=1.6Hz)

### Reference Example 33

The following compound was prepared using the same procedure as Reference Example 32.

### 4-Chloro-3-trifluoroacetylaminobenzonitrile

¹H-NMR(CDCl₃) δ ppm:
   7.49 (1H, dd, J=8.3Hz, 1.9Hz), 7.60 (1H, d, J=8.3Hz), 8.48 (1H, br-s), 8.72 (1H, d, J=1.9Hz)

### Reference Example 34

### 3-Trifluoroacetylaminobenzonitrile

3-Aminobenzonitrile (1.0 g) was dissolved in 10 ml of pyridine, 2.13 g of trifluoroacetic anhydride was added dropwise to the solution under ice-cooling, and the mixture was allowed to react at room temperature for 1 hour. The reaction mixture was concentrated under reduced pressure, and ethyl acetate was added to the resulting residue. The mixture was washed with 1 N hydrochloric acid and brine. The organic layer was dried over anhydrous magnesium sulfate, and the solvent was removed under reduced pressure to give 1.58 g of 3-trifluoroacetylaminobenzonitrile.
¹H-NMR(CDCl₃) δ ppm:
   7.50-7.59 (2H, m), 7.80 (1H, dt, J=6.6Hz, 2.5Hz), 7.92-8.15 (2H, m)

### Reference Example 35

### 4-Fluoro-3-trifluoroacetylaminobenzonitrile

4-Fluoro-3-nitrobenzamide (640 mg) and 5 mg of platinum(IV) oxide were added to 10 ml of acetic acid, and the mixture was stirred at atmospheric pressure under a hydrogen atmosphere at room temperature for 2 hours. The insoluble material was removed by filtration, the filtrate was concentrated under reduced pressure, and a saturated aqueous sodium hydrogen carbonate solution was added to the resulting residue. The insoluble material was collected by filtration and washed with brine. To the obtained material was added 5 ml of pyridine, and trifluoroacetic anhydride was added dropwise to the mixture under ice-cooling. The resulting mixture was stirred for 30 minutes. Dilute hydrochloric acid was added to the reaction mixture, and the precipitates were collected by filtration and washed with water to give 312 mg of 4-fluoro-3-trifluoroacetylaminobenzonitrile.
¹H-NMR(CDCl₃) δ ppm:
   7.20-7.40 (1H, m), 7.50-7.60 (1H, m), 8.15 (1H, br-s), 8.70 (1H, dd, J=7.1Hz, 2.0Hz)

### Reference Example 36

### 2-(5-Cyano-2-methylphenylamino)acetic acid

Sodium hydride (0.52 g, 60 % in oil) was suspended in dry hexane, and the supernatant was decanted. A solution of 1.97 g of 4-methyl-3-trifluoroacetylaminobenzonitrile in 50 ml of dimethylsulfoxide and 1.44 ml of ethyl bromoacetate were added to the residue under an argon atmosphere under ice-cooling, and the mixture was stirred at 60 °C for 20 hours. Water was added to the reaction mixture, and the mixture was extracted with diethyl ether. The organic layer was washed with brine and dried over anhydrous magnesium sulfate. The solvent was removed under reduced pressure, 20 ml of methanol and 30 ml of 1 N aqueous sodium hydroxide solution were added to the resulting residue, and the mixture was stirred at room temperature for 6 hours. The reaction mixture was acidified with dilute hydrochloric acid and extracted with diethyl ether. The organic layer was washed with brine and dried over anhydrous magnesium sulfate. The solvent was removed under reduced pressure to give 0.93 g of 2-(5-cyano-2-methylphenylamino)acetic acid.
¹H-NMR(DMSO-d₆) δ ppm:
   2.16 (3H, s), 3.92 (2H, s), 6.71 (1H, d, J=1.5Hz), 6.94 (1H, dd, J=7.5Hz, 1.5Hz), 7.16 (1H, d, J=7.5Hz)

### Reference Example 37

The following compounds were prepared using the same procedure as Reference Example 36.

### 2-(5-Cyano-2-fluorophenylamino)acetic acid

¹H-NMR(DMSO-d₆) δ ppm:
   3.94 (2H, d, J=6.3Hz), 6.15-6.30 (1H, m), 7.00-7.13 (2H, m, 7.25 (1H, dd, J=11.8Hz, 8.2Hz), 12.69 (1H, br-s)

### 2-(2-Chloro-5-cyanophenylamino)acetic acid

¹H-NMR(DMSO-d₆) δ ppm:
   3.99 (2H, d, J=6.0Hz), 6.08 (1H, t, J=6.0 Hz), 6.95-7.08 (2H, m), 7.47 (1H, d, J=8.0Hz), 12.78 (1H, br-s)

### Reference Example 38

### 3-Methylaminobenzonitrile

3-Trifluoroacetylaminobenzonitrile (950 mg) was dissolved in 15 ml of acetone, and 2.67 g of methyl iodide was added to the solution. To the mixture was added 1.05 g of potassium hydroxide powder under reflux, and the mixture was heated under reflux for 10 minutes. The reaction mixture was concentrated under reduced pressure, 10 ml of ethanol and 5 ml of water were added to the residue, and the mixture was heated under reflux for 10 minutes. The solvent was removed under reduced pressure, water was added to the resulting residue, and the mixture was extracted with ethyl acetate. The organic layer was washed with brine and dried over anhydrous magnesium sulfate. The solvent was removed under reduced pressure to give 550 mg of 3-methylaminobenzonitrile.
¹H-NMR(CDCl₃) δ ppm:
   2.85 (3H, s), 3.75-4.27 (1H, br), 6.75-6.82 (2H, m), 6.96 (1H, dt, J=7.5Hz, 1.2Hz), 7.19-7.28 (1H, m)

### Reference Example 39

### 2-[N-(3-Cyanophenyl)-N-methylaminolacetic acid

3-Methylaminobenzonitrile (545 mg) was dissolved in 15 ml of dimethylsulfoxide, and 570 mg of potassium carbonate and 828 mg of ethyl bromoacetate were added to the solution, and the mixture was allowed to react at 75 °C for 14 hours, the reaction mixture was poured into water. The mixture was extracted with ethyl acetate. The organic layer was washed with water and brine, and dried over anhydrous magnesium sulfate. The solvent was removed under reduced pressure, and the resulting residue was dissolved in 10 ml of ethanol. To the solution was added 4.0 ml of 2 N aqueous sodium hydroxide solution, and the mixture was allowed to react at room temperature for 22 hours. The reaction mixture was concentrated under reduced pressure, the resulting residue was well alkalized with 1 N aqueous sodium hydroxide solution, and the mixture was washed with ethyl acetate. The aqueous layer was acidified with 1 N hydrochloric acid, and the mixture was extracted with ethyl acetate. The organic layer was washed with brine and dried over anhydrous magnesium sulfate. The solvent was removed under reduced pressure to give 385 mg of 2-[*N*-(3-cyanophenyl)-*N*-methylamino]acetic acid.
¹H-NMR(CDCl₃) δ ppm:
   3.09 (3H, s), 4.14 (2H, s), 6.85-6.93 (2H, m), 7.02-7.08 (1H, m), 7.24-7.35 (1H, m)

### Reference Example 40

### 3-Amino-4-benzyloxybenzonitrile

4-Benzyloxy-3-nitrobenzonitrile (1,57 g) was dissolved in ethanol, 100 mg of 5% platinum on carbon was added to the solution, and the mixture was stirred at atmospheric pressure under a hydrogen atmosphere at room temperature for 3 hours. The insoluble material was removed by filtration, and the filtrate was concentrates under reduced pressure to give 1.30 g of 3-amino-4-benzyloxybenzonitrile.
¹H-NMR(CDCl₃) δ ppm:
   4.03 (2H, br-s), 5.13 (2H, s), 6.85 (1H, d, J=8.3Hz), 6.93 (1H, d, J=2.0 Hz), 7.02 (1H, dd, J=8.3Hz, 2.0Hz), 7.31-7.46 (5H, m)

### Reference Example 41

### Benzyl 2-(3-cyanophenylamino)acetate

3-Aminobenzonitrile (1.03 g) and 2.4 g of potassium carbonate were added to 20 ml of *N,N*-dimethylformamide, 1.8 ml of benzyl bromoacetate was added dropwise to the mixture, and the resulting mixture was stirred at 60 °C for 18 hours. The reaction mixture was acidified with dilute hydrochloric acid, and the mixture was extracted with diethyl ether. The organic layer was washed successively with a saturated aqueous sodium hydrogen carbonate solution and brine, and dried over anhydrous magnesium sulfate. The solvent was removed under reduced pressure, and the resulting residue was purified by silica gel column chromatography (eluent: ethyl acetate-hexane) to give 1.03 g of benzyl 2-(3-cyanophenylamino)acetate.
¹H-NMR(CDCl₃) δ ppm:
   3.95 (2H, d, J=5.5Hz), 4.46-4.60 (1H, m), 5.23 (2H, s), 6.75-6.85 (2H, m), 6.98-7.05 (1H, m), 7.20-7.45 (6H, m)

### Reference Example 42

The following compounds were prepared using the same procedure as Reference Example 41.

### Ethyl 2-(2-benzyloxy-5-cyanophenylamino)acetate

¹H-NMR(CDCl₃) δ ppm:
   1.30 (3H, t, J=7.1Hz), 3.91 (2H, s), 4.26 (2H, q, J=7.1Hz), 4.97-5.21 (3H, m), 6.65 (1H, d, J=2.0Hz), 6.83 (1H, d, J=8.3Hz), 7.01 (1H, dd, J=8.3Hz, 2.0Hz), 7.27-7.50 (5H, m)

### Ethyl 2-(3-cyanophenylamino)acetate

¹H-NMR(CDCl₃) δ ppm:
   1.32 (3H, t, J=7.1Hz), 3.90 (2H, d, J=5.0Hz), 4.27 (2H, q, J=7.1Hz), 4.48-4.62 (1H, m), 6.75-6.85 (2H, m), 6.97-7.05 (1H, m), 7.20-7.30 (1H, m)

### Reference Example 43

### Ethyl 2-[N-(3-cyanophenyl)-N-(2-methylallyl)amino]acetate

Ethyl 2-(3-cyanophenylamino)acetate (500 mg) was dissolved in 10 ml of 2-butanol, and 0.7 ml of N-ethyldiisopropylamine, 0.48 ml of 3-chloro-2-methyl-1-propene and 1.36 g of tetrabutylammonium iodide were added to the solution. The mixture was stirred at 120 °C for 45 hours, and the reaction mixture was concentrated under reduced pressure. Dilute hydrochloric acid was added to the resulting residue, and the mixture was extracted with diethyl ether. The organic layer was washed successively with a saturated aqueous sodium hydrogen carbonate solution and brine, and dried over anhydrous magnesium sulfate. The solvent was removed under reduced pressure, and the resulting residue was purified by silica gel column chromatography (eluent: ethyl acetate-hexane) to give 250 mg of ethyl 2-[N-(3-cyanophenyl)-N-(2-methylallyl)amino]acetate.
¹H-NMR(CDCl₃) δ ppm:
   1.29 (3H, t, J=7.1Hz), 1.75 (3H, d, J=0.4Hz), 3.88 (2H, s), 4.03 (2H, s), 4.22 (2H, q, J=7.1Hz), 4.79-4.86 (1H, m), 4.88-4.94 (1H, m), 6.75-6.85 (2H, m), 6.95-7.03 (1H, m), 7.20-7.30 (1H, m)

### Reference Example 44

The following compounds were prepared using the same procedure as Reference Example 43.

### Ethyl 2-[N-(3-cyanophenyl)-N-(4-ethylbenzyl)amino]acetate

¹H-NMR(CDCl₃) δ ppm:
   1.24 (3H, t, J=7.6Hz), 1.28 (3H, t, J=7.1Hz), 2.64 (2H, q, J=7.6Hz), 4.08 (2H, s), 4.22 (2H, q, J=7.1Hz), 4.61 (2H, s), 6.84-6.91 (2H, m), 7.01 (1H, dt, J=7.6Hz, 1.1Hz), 7.11-7.30 (5H, m)

### Ethyl 2-[N-(3-benzyloxybenzyl)-N-(3-cyanophenyl)amino]acetate

¹H-NMR(CDCl₃) δ ppm:
   1.28 (3H, t, J=7.1Hz), 4.07 (2H, s), 4.22 (2H, q, J=7.1Hz), 4.60 (2H, s), 5.04 (2H, s), 6.75-7.48 (13H, m)

### Reference Example 45

### 2-[N-(3-Cyanophenyl)-N-(3-methyl-2-butenyl)amino]acetic acid

Ethyl 2-(3-cyanophenylamino)acetate (0.91 g) was dissolved in 100 ml of N,N-dimethylformamide, 214 mg of sodium hydride (60 % in oil) was added to the solution under ice-cooling, and the mixture was stirred under an argon atmosphere for 15 minutes. To the reaction mixture was added dropwise slowly 0.64 ml of 1-bromo-3-methyl-2-butene, and the mixture was stirred for 2 hours. The reaction mixture was acidified with dilute hydrochloric acid, and the mixture was extracted with diethyl ether. The organic layer was washed successively with a saturated aqueous sodium hydrogen carbonate solution and brine, and dried over anhydrous magnesium sulfate. The solvent was removed under reduced pressure, and the resulting residue was purified by silica gel column chromatography (eluent: ethyl acetate-hexane). To the resulting ester compound were added 5 ml of methanol and 20 ml of 1 N aqueous sodium hydroxide solution, and the mixture was stirred at room temperature for 24 hours. The reaction mixture was washed with dichloromethane, and the aqueous layer was acidified with dilute hydrochloric acid and extracted with diethyl ether. The organic layer was washed with brine and dried over anhydrous magnesium sulfate. The solvent was removed under reduced pressure to give 0.72 g of 2-[N-(3-cyanophenyl)-N-(3-methyl-2-butenyl)amino]acetic acid.
¹H-NMR (DMSO-d₆) δ ppm:
   1.69 (6H, s), 3.95 (2H, d, J=6.4Hz), 4.10 (2H, s), 5.10-5.23 (1H, m), 6.85-7.05 (3H, m), 7.26-7.37 (1H, m), 12.30-13.00 (1H, br)

### Reference Example 46

The following compound was prepared using the same procedure as Reference Example 45.

### 2-[N-Allyl-N-(3-cyanophenyl)amino]acetic acid

¹H-NMR(DMSO-d₆) δ ppm:
   4.00 (2H, d, J=4.7Hz), 4.12 (2H, s), 5.00-5.25 (2H, m), 5.71-5.92 (1H, m), 6.80-7.05 (3H, m), 7.18-7.37 (1H, m)

### Reference Example 47

### 2-[N-(3-cyanophenyl)-N-(4-ethylbenzyl)amino]acetic acid

Ethyl 2-[N-(3-cyanophenyl)-N-(4-ethylbenzyl)amino]acetate (420 mg) was dissolved in 5 ml of ethanol, and 1.3 ml of 2 N aqueous sodium hydroxide solution was added to the solution, and the mixture was allowed to react at room temperature for 13 hours. The reaction mixture was concentrated under reduced pressure. Water was added to the resulting residue, and the mixture was washed with ethyl acetate. The aqueous layer was acidified with 1 N hydrochloric acid and extracted with ethyl acetate. The organic layer was washed with brine and dried over anhydrous magnesium sulfate. The solvent was removed under reduced pressure to give 350 mg of 2-[N-(3-cyanophenyl)-N-(4-ethylbenzyl)amino]acetic acid.
¹H-NMR(CDCl₃) δ ppm:
   1.24 (3H, t, J=7.6Hz), 2.65 (2H, q, J=7.6Hz), 4.15 (2H, s), 4.56-4.66 (2H, m), 6.87-6.93 (2H, m), 7.04 (1H, dt, J=7.6Hz, 1.0Hz), 7.15 (2H d, J=8.3Hz), 7.19 (2H, d, J=8.3Hz), 7.23-7.32 (1H, m)

### Reference Example 48

### 2-(3-Cyanophenylamino)acetic acid

To 80.0 g of ethyl 2-(3-cyanophenylamino)acetate were added 100 ml of methanol and 500 ml of 1 N aqueous sodium hydroxide solution, and the mixture was stirred at room temperature for 2 hours. The reaction mixture was acidified with dilute hydrochloric acid and extracted with diethyl ether. The organic layer was washed with brine and dried over anhydrous magnesium sulfate. The solvent was removed under reduced pressure to give 64.3 g of 2-(3-cyanophenylamino)acetic acid.
¹H-NMR(CDCl₃) δ ppm:
   3.99 (2H, s), 6.78-6.87 (2H, m), 7.00-7.10 (1H, m), 7.20-7.30 (1H, m)

### Reference Example 49

The following compound was prepared using the same procedure as Reference Example 48.

### 2-(2-Benzyloxy-5-cyanophenylamino)acetic acid

¹H-NMR(DMSO-d₆) δ ppm:
   3.92 (2H, s), 5.24 (2H, s), 6.73-6.83 (1H, m), 6.95-7.12 (2H, m), 7.27-7.55 (5H, m), 11.50-13.50 (1H, br)

### Reference Example 50

### 2-[N-(3-Cyanophenyl)-N-(2-methylallyl)amino]-N-(4-isopropoxyphenyl)acetamide

To 250 mg of ethyl 2-[N-(3-cyanophenyl)-N-(2-methylallyl)amino]acetate were added 1 ml of methanol and 5 ml of 1 N aqueous sodium hydroxide solution, and the mixture was stirred at room temperature for 2 hours. The reaction mixture was acidified with dilute hydrochloric acid and extracted with diethyl ether. The organic layer was washed with brine and dried over anhydrous magnesium sulfate. The solvent was removed under reduced pressure, and the resulting residue and 135 mg of 4-isopropoxyaniline were dissolved in 30 ml of dichloromethane. To the solution was added portionwise 213 mg of 1-ethyl-3-(3-dimethylaminopropyl)carbodiimide hydrochloride. The mixture was stirred at room temperature for 6 hours, and the reaction mixture was well acidified with dilute hydrochloric acid and extracted with dichloromethane. The organic layer was washed successively with a saturated aqueous sodium hydrogen carbonate solution and brine, and dried over anhydrous magnesium sulfate. The solvent was removed under reduced pressure, and the resulting residue was purified by silica gel column chromatography (eluent: ethyl acetate-hexane) to give 223 mg of 2-[N-(3-cyanophenyl)-N-(2-methylallyl)amino]-N-(4-isopropoxyphenyl)acetamide.
¹H-NMR(CDCl₃) δ ppm:
   1.30 (6H, d, J=6.1Hz), 1.79 (3H, s), 3.98 (2H, s), 4.05 (2H, s), 4.40-4.55 (1H, m), 4.73 (1H, s), 4.99 (1H, s), 6.79-6.88 (2H, m), 6.89-7.00 (2H, m), 7.05-7.13 (1H, m), 7.27-7.40 (3H, m), 7.90 (1H, br-s)

### Reference Example 51

The following compound was prepared using the same procedure as Reference Example 50.

### 2-[N-(3-Benzyloxybenzyl)-N-(3-cyanophenyl)amino-N-(4-isopropoxyphenyl)acetamide

¹H-NMR(CDCl₃) δ ppm:
   1.30 (6H, d, J=5.9Hz), 4.07 (2H, s), 4.39-4.55 (1H, m), 4.66 (2H, s), 5.02 (2H, s), 6.73-7.50 (17H, m), 7.76 (1H, br-s)

### Reference Example 52

### Benzyl 2-[N-(1-tert-butoxycarbonylimidazol-4-ylmethyl)-N-(3-cyanophenyl)amino]acetate

4-Hydroxymethylimidazole hydrochloride (2.5 g) and 4.46 g of di-tert-butyl dicarbonate were suspended in 30 ml of dichloromethane, and 5.4 ml of triethylamine was added to the suspension. After allowing to react at room temperature for 5 hours, the reaction mixture was concentrated under reduced pressure. To the resulting residue was added diethyl ether under ice-cooling, and the insoluble material was removed by filtration. The filtrate was concentrated under reduced pressure, the resulting residue was suspended with hexane, and the insoluble material was collected by filtration. The resulting residue was dissolved in 30 ml of dichloromethane, 5.02 g of triphenylphosphine was added under ice-cooling, and 6.44 g of carbon tetrabromide was added portionwise to the mixture. The resulting mixture was allowed to react under an argon atmosphere at room temperature for 1 hour, the reaction mixture was concentrated to about half volume under reduced pressure, and the concentrate was suspended with diethyl ether. The resulting precipitates were removed by filtration, and the filtrate was concentrated under reduced pressure. The resulting residue was suspended in a mixture of diethyl ether-hexane, and the insoluble material was removed by filtration. After the filtrate was concentrated to about one-third volume under reduced pressure, 30 ml of 2-butanol was added quickly to the concentrate. To the mixture were added 1.8 g of benzyl 2-(3-cyanophenylamino)acetate, 2.61g of sodium iodide and 3.0 ml of N-ethyldiisopropylamine, and the resulting mixture was allowed to react at 60 °C for 5 hours. Diethyl ether was added to the reaction mixture, the resulting insoluble material was removed by filtration, and the filtrate was concentrated under reduced pressure. The resulting residue was purified by silica gel column chromatography (eluent: diethyl ether-dichloromethane) to give 928 mg of benzyl 2-[N-(1-tert-butoxycarbonylimidazol-4-ylmethyl)-N-(3-cyanophenyl)amino]acetate.
¹H-NMR(CDCl₃) δ ppm:
   1.61 (9H s), 4.19 (2H, s), 4.52 (2H, s), 5.20 (2H, s), 6.87-6.95 (2H, m), 7.02 (1H, dt, J=7.5Hz, 1.1Hz), 7.22-7.43 (7H, m), 8.03 (1H, d, J=1.3Hz)

### Reference Example 53

### Benzyl 2-[N-(3-cyanophenyl)-N-(4-imidazolylmethyl)amino]acetate

Benzyl 2-[N-(1-tert-butoxycarbonylimidazol-4-ylmethyl)-N-(3-cyanophenyl)amino]acetate (300 mg) was dissolved in 6 ml of ethyl acetate, 0.7 ml of 2 N hydrochloric acid was added to the solution, and the mixture was allowed to react at 60 °C for 10 hours. The reaction mixture was concentrated under reduced pressure, and a saturated aqueous sodium hydrogen carbonate solution was added to the resulting residue, and the mixture was extracted with diethyl ether. The organic layer was dried over anhydrous magnesium sulfate. The solvent was removed under reduced pressure to give 199 mg of benzyl 2-[N-(3-cyanophenyl)-N-(4-imidazolylmethyl)amino]acetate.
¹H-NMR(CDCl₃) δ ppm:
   4.22 (2H, s), 4.62 (2H, br-s), 5.24 (2H, br-s), 6.65-7.10 (4H, m), 7.15-7.70 (7H, m), 8.90-11.30 (1H, br)

### Reference Example 54

### Benzyl 2-[N-(3-cyanophenyl)-N-(1-methylimidazol-4-ylmethyl)amino]acetate

Benzyl 2-[N-(3-cyanophenyl)-N-(4-imidazolylmethyl)amino]acetate (199 mg), 43 µl of methyl iodide and 87 mg of potassium carbonate were suspended in 2 ml of N,N-dimethylformamide, and the suspension was allowed to react at room temperature for 15 hours. Water was added to the reaction mixture, and the mixture was extracted with diethyl ether. The organic layer was washed with water and brine, and dried over anhydrous magnesium sulfate. The solvent was removed under reduced pressure, and the resulting residue was purified by silica gel column chromatography (eluent: ethyl acetate-hexane) to give 33 mg of benzyl 2-[N-(3-cyanophenyl)-N-(1-methylimidazol-4-ylmethyl)amino]acetate.
¹H-NMR(CDCl₃) δ ppm:
   3.60 (3H, s), 4.19 (2H, s), 4.52 (2H, s), 5.19 (2H, s), 6.74 (1H, br-s), 6.88-6.95 (2H, m), 6.97-7.03 (1H, m), 7.20-7.42 (7H, m)

### Reference Example 55

### 2-[N-(3-Cyanophenyl)-N-(1-methylimidazol-4-ylmethyl)amino]acetic acid

Benzyl 2-[N-(3-cyanophenyl)-N-(1-methylimidazol-4-ylmethyl)amino]acetate (55mg) and 22 mg of 10 % palladium on carbon (50 % wet) were suspended in a mixture of 1 ml of methanol and 2 ml of tetrahydrofuran, and the suspension was stirred at atmospheric pressure under a hydrogen atmosphere at room temperature for 1 hour. The insoluble material was removed through Celite®, and the filtrate was concentrated under reduced pressure. The resulting residue was suspended in diethyl ether, the supernatent was decanted, and the resulting precipitates were dried under reduced pressure to give 36 mg of 2-[N-(3-cyanophenyl)-N-(1-methylimidazol-4-ylmethyl)amino]acetic acid.
¹H-NMR(CDCl₃) δ ppm:
   3.76 (3H, s), 4.31 (2H, s), 4.63 (2H, s), 6.73-7.07 (4H, m), 7.47 (1H, s)

### Reference Example 56

The following compounds were prepared using the same procedure as Reference Example 55.

### 2-[N-(1-tert-Butoxycarbonyl-5-methylimidazol-4-ylmethyl)-N-(3-cyanophenyl)amino]acetic acid

¹H-NMR(CDCl₃) δ ppm:
   1.64 (9H, s), 2.52 (3H, s), 4.31 (2H, s), 4.55 (2H, s), 6.65 (1H, br-s), 6.78-6.84 (1H, m), 7.00-7.07 (1H, m), 7.22-7.33 (1H, m), 8.06 (1H, s)

### 2-[N-(1-tert-Butoxycarbonylimidazol-4-ylmethyl)-N-(3-cyanophenyl)amino]acetic acid

¹H-NMR(CDCl₃) δ ppm:
   1.65 (9H, s), 4.31 (2H, s), 4.62 (2H, s), 6.75-6.80 (1H, m), 6.83-6.90 (1H, m), 7.01-7.07 (1H, m), 7.24-7.35 (1H, m), 7.42-7.47 (1H, m), 8.10 (1H, d, J=1.1Hz)

### Reference Example 57

### 2-[N-(3-Cyanophenyl)-N-(1-propylimidazol-4-ylmethyl)amino]acetic acid

Benzyl 2-[N-(3-cyanophenyl)-N-(4-imidazolylmethyl)amino]acetate (177 mg) was dissolved in 1 ml of N,N-dimethyl-formamide, 134 mg of potassium carbonate and 0.05 ml of propyl iodide were added to the solution, and the mixture was allowed to react at 30 °C for 22 hours. Water was added to the reaction mixture, and the mixture was extracted with diethyl ether. The organic layer was washed with water and brine, and dried over anhydrous magnesium sulfate. The solvent was removed under reduced pressure, and the resulting residue was purified by silica gel column chromatography (eluent: methanol-diethyl ether-dichloromethane). The resulting ester compound and 4 mg of 10 % palladium on carbon were suspended in a mixture of 1 ml of methanol and 1 ml of tetrahydrofuran, and the mixture was stirred at atmospheric pressure under a hydrogen atmosphere at room temperature for 3 hours. The insoluble material was removed through Celite®, and the filtrate was concentrated under reduced pressure. The resulting residue was suspended in diethyl ether, and the resulting precipitates were collected by filtration to give 18 mg of 2-[N-(3-cyanophenyl)-N-(1-propylimidazol-4-ylmethyl)amino]acetic acid.
¹H-NMR(CDCl₃) δ ppm:
   0.95 (3H, t, J=7.4Hz), 1.76-1.93 (2H, m), 3.87-3.99 (2H, m), 4.21-4.38 (2H, br), 4.62 (2H, br-s), 6.72-7.03 (4H, m), 7.45-7.55 (1H, m)

### Reference Example 58

### 2-(3-Cyanophenylamino)-N-(4-isopropylphenyl)acetamide

2-(3-Cyanophenylamino)acetic acid (2.20 g) and 1.88 ml of 4-isopropylaniline were dissolved in 80 ml of dichloromethane, 2.88 g of 1-ethyl-3-(3-dimethylaminopropyl)carbodiimide hydrochloride was added portionwise to the solution, and the mixture was stirred at room temperature for 2 hours. The reaction mixture was well acidified with dilute hydrochloric acid, and the mixture was extracted with dichloromethane. The organic layer was washed successively with a saturated aqueous sodium hydrogen carbonate solution and brine, and dried over anhydrous magnesium sulfate. The solvent was removed under reduced pressure, and the resulting residue was recrystallized from ethyl acetate to give 3.01 g of 2-(3-cyanophenylamino)-N-(4-isopropylphenyl)acetamide.
¹H-NMR(CDCl₃) δ ppm:
   1.22 (6H, d, J=6.9Hz), 2.79-2.94 (1H, m), 3.93 (2H, d, J=5.4Hz), 4.62-4.74 (1H, m), 6.82-6.93 (2H, m), 7.05-7.35 (4H, m), 7.42 (2H, d, J=8.5Hz), 8.16 (1H, br-s)

### Reference Example 59

The following compounds were prepared using the same procedure as Reference Example 58.

### 2-(3-Cyanophenylamino)-N-[4-(1-trifluoroacetylpiperidin-4-yl)phenyl]acetamide

¹H-NMR(CDCl₃) δ ppm:
   1.55-1.75 (2H, m), 1.88-2.01 (2H, m), 2.72-2.91 (2H, m), 3.16-3.28 (1H, m), 3.94 (2H, d, J=5.4Hz), 4.07-4.19 (1H, m), 4.62-4.77 (2H, m), 6.85-6.94 (2H, m), 7.06-7.20 (3H, m), 7.25-7.36 (1H, m), 7.47 (2H d, J=8.5Hz), 8.24 (1H, s)

### 2-[N-(1-tert-Butoxycarbonylimidazol-4-ylmethyl)-N-(3-cyanophenyl)amino]-N-(4-isopropylphenyl)acetamide

¹H-NMR(CDCl₃) δ ppm:
   1.22 (6H, d, J=6.9Hz), 1.66 (9H, s), 2.80-2.94 (1H, m), 4.20 (2H, s), 4.61 (2H, s), 6.87-6.97 (2H, m), 7.00-7.07 (1H, m), 7.18 (2H, d, J=8.5 Hz), 7.22-7.30 (1H, m), 7.42 (1H, d, J=1.1Hz), 7.58 (2H, d, J=8.5Hz), 8.08 (1H, d, J=1.1Hz), 11.11 (1H, s)

### 2-[N-(3-Cyanophenyl)-N-(1-propylimidazol-4-ylmethyl)amino]-N-(4-isopropylphenyl)acetamide

¹H-NMR(CDCl₃) δ ppm:
   0.94 (3H, t, J=7.4Hz), 1.21 (6H, d, J=6.9Hz), 1.74-1.91 (2H, m), 2.75-2.92 (1H, m), 3.91 (2H, t, J=7.1Hz), 4.21 (2H, s), 4.62 (2H, s), 6.87-7.08 (4H, m), 7.12-7.29 (3H, m), 7.46 (1H, 6, J=1.2Hz), 7.60 (2H, d, J=8.6Hz), 11.74 (1H, s)

### 2-[N-(3-Cyanophenyl)-N-(1-methylimidazol-4-ylmethyl)amino]-N-(4-isopropylphenyl)acetamide

¹H-NMR(CDCl₃) δ ppm:
   1.21 (6H, d, J=6.9Hz), 2.78-2.91 (1H, m), 3.73 (3H, s), 4.21 (2H, s), 4.61 (2H, s), 6.87-7.06 (4H, m), 7.13-7.35 (3H, m), 7.44 (1H, s), 7.60 (2H, d, J=8.6Hz), 11.71 (1H, s)

### 2-[N-(3-Cyanophenyl)-N-(3-methyl-2-butenyl)amino]-N-[4-(1-trifluoroacetylpiperidin-4-yl)phenyl]acetamide

¹H-NMR(CDCl₃) δ ppm:
   1.45-1.85 (8H, m), 1.90-2.05 (2H, m), 2.73-2.92 (2H, m), 3.16-3.30 (1H, m), 3.97 (2H, s), 4.03 (2H, d, J=8.3Hz), 4.08-4.20 (1H, m), 4.62-4.75 (1H, m), 5.20-5.30 (1H, m), 6.90-7.52 (8H, m), 8.12 (1H, br-s)

### 2-[N-(1-tert-Butoxycarbonylimidazol-4-ylmethyl)-N-(3-cyanophenyl)amino]-N-(4-isopropoxyphenyl)acetamide

¹H-NMR(CDCl₃) δ ppm:
   1.31 (6H, d, J=6.1Hz), 1.65 (9H, s), 4.19 (2H, s), 4.44-4.54 (1H, m), 4.61 (2H, s), 6.81-6.96 (4H, m), 7.01-7.09 (1H, m), 7.22-7.32 (1H, m), 7.42 (1H, d, J=1.1Hz), 7.54 (2H, d, J=9.0Hz), 8.07 (1H, d, J=1.1Hz), 11.04 (1H, br-s)

### 2-[N-(1-tert-Butoxycarbonyl-5-methylimidazol-4-ymethyl)-N-(3-cyanophenyl)amino]-N-(4-isopropoxyphenyl)acetamide

¹H-NMR(CDCl₃) δ ppm:
   1.31 (6H, d, J=6.1Hz), 1.65 (9H, s), 2.52 (3H, s), 4.19 (2H, s), 4.42-4.57 (3H, m), 6.73-6.79 (1H, m), 6.81-6.89 (3H, m), 6.99-7.06 (1H, m) 7.20-7.30 (1H, m), 7.55 (2H, d, J=9.0Hz), 8.03 (1H, s), 11.32 (1H, br-s)

### 2-(3-Cyanophenylamino)-N-(4-isopropoxyphenyl)acetamide

¹H-NMR(CDCl₃) δ ppm:
   1.31 (6H, d, J=6.0Hz), 3.93 (2H, d, J=5.3Hz), 4.42-4.56 (1H, m), 4.58-4.69 (1H, m), 6.80-6.95 (4H, m), 7.05-7.15 (1H, m), 7.27-7.43 (3H, m), 8.05 (1H, br-s)

### 2-[N-(3-Cyanophenyl)-N-(3-methyl-2-butenyl)amino]-N-(4-isopropoxyphenyl)acetamide

¹H-NMR(CDCl₃) δ ppm:
   1.31 (6H, d, J=6.0Hz), 1.72-1.81 (6H, m), 3.96 (2H, s), 4.03 (2H, d, J=6.6Hz), 4.43-4.57 (1H, m), 5.19-5.29 (1H, m), 6.84 (2H, d, J=9.0Hz), 6.92-6.99 (1H, m), 7.00-7.06 (1H, m), 7.08-7.15 (1H, m), 7.30-7.40 (3H, m), 8.03 (1H, br-s)

### 2-[N-Allyl-N-(3-cyanophenyl)amino]-N-(4-isopropoxyphenyl)acetamide

¹H-NMR(CDCl₃) δ ppm:
   1.31 (6H, d, J=6.1Hz), 4.02 (2H, s), 4.09 (2H, d, J=5.1Hz), 4.43-4.55 (1H, m), 5.17-5.37 (2H, m), 5.80-5.96 (1H, m), 6.76-7.43 (8H, m), 7.96 (1H, br-s)

### 2-[N-(3-Cyanophenyl)-N-methylamino]-N-(4-isopropylphenyl)acetamide

¹H-NMR(CDCl₃) δ ppm:
   1.22 (6H, d, J=6.9Hz), 2.82-2.94 (1H, m), 3.14 (3H, s), 4.00 (2H, s), 6.95-7.01 (1H, m), 7.02-7.07 (1H, m), 7.12-7.22 (3H, m), 7.32-7.45 (3H, m), 8.01 (1H, br-s)

### 2-[N-(3-Cyanophenyl)-N-methylamino]-N-(4-isopropoxyphenyl)acetamide

¹H-NMR(CDCl₃) δ ppm:
   1.31 (6H, d, J=6.1Hz), 3.13 (3H, s), 3.99 (2H, s), 4.43-4.56 (1H, m), 6.84 (2H, d, J=9.0Hz), 6.95-7.01 (1H, m), 7.02-7.06 (1H, m), 7.12-7.17 (1H, m). 7.32-7.40 (3H, m), 7.93 (1H, br-s)

### 2-[N-(3-Cyanophenyl)-N-(4-ethylbenzyl)amino]-N-(4-isopropoxyphenyl)acetamide

¹H-NMR(CDCl₃) δ ppm:
   1.23 (3H, t, J=7.6Hz), 1.30 (6H, d, J=6.0Hz), 2.65 (2H, q, J=7.6Hz), 4.10 (2H, s), 4.42-4.53 (1H, m), 4.67 (2H, s), 6.81 (2H, d, J=9.0Hz), 7.02 (1H, dd, J=8.6Hz, 2.8Hz), 7.06-7.27 (8H, m), 7.30-7.40 (1H, m), 7.75 (1H, s)

### N-[4-(1-tert-Butoxycarbonylpiperidin-4-yloxy)phenyl]-2-(2-tert-butyldimethylsilyloxymethyl-5-cyanophenylamino)acetamide

¹H-NMR(CDCl₃) δ ppm:
   0.11 (6H, s), 0.90 (9H, s), 1.46 (9H, s), 1.65-1.79 (2H, m), 1.83-1.96 (2H, m), 3.26-3.39 (2H, m), 3.62-3.77 (2H, m), 3.95 (2H, d, J=5.6Hz), 4.37-4.47 (1H, m), 4.80 (2H, s), 5.93 (1H, t, J=5.6Hz), 6.78-6.83 (1H, m), 6.87 (2H, d, J=9.0Hz), 7.05-7.12 (1H, m), 7.16 (1H, d, J=7.6Hz), 7.38 (2H, d, J=9.0Hz), 8.06 (1H, s)

### 2-(2-Benzyloxy-5-cyanophenylamino)-N-[4-(1-tert-butoxycarbonylpiperidin-4-yloxy)phenyl]acetamide

¹H-NMR(CDCl₃) δ ppm:
   1.46 (9H, s), 1.65-1.79 (2H, m), 1.83-1.96 (2H, m), 3.26-3.38 (2H, m), 3.62-3.75 (2H, m), 3.91 (2H, d, J=5.7Hz), 4.36-4.46 (1H, m), 5.10-5.20 (3H, m), 6.76 (1H, d, J=1.9Hz), 6.83-6.97 (3H, m), 7.12 (1H, dd, J=8.3Hz, 1.9Hz), 7.35-7.50 (7H, m), 8.18 (1H, br-s)

### N-[4-(1-tert-Butoxycarbonylpiperidin-4-yloxy)phenyl]-2-(5-cyano-2-methylphenylamino)acetamide

¹H-NMR(CDCl₃) δ ppm:
   1.47 (9H, s), 1.65-1.80 (2H, m), 1.83-1.98 (2H, m), 2.31 (3H, s), 3.26-3.39 (2H, m), 3.61-3.75 (2H, m), 3.97 (2H, d, J=5.2Hz), 4.36-4.49 (1H, m), 4.56 (1H, t, J=5.2Hz), 6.75 (1H, d, J=1.3Hz), 6.88 (2H, d, J=9.0Hz), 7.06 (1H, dd, J=7.6Hz, 1.3Hz), 7.19 (1H, d, J=7.6Hz), 7.41 (2H, d, J=9.0Hz), 7.99 (1H, s)

### N-[4-(1-tert-Butoxycarbonylpiperidin-4-yloxy)phenyl]-2-(2-chloro-5-cyanophenylamino)acetamide

¹H-NMR(CDCl₃) δ ppm:
   1.47 (9H, s), 1.67-1.80 (2H, m), 1.84-1.97 (2H, m), 3.27-3.39 (2H, m), 3.63-3.76 (2H, m), 4.00 (2H, d, J=5.3Hz), 4.38-4.48 (1H, m), 5.30 (1H, t, J=5.3Hz), 6.81-6.95 (3H, m), 7.06 (1H, dd, J=8.1Hz, 1.8Hz), 7.37-7.46 (3H, m), 7.89 (1H, br-s)

### 2-(5-Cyano-2-fluorophenylamino)-N-(4-isopropoxyphenyl)acetamide

¹H-NMR(CDCl₃) δ ppm:
   1.32 (6H, d, J=6.1Hz), 3.97 (2H, d, J=5.5Hz), 4.45-4.55 (1H, m), 4.85-4.95 (1H, m), 6.82-6.95 (3H, m), 7.08-7.19 (2H, m), 7.40 (2H, d, J=9.0Hz), 7.93 (1H, br-s)

### N-[4-(1-tert-Butoxycarbonylpiperidin-4-yloxy)phenyl]-2-(3-cyanophenylamino)acetamide

¹H-NMR(DMSO-d₆) δ ppm:
   1.25-1.60 (11H, m), 1.78-1.95 (2H, m), 3.05-3.25 (2H, m), 3.55-3.75 (2H, m), 3.89 (2H, d, J=6.2Hz), 4.37-4.55 (1H, m), 6.43-6.57 (1H, m), 6.82-7.02 (5H, m), 7.20-7.35 (1H, m), 7.48 (2H, d, J=8.8Hz), 9.87 (1H, s)

### Reference Example 60

The following compounds were prepared using the same procedure as Reference Example 52.

### Benzyl 2-[N-(1-tert-butoxycarbonyl-5-methylimidazol-4-ylmethyl)-N-(3-cyanophenyl)amino]acetate

¹H-NMR(CDCl₃) δ ppm:
   1.61 (9H, s), 2.35 (3H, s), 4.14 (2H, s), 4.43 (2H, s), 5.17 (2H, s), 6.98-7.06 (3H, m), 7.22-7.42 (6H, m), 7.98 (1H, s)

### 2-[N-(1-tert-Butoxycarbonyl-5-methylimidazol-4-ylmethyl)-N-(3-cyanophenyl)amino]-N-[4-(1-trifluoroacetylpiperidin-4-yl)phenyl]acetamide

¹H-NMR(CDCl₃) δ ppm:
   1.59-1.77 (11H, m), 1.89-2.00 (2H, m), 2.52 (3H, s), 2.71-2.91 (2H, m), 3.18-3.29 (1H, m), 4.08-4.18 (1H, m), 4.20 (2H, s), 4.53 (2H, s), 4.64-4.74 (1H, m), 6.71-6.80 (1H, m), 6.81-6.87 (1H, m), 6.98-7.06 (1H, m), 7.15 (2H, d, J=8.6Hz), 7.20-7.30 (1H, m), 7.62 (2H, d, J=8.6Hz), 8.04 (1H, s), 11.48 (1H, s)

### 2-[N-(1-tert-Butoxycarbonyl-5-methylimidazol-4-ylmethyl)-N-(3-cyanophenyl)amino]-N-(4-isopropylphenyl)acetamide

¹H-NMR(CDCl₃) δ ppm:
   1.22 (6H, d, J=6.9Hz), 1.65 (9H, s), 2.52 (3H, s), 2.80-2.93 (1H, m), 4.19 (2H, s), 4.53 (2H, s), 6.74-6.80 (1H, m), 6.82-6.89 (1H, m), 6.98-7.05 (1H, m), 7.17 (2H, d, J=8.5Hz), 7.21-7.29 (1H, m), 7.57 (2H, d, J=8.5Hz), 8.04 (1H, s), 11.31 (1H, br-s)

### Reference Example 61

### 2-(3-Cyanophenylamino)-N-[4-(1-trifluoroacetylpiperidin-4-yloxy)phenyl]acetamide

N-[4-(1-tert-Butoxycarbonylpiperidin-4-yloxy)phenyl]-2-(3-cyanophenylamino)acetamide (330 mg) was dissolved in a mixture of 30 ml of ethanol and 20 ml of dichloromethane, and hydrogen chloride gas was introduced into the solution under ice-cooling. After precipitation, the mixture was stirred at room temperature for 10 hours. The reaction mixture was concentrated under reduced pressure, and the resulting residue was dissolved in 4 ml of pyridine. To the solution was added dropwise 0.12 ml of trifluoroacetic anhydride under ice-cooling, and the mixture was stirred at room temperature for 10 minutes. The reaction mixture was concentrated under reduced pressure, and the resulting residue was purified by silica gel column chromatography (eluent: ethyl acetate-hexane) to give 290 mg of 2-(3-cyanophenylamino)-N-[4-(1-trifluoroacetylpiperidin-4-yloxy)phenyl]acetamide.
¹H-NMR(CDCl₃) δ ppm:
   1.88-2.01 (4H, m), 3.60-3.82 (3H, m), 3.86-3.95 (1H, m), 4.43 (2H, s), 4.57-4.63 (1H, m), 6.90 (2H, d, J=8.9Hz), 7.38-7.48 (3H, m), 7.54-7.64 (1H, m), 7.71-7.83 (3H, m)

### Reference Example 62

### 2-[N-(3-Cyanophenyl)-N-(3-ethoxycarbonylmethyloxybenzyl)amino]-N-[4-(1-trifluoroacetylpiperidin-4-yl)phenyl]acetamide

2-(3-Cyanophenylamino)-N-[4-(1-trifluoroacetylpiperidin-4-yl)phenyl]acetamide (6.14 g), 5.5 g of methyl 2-(3-bromomethylphenyloxy)acetate and 2.57 g of sodium iodide were suspended in 100 ml of ethanol, 3.73 ml of N-ethyldiisopropylamine was added to the suspension, and the mixture was heated under reflux for 16 hours. Water was added to the reaction mixture, and the mixture was extracted with ethyl acetate. The organic layer was washed successively with water, dilute hydrochloric acid and brine, and dried over anhydrous magnesium sulfate. The solvent was removed under reduced pressure, and the resulting residue was purified by silica gel column chromatography (eluent: tetrahydrofurandichloromethane-hexane) to give 4.69 g of 2-(N-(3-cyanophenyl)-N-(3-ethoxycarbonylmethyloxybenzyl)amino]-N-[4-(1-trifluoroacetylpiperidin-4-yl)phenyl]acetamide.
¹H-NMR(CDCl₃) δ ppm:
   1.28 (3H, t, J=7.1Hz), 1.60-1.73 (2H, m), 1.89-2.01 (2H, m), 2.72-2.91 (2H, m), 3.17-3.28 (1H, m), 4.05-4.19 (3H, m), 4.24 (2H, q, J=7.1Hz), 4.59 (2H, s), 4.63-4.74 (3H, m), 6.77-6.90 (3H, m), 6.97-7.06 (2H, m), 7.10-7.19 (3H, m), 7.23-7.42 (4H, m), 7.83 (1H, br-s)

### Reference Example 63

The following compounds were prepared using the same procedure as Reference Example 62.

### 2-[N-(3-Benzyloxybenzyl)-N-(3-cyanophenyl)amino]-N-(4-isopropylphenyl)acetamide

¹H-NMR(CDCl₃) δ ppm:
   1.21 (6H, d, J=6.9Hz), 2.78-2.92 (1H, m), 4.08 (2H, s), 4.67 (2H, s), 5.02 (2H, s), 6.78-6.85 (2H, m), 6.89-7.18 (6H, m), 7.23-7.40 (9H, m), 7.82 (1H, br-s)

### 2-[N-(3-Chlorobenzyl)-N-(3-cyanophenyl)amino]-N-(4-isopropylphenyl)acetamide

¹H-NMR(DMSO-d₆) δ ppm:
   1.17 (6H, d, J=6.9Hz), 2.76-2.90 (1H, m), 4.34 (2H, s), 4.73 (2H, s), 6.92 (1H, dd, J=8.6Hz, 2.4Hz), 6.98-7.08 (2H, m), 7.18 (2H, d, J=8.5Hz), 7.24-7.43 (5H, m), 7.49 (2H, d, J=8.5Hz), 10.02 (1H, s)

### 2-[N-(4-Benzyloxy-2-butenyl)-N-(3-cyanophenyl)amino]-N-(4-isopropylphenyl)acetamide

¹H-NMR(CDCl₃) δ ppm:
   1.15-1.25 (6H, m), 2.80-2.94 (1H, m), 3.92-4.19 (6H, m), 4.48-4.60 (2H, m), 5.60-6.00 (2H, m), 6.92-7.42 (13H, m), 8.02 (1H, br-s)

### 2-[N-(3-Cyanophenyl)-N-cyclopropylmethylamino]-N-[4-(1-trifluoroacetylpiperidin-4-yl)phenyl]acetamide

¹H-NMR(DMSO-d₆) δ ppm:
   0.20-0.35 (2H, m), 0.40-0.55 (2H, m), 0.99-1.12 (1H, m), 1.49-1.66 (2H, m), 1.80-1.95 (2H, m), 2.75-3.02 (2H, m), 3.88-4.01 (1H, m), 4.25 (2H, s), 4.35-4.47 (1H, m), 6.92-7.10 (3H, m), 7.19 (2H, d, J=8.6Hz), 7.26-7.38 (1H, m), 7.50 (2H, d, J=8.6Hz), 9.93 (1H, s)

### 2-[N-(3-Cyanophenyl)-N-(3-methoxymethyloxybenzyl)amino]-N-[4-(1-trifluoroacetylpiperidin-4-yl)phenyl]acetamide

¹H-NMR(CDCl₃) δ ppm:
   1.58-1.75 (2H, m), 1.88-1.98 (2H, m), 2.72-2.90 (2H, m), 3.16-3.28 (1H, m), 3.44 (3H, s), 4.06-4.18 (3H, m), 4.62-4.73 (3H, m), 5.14 (2H, s), 6.82-6.94 (2H, m), 6.96-7.18 (6H, m), 7.25-7.40 (4H, m), 7.87 (1H, br-s)

### 2-[N-(4-Benzyloxy-2-butenyl)-N-(3-cyanophenyl)amino]-N-(4-(1-trifluoroacetylpiperidin-4-yl)phenyl]acetamide

¹H-NMR(CDCl₃) δ ppm:
   1.60-1.75 (2H, m), 1.88-2.00 (2H, m), 2.72-2.90 (2H, m), 3.14-3.30 (1H, m), 3.98 (2H, s), 4.08-4.20 (5H, m), 4.56 (2H, s), 4.63-4.75 (1H, m), 5.60-5.70 (1H, m), 5.88-5.99 (1H, m), 6.91-7.47 (13H, m), 8.08 (1H, br-s)

### 2-[N-(3-Chlorobenzyl)-N-(3-cyanophenyl]amino]-N-[4-(1-trifluoroacetylpiperidin-4-yl)phenyl]acetamide

¹H-NMR(DMSO-d₆) δ ppm:
   1.50-1.70 (2H, m), 1.80-2.00 (2H, m), 2.85-3.05 (2H, m), 3.90-4.00 (1H, m), 4.25-4.50 (3H, m), 4.73 (2H, s), 6.86-7.10 (3H, m), 7.15-7.60 (9H, m), 10.04 (1H, s)

### 2-[N-(2-Bromobenzyl)-N-(3-cyanophenyl)amino]-N-(4-isopropylphenyl)acetamide

¹H-NMR(DMSO-d₆) δ ppm:
   1.17 (6H, d, J=6.9Hz), 2.76-2.90 (1H, m), 4.35 (2H, s), 4.70 (2H, s), 6.84 (1H, dd, J=8.7Hz, 2.5Hz), 6.94-6.99 (1H, m), 7.05 (1H, d, J=7.5Hz), 7.13-7.40 (6H, m), 7.50 (2H, d, J=8.5Hz), 7.68 (1H, d, J=7.9Hz), 10.01 (1H, s)

### 2-[N-Benzyl-N-(3-cyanophenyl)amino]-N-[4-(1-tert-butoxycarbonylpiperidin-4-yloxy)phenyl]acetamide

¹H-NMR(CDCl₃) δ ppm:
   1.46 (9H, s), 1.66-1.78 (2H, m), 1.84-1.95 (2H, m), 3.27-3.36 (2H, m), 3.62-3.73 (2H, m), 4.11 (2H, s) 4.36-4.46 (1H, m), 4.72 (2H, s), 6.85 (2H, d, J=9.0Hz), 6.99-7.16 (3H, m), 7.19-7.43 (8H, m), 7.80 (1H, s)

### 2-[N-Benzyl-N-(3-cyanophenyl)amino]-N-(4-isopropoxyphenyl)acetamide

¹H-NMR(CDCl₃) δ ppm:
   1.30 (6H, d, J=6.1Hz), 4.11 (2H, s), 4.45-4.53 (1H, m), 4.71 (2H, s), 6.82 (2H, d, J=9.0Hz), 7.00-7.15 (3H, m), 7.19-7.42 (8H, m), 7.77 (1H, br-s)

### 2-[N-(3-Cyanophenyl)-N-(3-methoxycarbonylbenzyl)amino]-N-(4-isopropoxyphenyl)acetamide

¹H-NMR(CDCl₃) δ ppm:
   1.30 (6H, d, J=6.1Hz), 3.90 (3H, s), 4.12 (2H, s), 4.41-4.55 (1H, m), 4.75 (2H, s), 6.82 (2H, d, J=9.0Hz), 6.97-7.07 (2H, m), 7.13 (1H, d, J=7.6Hz), 7.27-7.49 (5H, m), 7.77 (1H, br-s), 7.92 (1H, s), 7.99 (1H, d, J=7.5Hz)

### 2-[N-(4-Chlorobenzyl)-N-(3-cyanophenyl)amino]-N-(4-isopropylphenyl)acetamide

¹H-NMR(DMSO-d₆) δ ppm:
   1.17 (6H, d. J=6.9Hz), 2.75-2.90 (1H, m), 4.31 (2H, s), 4.71 (2H, s), 6.92 (1H, dd, J=8.4Hz, 2.6Hz), 6.98-7.07 (2H, m), 7.17 (2H, d, J=8.6Hz), 7.25-7.45 (5H, m), 7.49 (2H, d, J=8.6Hz), 10.00 (1H, s)

### 2-[N-(2-Chlorobenzyl)-N-(3-cyanophenyl)amino]-N-(4-isopropylphenyl)acetamide

¹H-NMR(DMSO-d₆) δ ppm:
   1.17 (6H, d, J=6.9Hz), 2.75-2.90 (1H, m), 4.34 (2H, s), 4.75 (2H, s), 6.82-6.90 (1H, m), 6.95-7.01 (1H, m), 7.02-7.10 (1H, m), 7.18 (2H, d, J=8.7Hz), 7.22-7.38 (4H, m), 7.45-7.57 (3H, m), 10.02 (1H, s)

### 2-[N-(3-Cyanophenyl)-N-(3,4-dichlorobenzyl)amino]-N-(4-isopropylphenyl)acetamide

¹H-NMR(DMSO-d₆) δ ppm:
   1.17 (6H, d, J=6.9Hz), 2.75-2.90 (1H, m), 4.34 (2H, s), 4.73 (2H, s), 6.87-6.95 (1H, m), 6.98-7.08 (2H, m), 7.18 (2H, d, J=8.6Hz), 7.28-7.38 (2H, m), 7.49 (2H, d, J=8.6Hz), 7.58-7.66 (2H, m), 10.03 (1H, s)

### 2-[N-(3-Cyanophenyl)-N-cyclopropylmethylamino]-N-(4-isopropylphenyl)acetamide

¹H-NMR(DMSO-d₆) δ ppm:
   0.25-0.35 (2H, m), 0.43-0.55 (2H, m), 0.95-1.30 (7H, m), 2.75-2.90 (1H, m), 4.26 (2H, s), 6.92-7.13 (3H, m), 7.17 (2H, d, J=8.5Hz), 7.30-7.40 (1H, m), 7.49 (2H, d, J=8.5Hz), 9.93 (1H, s)

### 2-[N-(3-Cyanophenyl)-N-(3-methanesulfonylbenzyl)amino]-N-(4-isopropylphenyl)acetamide

¹H-NMR(DMSO-d₆) δ ppm:
   1.17 (6H, d, J=6.9Hz), 2.75-2.90 (1H, m), 3.19 (3H, s), 4.35 (2H, s), 4.84 (2H, s), 6.90-7.00 (1H, m), 7.01-7.10 (2H, m), 7.17 (2H, d, J=8.6Hz), 7.28-7.38 (1H, m), 7.49 (2H, d, J=8.6Hz), 7.58-7.72 (2H, m), 7.82 (1H, d, J=7.4Hz), 7.88 (1H, s), 10.04 (1H, s)

### 2-[N-(3-Cyanophenyl)-N-(3-sulfamoylbenzyl)amino]-N-(4-isopropylphenyl)acetamide

¹H-NMR(DMSO-d₆) δ ppm:
   1.17 (6H, d, J=6.9Hz), 2.75-2.90 (1H, m), 4.34 (2H, s), 4.81 (2H, s), 6.90-6.98 (1H, m), 7.00-7.10 (2H, m), 7.18 (2H, d, J=8.6Hz), 7.28-7.40 (3H, m), 7.45-7.60 (4H, m), 7.67-7.79 (2H, m), 10.03 (1H, s)

### 2-[N-Benzyl-N-(3-cyanophenyl)amino]-N-[4-(1-trifluoroacetylpiperidin-4-yl)phenyl]acetamide

¹H-NMR(DMSO-d₆) δ ppm:
   1.50-1.67 (2H, m), 1.80-1.95 (2H, m), 2.85-3.05 (2H, m), 3.26-3.42 (1H, m), 3.88-4.00 (1H, m), 4.31 (2H, s), 4.37-4.50 (1H, m), 4.72 (2H, s), 6.90-7.05 (3H, m), 7.15-7.40 (8H, m), 7.51 (2H, d, J=8.4Hz), 10.02 (1H, s)

### Reference Example 64

### 2-[N-(3-cyanophenyl)-N-(4-imidazolylmethyl)amino]-N-(4-isopropylphenyl)acetamide

2-[N-(1-tert-Butoxycarbonylimidazol-4-ylmethyl)-N-(3-cyanophenyl)amino]-N-(4-isopropylphenyl)acetamide (254 mg) was dissolved in 5 ml of methanol, 0.6 ml of 2 N hydrochloric acid was added to the solution, and the mixture was allowed to react at room temperature for 4.5 hours. Water was added to the reaction mixture, and the mixture was alkalized with a saturated aqueous sodium hydrogen carbonate solution. The precipitates were collected by filtration and the resulting residue was dissolved in dichloromethane. The solution was dried over anhydrous magnesium sulfate and the solvent was removed under reduced pressure to give 188 mg of 2-[N-(3-cyanophenyl)-N-(4-imidazolylmethyl)amino]-N-(4-isopropylphenyl)acetamide.
¹H-NMR(CDCl₃) δ ppm:
   1.22 (6H, d, J=6.9 Hz), 2.80-2.93 (1H, m), 4.23 (2H, s), 4.67 (2H, s), 6.88-6.95 (2H, m), 6.99 (1H, d, J=7.3Hz), 7.10 (1H, s), 7.17 (2H, d, J=8.5Hz), 7.20-7.31 (1H, m), 7.61 (2H, d, J=8.5Hz), 7.67 (1H, s), 9.17-9.29 (1H, m), 11.60 (1H, br-s)

### Reference Example 65

### 2-[N-(1-tert-Butoxycarbonylmethylimidazol-4-ylmethyl)-N-(3-cyanophenyl)amino]-N-(4-isopropylphenyl)acetamide

2-[N-(3-cyanophenyl)-N-(4-imidazolylmethyl)amino]-N-(4-isopropylphenyl)acetamide (80 mg), 38 µl of tert-butyl bromoacetate and 44 mg of potassium carbonate were suspended in 1 ml of N,N-dimethylformamide, and the suspension was allowed to react at room temperature for 3 hours. Water was added to the reaction mixture, and the mixture was extracted with diethyl ether. The organic layer was washed with water and brine, and dried over anhydrous magnesium sulfate. The solvent was removed under reduced pressure, and the resulting residue was purified by silica gel column chromatography (eluent: ethyl acetate-hexane) to give 90 mg of 2-[N-(1-tert-butoxycarbonylmethylimidazol-4-ylmethyl)-N-(3-cyanophenyl)amino]-N-(4-isopropylphenyl)acetamide.
¹H-NMR(DMSO-d₆) δ ppm:
   1.21 (6H, d, J=6.9Hz), 1.49 (9H, s), 2.80-2.91 (1H, m), 4.21 (2H, s), 4.60 (2H, s), 4.63 (2H, s), 6.87-7.03 (4H, m), 7.15 (2H, d, J=8.5Hz), 7.20-7.30 (1H, m), 7.50 (1H, d, J=1.2Hz), 7.59 (2H, d, J=8.5Hz), 11.61 (1H, br-s)

### Reference Example 66

The following compounds were prepared using the same procedure as Reference Example 65.

### 2-[N-(3-Cyanophenyl)-N-[1-(4-methylbenzyl)imidazol-4- ylmethyl]amino]-N-(4-isopropylphenyl)acetamide

¹H-NMR(CDCl₃) δ ppm:
   1.21 (6H, d, J=6.9Hz), 2.36 (3H, s), 2.77-2.93 (1H, m), 4.20 (2H, s), 4.60 (2H, s), 5.08 (2H, s), 6.85-6.94 (3H, m), 6.99 (1H, d, J=7.6Hz), 7.06 (2H, d, J=8.1Hz), 7.11-7.30 (5H, m), 7.53 (1H, s), 7.60 (2H, d, J=8.5Hz), 11.72 (1H, s)

### 2-[N-(3-Cyanophenyl)-N-[1-(4-hydroxybenzyl)imidazol-4-ylmethyl]amino]-N-(4-isopropylphenyl)acetamide

¹H-NMR(CDCl₃) δ ppm:
   1.21 (6H, d, J=6.9Hz), 2.80-2.90 (1H, m), 4.20 (2H, s), 4.59 (2H, s), 5.05 (2H, s), 5.22 (1H, br-s), 6.81-7.02 (6H, m), 7.06 (2H, d, J=8.6Hz), 7.16 (2H, d, J=8.6Hz), 7.19-7.30 (1H, m), 7.52 (1H, s), 7.60 (2H, d, J=8.6Hz), 11.71 (1H, s) MS(FAB, m/z) : 480 (M+H)

### Reference Example 67

### 2-[N-(3-Cyanophenyl)-N-(3-hydroxybenzyl)amino]-N-(4-isopropylphenyl)acetamide

2-[N-(3-Benzyloxybenzyl)-N-(3-cyanophenyl)amino]-N-(4-isopropylphenyl)acetamide (450 mg) was dissolved in 5 ml of ethanol, and 50 mg of 10 % palladium on carbon was added to the solution, and the mixture was stirred at atmospheric pressure under a hydrogen atmosphere at room temperature for 2 hours. The insoluble material was removed by filtration, and the filtrate was concentrated under reduced pressure to give 300 mg of 2-[N-(3-cyanophenyl)-N-(3-hydroxybenzyl)amino]-N-(4-isopropyl phenyl)acetamide.
¹H-NMR(CDCl₃) δ ppm:
   1.17 (6H, d, J=6.9Hz), 2.76-2.90 (1H, m), 4.28 (2H, s), 4.63 (2H, s), 6.59-6.76 (3H, m), 6.90-7.55 (9H, m), 9.38 (1H, s), 9.99 (1H, s)

### Reference Example 68

### 2-[N-(3-Cyanophenyl)-N-(3-methoxycarbonylmethyloxybenzyl)amino]-N-(4-isopropylphenyl)acetamide

2-[N-(3-Cyanophenyl)-N-(3-hydroxybenzyl)amino]-N-(4-isopropylphenyl)acetamide (300 mg) and 104 mg of potassium carbonate were added to 15 ml of acetonitrile, 0.08 ml of methyl bromoacetate was added dropwise to the mixture, and the mixture was stirred at 60 °C for 4 hours. The reaction mixture was acidified with dilute hydrochloric acid, and the mixture was extracted with ethyl acetate. The organic layer was washed successively with a saturated aqueous sodium hydrogen carbonate solution and brine, and dried over anhydrous magnesium sulfate. The solvent was removed under reduced pressure, and the resulting residue was purified by silica gel column chromatography (eluent: ethyl acetate-hexane) to give 312 mg of 2-[N-(3-cyanophenyl)-N-(3-methoxycarbonylmethyloxybenzyl)amino]-N-(4-isopropylphenyl)acetamide.
¹H-NMR(CDCl₃) δ ppm:
   1.21 (6H, d, J=6.9Hz), 2.80-2.95 (1H, m), 3.78 (3H, s), 4.11 (2H, s), 4.61 (2H, s), 4.68 (2H, s), 6.75-7.46 (12H, m), 7.83 (1H, br-s)

### Reference Example 69

### 2-[N-(3-Cyanophenyl)-N-[3-(2-hydroxyethyloxy)benzyl]amino]-N-[4-(4-piperidinyl)phenyl]acetamide

2-[N-(3-Cyanophenyl)-N-(3-ethoxycarbonylmethyloxybenzyl)amino]-N-[4-(1-trifluoroacetylpiperidin-4-yl)phenyl]acetamide (1.0 g), 287 mg of sodium borohydride and 320 mg of lithium chloride were suspended in 15 ml of tetrahydrofuran, 15 ml of ethanol was added to the suspension, and the mixture was allowed to react under an argon atmosphere at room temperature for 20 hours. Water was added to the reaction mixture, and the mixture was extracted with ethyl acetate. The organic layer was washed with brine and dried over anhydrous magnesium sulfate. The solvent was removed under reduced pressure, and the resulting residue was purified by aminopropylated silica gel column chromatography (eluent: methanol-dichloromethane) to give 372 mg of 2-[N-(3-cyanophenyl)-N-[3-(2-hydroxyethyloxy)benzyl]amino]-N-[4-(4-piperidinyl)phenyl]acetamide.
¹H-NMR(DMSO-d₆) δ ppm:
   1.38-1.42 (2H, m), 1.59-1.69 (2H, m), 2.94-3.04 (2H m), 3.63-3.73 (2H, m), 3.94 (2H, t, J=5.0Hz), 4.31 (2H, s), 4.68 (2H, s), 4.82 (1H, t, J=5.5Hz), 6.78-7.05 (6H, m), 7.15 (2H, d, J=8.5Hz), 7.21-7.35 (2H, m), 7.49 (2H, d, J=8.5Hz), 9.98 (1H, s)

### Reference Example 70

The following compound was prepared using the same procedure as Reference Example 69.

### 2-[N-(3-Cyanophenyl)-N-[3-(2-hydroxyethyloxy)benzyl]amino]-N-(4-isopropylphenyl)acetamide

¹H-NMR(CDCl₃) δ ppm;
   1.21 (6H, d, J=6.9Hz), 1.90-2.05 (1H, br), 2.80-2.95 (1H, m), 3.88-4.00 (2H, br), 4.01-4.08 (2H, m), 4.12 (2H, s), 4.68 (2H, s), 6.75-6.90 (3H, m), 6.97-7.20 (5H, m), 7.25-7.38 (4H, m), 7.83 (1H, br-s)

### Reference Example 71

### 2-[N-(4-Benzyloxybutyl)-N-(3-cyanophenyl)amino]-N-[4-(1-trifluoroacetylpiperidin-4-yl)phenyl]acetamide

2-[N-(4-Benzyloxy-2-butenyl)-N-(3-cyanophenyl)amino]-N-[4-(1-trifluoroacetylpiperidin-4-yl)phenyl]acetamide (350 mg) was dissolved in 20 ml of ethanol, 5 mg of 10 % palladium on carbon was added to the solution, and the mixture was stirred at atmospheric pressure under a hydrogen atmosphere at room temperature for 2 hours. The insoluble material was removed by filtration, and the filtrate was concentrated under reduced pressure. The resulting residue was purified by silica gel column chromatography (eluent: ethyl acetate-hexane) to give 330 mg of 2-[N-(4-benzyloxybutyl)-N-(3-cyanophenyl)amino]-N-[4-(1-trifluoroacetylpiperidin-4-yl)phenyl]acetamide.
¹H-NMR(CDCl₃) δ ppm:
   1.55-1.83 (6H, m), 1.88-2.00 (2H, m), 2.72-2.90 (2H, m), 3.16-3.27 (1H, m), 3.43-3.58 (4H, m), 3.98 (2H, s), 4.07-4.18 (1H, m), 4.51 (2H, s), 4.62-4.74 (1H, m), 6.88-7.18 (5H, m), 7.24-7.46 (8H, m), 7.97 (1H, br-s)

### Reference Example 72

The following compound was prepared using the same procedure as Reference Example 71.

### 2-[N-(4-Benzyloxybutyl)-N-(3-cyanophenyl)amino]-N-(4-isopropylphenyl)acetamide

¹H-NMR(CDCl₃) δ ppm:
   1.21 (6H, d, J=6.9Hz), 1.64-1.84 (4H, m), 2.79-2.93 (1H, m), 3.42-3.58 (4H, m), 3.98 (2H, s), 4.52 (2H, s), 6.91 (1H, dd, J=8.5Hz, 2.7Hz), 6.98-7.03 (1H, m), 7.08 (1H, d, J=7.6Hz), 7.16 (2H, d, J=8.5Hz), 7.24-7.40 (8H, m), 7.93 (1H, br-s)

### Reference Example 73

### 2-[N-(3-Cyanophenyl)-N-(1-naphthylmethyl)amino]-N-4-(1-trifluoroacetylpiperidin-4-yl)phenyl]acetamide

1-Hydroxymethylnaphthalene (2.0 g) and 6.3 g of carbon tetrabromide were dissolved in 20 ml of dichloromethane, 4.0 g of triphenylphosphine was added portionwise to the solution, and the mixture was stirred at room temperature for 7 minutes. The reaction mixture was concentrated under reduced pressure, and the resulting residue was purified by silica gel column chromatography (eluent: ethyl acetate-hexane) to give 1.4 g of a bromide compound. The bromide compound (724 mg) was dissolved in 10 ml of isopropanol, and 0.43 ml of N-ethyldiisopropylamine, 295 mg of sodium iodide and 705 mg of 2-(3-cyanophenylamino)-N-[4-(1-trifluoroacetylpiperidin-4-yl)phenyl]acetamide were added to the solution. After heating under reflux for 45 hours, the reaction mixture was concentrated under reduced pressure. Water and methanol were added to the resulting residue, the precipitates were collected by filtration to give 690 mg of 2-[N-(3-cyanophenyl)-N-(1-naphthylmethyl)amino]-N-[4-(1-trifluoroacetylpiperidin-4-yl)phenyl]acetamide.
¹H-NMR(DMSO-d₆) δ ppm:
   1.49-1.66 (2H, m), 1.79-1.96 (2H, m), 2.76-3.04 (2H,m), 3.87-4.01 (1H, m), 4.31 (2H, s), 4.37-4.48 (1H, m), 5.18 (2H, s), 6.92-7.10 (3H, m), 7.16-7.36 (4H, m), 7.40-7.64 (5H, m), 7.80-7.90 (1H, m), 7.94-8.09 (2H, m), 9.98 (1H, s)

### Reference Example 74

### 2-[N-(3-Cyanophenyl)-N-(5-quinoxalinylmethyl)amino]-N-[4-(1-trifluoroacetylpiperidin-4-yl)phenyl]acetamide

5-Methylquinoxaline (710 mg) and 920 mg of N-bromosuccinimide were dissolved in 8 ml of carbon tetrachloride, 50 mg of 2,2'-azobis(isobutyronitrile) was added to the solution, and the mixture was heated under reflux under an argon atmosphere for 5 hours. The reaction mixture was concentrated under reduced pressure, and the resulting residue was dissolved in 8 ml of isopropanol. To the solution were added 0.60 ml of N-ethyldiisopropylamine, 418 mg of sodium iodide and 1.0 g of 2-(3-cyanophenylamino)-N-[4-(1-trifluoroacetylpiperidin-4-yl)phenyl]acetamide, and the mixture was heated under reflux for 45 hours. The reaction mixture was concentrated under reduced pressure, and the resulting residue was purified by silica gel column chromatography (eluent: ethyl acetate-hexane) to give 560 mg of 2-[N-(3-cyanophenyl)-N-(5-quinoxalinylmethyl)amino]-N-[4-(1-trifluoroacetylpiperidin-4-yl)phenyl]acetamide.
¹H-NMR(CDCl₃) δ ppm:
   1.56-1.72 (2H, m), 1.85-2.00 (2H, m), 2.70-2.93 (2H, m), 3.16-3.30 (1H, m), 4.05-4.17 (1H, m), 4.21 (2H, s), 4.60-4.72 (1H, m), 5.33 (2H, s), 7.00-7.19 (5H, m), 7.23-7.38 (3H, m), 7.53-7.61 (1H, m), 7.70-7.80 (1H, m), 8.05-8.26 (2H, m), 8.80-8.94 (2H, m)

### Example 1

### 2-(5-Amidino-2-fluorophenylamino)-N-(4-isopropoxyphenyl)acetamide hydrochloride (Compound 1)

A saturated hydrogen chloride ethanol solution (20 ml) was added to 26 mg of 2-(5-cyano-2-fluorophenylamino)-N-(4-isopropoxyphenyl)acetamide, and the mixture was sealed and allowed to react for 5 hours. The reaction mixture was concentrated under reduced pressure, 50 ml of a saturated ammonia methanol solution was added to the resulting residue, and the mixture was stirred for 12 hours. The reaction mixture was concentrated under reduced pressure, the resulting residue was separated by aminopropylated silica gel column chromatography (eluent: 25 % aqueous ammonia solution-methanol-dichloromethane), and the desired fraction was concentrated under reduced pressure. Methanol was added to the resulting residue, and the mixture was acidified with dilute hydrochloric acid. The solvent was removed under reduced pressure to give 19 mg of 2-(5-amidino-2-fluorophenylamino)-N-(4-isopropoxyphenyl)acetamide hydrochloride.
¹H-NMR(DMSO-d₆) δ ppm:
   1.23 (6H, d, J=6.0Hz), 4.04 (2H, s), 4.46-4.58 (1H, m), 6.10-6.35 (1H, br), 6.85 (2H, d, J=9.1Hz), 7.05-7.10 (1H, m), 7.14 (1H, dd, J=8.1Hz, 2.1Hz), 7.31 (1H, dd, J=11.5Hz, 8.4Hz), 7.49 (2H, d, J=9.1Hz), 8.93 (2H, s), 9.25 (2H, s), 10.04 (1H, s)

### Example 2

The following compounds were prepared using the same procedure as Example 1.

### 2-[N-(3-Amidinophenyl)-N-[3-(2-hydroxyethyloxy)benzyl]-amino]-N-[4-(4-piperidinyl)phenyl]acetamide dihydrochloride (Compound 2)

¹H-NMR(DMSO-d₆) δ ppm:
   1.73-1.98 (4H, m), 2.73-2.86 (1H, m), 2.90-3.05 (2H, m), 3.69 (2H, t, J=5.0Hz), 3.95 (2H, t, J=5.0Hz), 4.38 (2H, s), 4.70-5.00 (3H, m), 6.80-7.40 (10H, m), 7.58 (2H, d, J=8.6Hz), 8.65-9.00 (4H, m), 9.27 (2H, s), 10.24 (1H, s) MS(FAB, m/z): 502(M+H)

### 2-[N-(3-Amidinophenyl)-N-(3-hydroxybenzyl)amino]-N-[4-(4-piperidinyl)phenyl]acetamide dihydrochloride (Compound 3)

¹H-NMR(DMSO-d₆) δ ppm:
   1.73-1.95 (4H, m), 2.71-2.85 (1H, m), 2.90-3.05 (2H, m), 4.34 (2H, s), 4.67 (2H, s), 6.60-6.75 (3H, m), 6.92-7.20 (6H, m), 7.30-7.40 (1H, m), 7.53-7.61 (2H, m), 8.70-9.00 (4H, m), 9.27 (2H, s), 9.37 (1H, br-s), 10.24 (1H, s)

### 2-[N-(3-Amidinophenyl)-N-(4-benzyloxy-2-butenyl)amino]-N-(4-isopropylphenyl]acetamide hydrochloride (Compound 4)

¹H-NMR(DMSO-d₆) δ ppm:
   1.17 (6H, d, J=6.9Hz), 2.75-2.90 (1H, m), 3.90-4.53 (8H, m), 5.64-5.84 (2H, m), 6.93-7.10 (3H, m), 7.16 (2H, d, J=8.6Hz), 7.23-7.40 (6H, m), 7.50 (2H, d, J=8.6Hz), 8.89 (2H, s), 9.28 (2H, s), 10.07 (1H, s)

### 2-[N-(3-Amidinophenyl)-N-(4-benzyloxybutyl)amino]-N-(4-isopropylphenyl)acetamide hydrochloride (Compound 5)

¹H-NMR(DMSO-d₆) δ ppm:
   1.17 (6H, d, J=6.9Hz), 1.55-1.73 (4H, m), 2.77-2.88 (1H, m), 3.41-3.52 (4H, m), 4.23 (2H, s), 4.46 (2H, s), 6.91-7.06 (3H, m), 7.16 (2H, d, J=8.5Hz), 7.22-7.37 (6H, m), 7.51 (2H, d, J=8.5Hz), 8.94 (2H, s), 9.27 (2H, s), 10.12 (1H, s)

### 2-[N-(3-Amidinophenyl)-N-(5-methylimidazol-4-ylmethyl)amino]-N-[4-(4-piperidinyl)phenyl]acetamide trihydrochloride (Compound 6)

¹H-NMR(DMSO-d₆) δ ppm:
   1.74-1.97 (4H, m), 2.31 (3H, s), 2.70-2.85 (1H, m), 2.88-3.05 (2H, m), 4.42 (2H, s), 4.80 (2H, s), 7.05 (1H, d, J=8.6Hz), 7.10-7.21 (3H, m), 7.26 (1H, s), 7.35-7.45 (1H, m), 7.59 (2H, d, J=8.6Hz), 8.80-9.17 (5H, m), 9.40 (2H, s), 10.64 (1H, s), 14.30-14.55 (2H, m)

### 2-[N-(3-Amidinophenyl)-N-[3-(2-hydroxyethyloxy)benzyl]amino]-N-(4-isopropylphenyl)acetamide hydrochloride (Compound 7)

¹H-NMR(DMSO-d₆) δ ppm:
   1.17 (6H, d, J=6.9Hz), 2.78-2.89 (1H, m), 3.64-3.71 (2H, m), 3.90-3.96 (2H, m), 4.34 (2H, s), 4.71 (2H, s), 6.80-7.40 (10H, m), 7.50 (2H, d, J=8.5Hz), 8.86 (2H, s), 9.24 (2H, s), 10.08 (1H, s)

### 2-[N-(3-Amidinophenyl)-N-(3-carbamoylmethyloxybenzyl)amino]-N-(4-isopropylphenyl)acetamide hydrochloride (Compound 8)

¹H-NMR(DMSO-d₆) δ ppm:
   1.17 (6H, d, J=6.9Hz), 2.78-2.89 (1H, m), 4.35 (2H, s), 4.39 (2H, s), 4.72 (2H, s), 6.80-7.56 (14H, m), 8.90 (2H, s), 9.26 (2H, s), 10.13 (1H, s)
MS(FAB, m/z): 474(M+H)

### 2-[N-(3-Amidinophenyl)-N-(1-carbamoylmethylimidazol-4-ylmethyl)amino]-N-(4-isopropylphenyl)acetamide dihydrochloride (Compound 9)

¹H-NMR(DMSO-d₆) δ ppm:
   1.17 (6H, d, J=6.9 Hz), 2.77-2.91 (1H, m), 4.42 (2H, s), 4.85 (2H, s), 4.90 (2H, s), 6.97-7.27 (5H, m), 7.35-7.69 (5H, m), 7.85 (1H, s), 8.85-9.15 (3H, m), 9.32 (2H, s), 10.35-10.70 (1H, br), 14.50-15.05 (1H, br)
MS(FAB, m/z): 448(M+H)

### 2-[N-(3-Amidinophenyl)-N-(3-sulfamoylbenzyl)amino]-N-(4-isopropylphenyl)acetamide hydrochloride (Compound 10)

¹H-NMR(DMSO-d₆) δ ppm:
   1.17 (6H, d, J=6.9Hz), 2.78-2.89 (1H, m), 4.36 (2H, s), 4.84 (2H, s), 6.90-7.80 (14H, m), 8.81 (2H, br-s), 9.23 (2H, br-s), 10.09 (1H, br-s)

### 2-[N-(3-Amidinophenyl)-N-(3-methanesulfonylbenzyl)amino]-N-(4-isopropylphenyl)acetamide hydrochloride (Compound 11)

¹H-NMR(DMSO-d₆) δ ppm:
   1.17 (6H, d, J=6.9Hz), 2.78-2.89 (1H, m), 3.19 (3H, s), 4.39 (2H, s), 4.87 (2H, s), 6.93-7.20 (5H, m), 7.32-7.41 (1H, m), 7.50 (2H, d, J=8.8Hz), 7.60-7.72 (2H, m), 7.78-7.92 (2H, m), 8.83 (2H, br-s), 9.23 (2H, br-s), 10.12 (1H, br-s)

### 2-[N-(3-Amidinophenyl)-N-(1-propylimidazol-4-ylmethyl)amino]-N-(4-isopropylphenyl)acetamide dihydrochloride (Compound 12)

¹H-NMR(DMSO-d₆) δ ppm:
   0.81 (3H, t, J=7.4Hz), 1.17 (6H, d, J=6.9Hz), 1.69-1.84 (2H, m), 2.76-2.90 (1H, m), 4.09 (2H, t, J=7.1Hz), 4.42 (2H, s), 4.84 (2H, s), 6.99-7.09 (1H, m), 7.10-7.28 (4H, m), 7.32-7.47 (1H, m), 7.53 (2H, d, J=8.5Hz), 7.69 (1H, s), 8.93-9.15 (3H, m), 9.35 (2H, s), 10.40-10.70 (1H, br), 14.61-14.92 (1H, br)

### 2-[N-(3-Amidinophenyl)-N-(1-methylimidazol-4-ylmethyl)amino]-N-(4-isopropylphenyl)acetamide dihydrochloride (Compound 13)

¹H-NMR(DMSO-d₆) δ ppm:
   1.17 (6H, d, J=6.9Hz), 2.76-2.91 (1H, m), 3.81 (3H, s), 4.41 (2H, br-s), 4.83 (2H, s), 6.99-7.23 (5H, m), 7.32-7.46 (1H, m), 7.47-7.66 (3H, m), 8.82-9.10 (3H, m), 9.34 (2H, s), 10.37-10.60 (1H, br), 14.40-14.75 (1H, br)
MS(FAB, m/z): 405(M+H)

### 2-[N-(3-Amidinophenyl)-N-cyclopropylmethylamino]-N-(4-isopropylphenyl)acetamide hydrochloride (Compound 14)

¹H-NMR(DMSO-d₆) δ ppm:
   0.25-0.35 (2H, m), 0.45-0.55 (2H, m), 1.00-1.20 (7H, m), 2.75-2.90 (1H, m), 4.32 (2H, s), 6.90-7.55 (8H, m), 8.85-9.05 (2H, m), 9.27 (2H, br-s), 9.97-10.20 (1H, m)

### 2-[N-(3-Amidinophenyl)-N-(3,4-dichlorobenzyl)amino]-N-(4-isopropylphenyl)acetamide hydrochloride (Compound 15)

¹H-NMR(DMSO-d₆) δ ppm:
   1.17 (6H, d, J=6.9Hz), 2.76-2.90 (1H, m), 4.39 (2H, s), 4.77 (2H, s), 6.92 (1H, dd, J=7.4Hz, 2.1Hz). 7.00-7.40 (6H, m), 7.52 (2H, d, J=8.5Hz), 7.56-7.66 (2H, m), 8.93 (2H, s), 9.28 (2H, s), 10.22 (1H, s)

### 2-[N-(3-Amidinophenyl)-N-(2-chlorobenzyl)amino]-N-(4-isopropylphenyl)acetamide hydrochloride (Compound 16)

¹H-NMR(DMSO-d₆) δ ppm:
   1.17 (6H, d, J=6.9Hz), 2.76-2.90 (1H, m), 4.38 (2H, s), 4.80 (2H, s), 6.89 (1H, dd, J=8.5Hz, 2.1Hz), 7.00-7.40 (8H, m), 7.45-7.57 (3H, m), 8.90 (2H, s), 9.28 (2H, s), 10.17 (1H, s)

### 2-[N-(3-Amidinophenyl)-N-(3-methyl-2-butenyl)amino]-N-[4-(4-piperidinyl)phenyl]acetamide dihydrochloride (Compound 17)

¹H-NMR(DMSO-d₅) δ ppm:
   1.71 (6H, s), 1.75-1.95 (4H, m), 2.70-3.05 (3H, m), 3.25-3.40 (2H, m), 4.06 (2H d, J=5.8Hz), 4.22 (2H, s), 5.21-5.30 (1H, m), 6.92-7.20 (5H, m), 7.30-7.40 (1H, m), 7.56 (2H, d, J=8.5Hz), 8.70-9.03 (4H, m), 9.27 (2H, s), 10.18 (1H, s)

### 2-[N-(3-Amidinophenyl)-N-(4-imidazolylmethyl)amino]-N-(4-isopropoxyphenyl)acetamide dihydrochloride (Compound 18)

¹H-NMR(DMSO-d₆) δ ppm:
   1.24 (6H, d, J=6.0Hz), 4.35-4.62 (3H, m), 4.87 (2H, s), 6.88 (2H, d, J=9.0Hz), 7.00-7.45 (4H, m), 7.53 (2H, d, J=9.0Hz), 7.63 (1H, s), 8.87-9.15 (3H, m), 9.38 (2H, s), 10.48 (1H, s), 13.90-14.90 (2H, br)

### 2-[N-(3-Amidinophenyl)-N-(5-methylimidazol-4-ylmethyl)amino]-N-(4-isopropoxyphenyl)acetamide dihydrochloride (Compound 19)

¹H-NMR(DMSO-d₆) δ ppm:
   1.23 (6H, d, J=6.0Hz), 2.31 (3H, s), 4.39 (2H, s), 4.46-4.59 (1H, m), 4.79 (2H, s), 6.87 (2H, d, J=9.0Hz), 7.00-7.25 (3H, m), 7.35-7.45 (1H, m), 7.50 (2H, d, J=9.0Hz), 8.85-9.10 (3H, m), 9.35 (2H, s), 10.43 (1H, br-s), 14.20-14.55 (2H, m) MS(FAB, m/z): 421(M+H)

### 2-[N-(3-Amidinophenyl)-N-(3-methoxycarbonylbenzyl)amino]-N-(4-isopropoxyphenyl)acetamide hydrochloride (Compound 20)

¹H-NMR(DMSO-d₆) δ ppm:
   1.23 (6H, d, J=6.0Hz), 3.83 (3H, s), 4.35 (2H, s), 4.47-4.60 (1H, m), 4.82 (2H, s), 6.85 (2H, d, J=9.1Hz), 6.98 (1H, dd, J=8.8Hz, 2.1Hz), 7.02-7.12 (2H, m), 7.30-7.40 (1H, m), 7.45-7.67 (4H, m), 7.82-7.94 (2H, m), 8.84 (2H, s), 9.23 (2H, s), 10.01 (1H, s)

### 2-[N-(3-Amidinophenyl)-N-(2-methylallyl)amino]-N-(4-isopropoxyphenyl)acetamide hydrochloride (Compound 21)

¹H-NMR(DMSO-d₆) δ ppm:
   1.23 (6H, d, J=6.0Hz), 1.73 (3H, s), 4.01 (2H, s), 4.21 (2H, s), 4.47-4.60 (1H, m), 4.77 (1H, br-s), 4.84 (1H, br-s), 6.85 (2H, d, J=9.1Hz), 6.89-7.06 (3H, m), 7.30-7.40 (1H, m), 7.48 (2H, d, J=9.1Hz), 8.89 (2H, s), 9.25 (2H, s), 10.02 (1H, s)

### 2-[N-(3-Amidinophenyl)-N-(3-methyl-2-butenyl)amino]-N-(4-isopropoxyphenyl)acetamide hydrochloride (Compound 22)

¹H-NMR(DMSO-d₆) δ ppm:
   1.23 (6H, d, J=6.0Hz), 1.71 (6H, s), 4.05 (2H, d, J=6.5Hz), 4.17 (2H, s), 4.45-4.59 (1H, m), 5.20-5.30 (1H, m), 6.84 (2H, d, J=9.0Hz), 6.90-7.10 (3H, m), 7.30-7.40 (1H, m), 7.47 (2H, d, J=9.0Hz), 8.90 (2H, s), 9.25 (2H, s), 9.97 (1H, s)

### 2-[N-Allyl-N-(3-amidinophenyl)amino]-N-(4-isopropoxyphenyl)acetamide hydrochloride (Compound 23)

¹H-NMR(DMSO-d₆) δ ppm:
   1.22 (6H, d, J=6.0Hz), 4.12 (2H, d, J=4.7Hz), 4.21 (2H, s), 4.46-4.60 (1H, m), 5.10-5.28 (2H, m), 5.85-6.00 (1H, m), 6.85 (2H, d, J=9.1Hz), 6.96 (1H, dd, J=8.5Hz, 2.1Hz), 7.00-7.10 (2H, m), 7.30-7.40 (1H, m), 7.50 (2H, d, J=9.1Hz), 8.98 (2H, s), 9.29 (2H, s), 10.09 (1H, s)

### 2-[N-(3-Amidinophenyl)-N-methylamino]-N-(4-isopropylphenyl)-acetamide hydrochloride (Compound 24)

¹H-NMR(DMSO-d₆) δ ppm:
   1.16 (6H, d, J=6.9 Hz), 2.77-2.88 (1H, m), 3.09 (3H, s), 4.27 (2H, s), 6.97-7.12 (3H, m), 7.15 (2H, d, J=8.6Hz), 7.32-7.42 (1H, m), 7.50 (2H, d, J=8.6Hz), 8.95 (2H, br-s), 9.29 (2H, br-s), 10.13 (1H, s)

### 2-[N-(3-Amidinophenyl)-N-methylamino]-N-(4-isopropoxyphenyl)acetamide hydrochloride (Compound 25)

¹H-NMR(DMSO-d₆) δ ppm:
   1.22 (6H, d, J=6.0Hz), 3.09 (3H, s), 4.25 (2H, s), 4.46-4.58 (1H, m), 6.84 (2H, d, J=9.0Hz), 6.96-7.13 (3H, m), 7.32-7.42 (1H, m), 7.48 (2H, d, J=9.0Hz), 8.97 (2H, s), 9.30 (2H, s), 10.07 (1H, s)

### 2-[N-(3-Amidinophenyl)-N-(4-ethylbenzyl)amino]-N-(4-isopropoxyphenyl)acetamide hydrochloride (Compound 26)

¹H-NMR(DMSO-d₆) δ ppm:
   1.16 (3H, t, J=7.6Hz), 1.23 (6H, d, J=6.0Hz), 4.27 (2H, s), 4.48-4.58 (1H, m), 4.70 (2H, s), 6.85 (2H, d, J=9.0Hz), 6.95-7.24 (7H, m), 7.30-7.40 (1H, m), 7.46 (2H, d, J=9.0Hz), 8.77 (2H, s), 9.20 (2H, s), 9.90 (1H, s)

### 2-(5-Amidino-2-hydroxymethylphenylamino)-N-[4-(4-piperidinyloxy)phenyl]acetamide dihydrochloride (Compound 27)

¹H-NMR(DMSO-d₆) δ ppm:
   1.72-1.87 (2H, m), 1.99-2.14 (2H, m), 2.96-3.28 (4H, m), 4.04 (2H, s), 4.43-4.76 (3H, m), 6.85-7.01 (4H, m), 7.36 (1H, d, J=7.8Hz), 7.54 (2H, d, J=9.0Hz), 8.65-9.05 (4H, m), 9.25 (2H, s), 10.14 (1H, s)
MS(FAB, m/z): 398(M+H)

### 2-(5-Amidino-2-benzyloxyphenylamino)-N-[4-(4-piperidinyloxy)phenyl]acetamide dihydrochloride (Compound 28)

¹H-NMR(DMSO-d₆) δ ppm:
   1.75-1.90 (2H, m), 2.02-2.12 (2H, m), 2.95-3.10 (2H, m), 3.13-3.26 (2H, m), 4.07 (2H, s), 4.52-4.62 (1H, m), 5.29 (2H, s), 6.95 (2H, d, J=9.1Hz), 7.03-7.20 (3H, m), 7.30-7.47 (3H, m), 7.49-7.62 (4H, m), 8.85-9.30 (6H, m), 10.31 (1H, s)

### 2-[N-(3-Amidinophenyl)-N-benzylamino]-N-[4-(4-piperidinyloxy)phenyl]acetamide dihydrochloride (Compound 29)

¹H-NMR(DMSO-d₆) δ ppm:
   1.73-1.85 (2H, m), 2.01-2.12 (2H, m), 2.98-3.11 (2H, m), 3.15-3.28 (2H, m), 4.33 (2H, s), 4.51-4.61 (1H, m), 4.75 (2H, s), 6.88-7.14 (5H, m), 7.21-7.39 (6H, m), 7.53 (2H, d, J=9.0Hz), 8.65-8.98 (4H, m), 9.61 (2H, s), 10.14 (1H, s)

### 2-[N-(3-Amidinophenyl)-N-benzylamino]-N-(4-isopropoxyphenyl]acetamide hydrochloride (Compound 30)

¹H-NMR(DMSO-d₆) δ ppm:
   1.23 (6H, d, J=6.0Hz), 4.32 (2H, s), 4.45-4.60 (1H, m), 4.75 (2H, s), 6.85 (2H, d, J=9.0Hz), 6.92-7.13 (3H, m), 7.23-7.38 (6H, m), 7.48 (2H, d, J=9.0Hz), 8.86 (2H, s), 9.24 (2H, s), 10.02 (1H, s)

### 2-(5-Amidino-2-methylphenylamino)-N-[4-(4-piperidinyloxy)phenly]acetamide dihydrochloride (Compound 31)

¹H-NMR(DMSO-d₆) δ ppm:
   1.75-1.88 (2H, m), 2.02-2.15 (2H, m), 2.24 (3H, s), 2.97-3.11 (2H, m), 3.15-3.28 (2H, m), 4.50-4.60 (1H, m), 6.94-7.06 (4H, m), 7.17-7.27 (1H, m), 7.55 (2H, d, J=9.0Hz), 8.80-9.12 (4H, m), 9.22 (2H, s), 10.20(1H, s)

### 2-(5-Amidino-2-chlorophenylamino)-N-[4-(4-piperidinyloxy)phenyl]acetamide dihydrochloride (Compound 32)

¹H-NMR(DMSO-d₆) δ ppm:
   1.74-1.87 (2H, m), 2.01-2.15 (2H, m), 2.98-3.11 (2H, m), 3.15-3.29 (2H, m), 4.13 (2H, s), 4.52-4.62 (1H, m), 5.95-6.30 (1H, br), 6.95 (2H, d, J=9.1Hz), 7.00-7.12 (2H, m), 7.48-7.60 (3H, m), 8.80-9.15 (4H, m), 9.37 (2H, s), 10.26 (1H, s)

### 2-(3-Amidinophenylamino)-N-[4-(4-piperidinyloxy)phenyl]acetamide dihydrochloride (Compound 33)

¹H-NMR(DMSO-d₆) δ ppm:
   1.74-1.87 (2H, m), 2.02-2.11 (2H, m), 3.00-3.10 (2H, m), 3.15-3.27 (2H, m), 3.96 (2H, s), 6.90-7.00 (5H, m), 7.25-7.35 (1H, m), 7.53 (2H, d, J=9.0Hz), 8.70-8.95 (4H, m), 9.23 (2H, s), 10.10 (1H, s)

### 2-(3-Amidinophenylamino)-N-(4-isopropylphenyl)acetamide hydrochloride (Compound 34)

¹H-NMR(DMSO-d₆) δ ppm:
   1.17 (6H, d, J=6.9Hz), 2.75-2.90 (1H, m), 3.98 (2H, s), 6.91-7.05 (3H, m), 7.16 (2H, d, J=8.5Hz), 7.27-7.37 (1H, m), 7.53 (2H, d, J=8.5Hz), 8.98 (2H, s), 9.25 (2H, s), 10.15 (1H, s)

### Example 3

The following compounds in a free form were prepared using the same procedure as Example 1.

### 2-[N-(3-Amidinophenyl)-N-(5-quinoxalinylmethyl)amino]-N-[4-(4-piperidinyl)phenyl]acetamide (Compound 35)

¹H-NMR(DMSO-d₆) δ ppm:
   1.35-1.70 (4H, m), 2.92-3.06 (2H, m), 5.36 (2H, s), 6.70 (1H, dd, J=8.4Hz, 2.3Hz), 6.98 (1H d, J=7.6Hz), 7.02-7.23 (4H, m), 7.50 (2H, d, J=8.5Hz), 7.68 (1H, d, J=7.1Hz), 7.76-7.85 (1H, m), 8.00 (1H, d, J=8.4Hz), 8.95-9.05 (2H, m), 9.55-10.35 (1H, br)

### 2-[N-(3-Amidinophenyl)-N-(3-chlorobenzyl)amino]-N-[4-(4-piperidinyl)phenyl]acetamide (Compound 36)

¹H-NMR(DMSO-d₆) δ ppm:
   1.40-1.80 (4H, m), 2.65-2.75 (2H, m), 3.05-3.15 (2H, m), 4.37 (2H, s), 4.77 (2H, s), 6.70-7.70 (16H, m), 10.13 (1H, s)

### 2-[N-(3-Amidinophenyl)-N-cyclopropylmethylamino]-N-[4-(4-piperidinyl)phenyl]acetamide (Compound 37)

¹H-NMR(DMSO-d₆) δ ppm:
   0.25-0.35 (2H, m), 0.45-0.55 (2H, m), 1.05-1.20 (1H, m), 1.45-1.60 (2H, m), 1.62-1.75 (2H, m), 3.00-3.10 (2H, m), 4.32 (2H, s), 6.90-7.90 (12H, m), 10.04 (1H, s)

### 2-[N-(3-Amidinophenyl)-N-(3-chlorobenzyl)amino]-N-(4-isopropylphenyl)acetamide (Compound 38)

¹H-NMR(DMSO-d₆) δ ppm:
   1.17 (6H, d, J=6.9Hz), 2.78-2.89 (1H, m), 4.38 (2H, s), 4.77 (2H, s), 6.90-7.55 (15H, m), 10.15 (1H, s)

### 2-[N-(3-Amidinophenyl)-N-benzylamino]-N-[4-(4-piperidinyl)phenyl]acetamide (Compound 39)

¹H-NMR(DMSO-d₆) δ ppm:
   1.40-1.55 (2H, m), 1.60-1.70 (2H, m), 2.52-2.65 (2H, m), 2.95-3.10 (2H, m), 4.33 (2H, s), 4.75 (2H, s), 6.90-7.20 (5H, m), 7.23-7.40 (6H, m), 7.51 (2H, d, J=8.5Hz), 10.07 (1H, s)

### 2-[N-(3-Amidinophenyl)-N-(4-chlorobenzyl)amino]-N-(4-isopropylphenyl)acetamide (Compound 40)

¹H-NMR(DMSO-d₆) δ ppm:
   1.17 (6H, d, J=6.9Hz), 2.76-2.90 (1H, m), 4.33 (2H, s), 4.74 (2H, s), 6.90-7.10 (3H, m), 7.17 (2H, d, J=8.9Hz), 7.30-7.66 (7H, m), 8.40-9.40 (3H, m), 10.00-10.20 (1H, m)

### Example 4

### 2-[N-(3-Amidinophenyl)-N-(3-carbamoylmethyloxybenzyl)amino]-N-[4-(4-piperidinyl)phenyl]acetamide dihydrochloride (Compound 41)

### 2-[N-(3-Amidinophenyl)-N-(3-methoxycarbonylmethyloxybenzyl)amino]-N-[4-(4-piperidinyl)phenyl]acetamide dihydrochloride (Compound 42)

To 3.0 g of 2-[N-(3-cyanophenyl)-N-(3-ethoxycarbonylmethyloxybenzyl)amino]-N-[4-(1-trifluoroacetylpiperidin-4-yl)phenyl]acetamide was added 100 ml of a saturated hydrogen chloride methanol solution, and the mixture was sealed and allowed to react at room temperature for 5 hours. The reaction mixture was concentrated under reduced pressure to give an imidate compound. The imidate compound (1.0 g) was dissolved in 25 ml of a saturated ammonia methanol solution. After stirring at room temperature for 15 hours, the reaction mixture was concentrated under reduced pressure. The resulting residue was separated by octadesyl-bounded silica gel medium pressure liquid column chromatography (eluent: water-methanol), and the desired fraction obtained was acidified with dilute hydrochloric acid. The solvent was removed under reduced pressure to give 423 mg of the Compound 41.

Ethyl acetate and dichloromethane were added to the imidate compound that was the remainders of the preceding reaction, and the mixture was washed with a saturated aqueous sodium hydrogen carbonate solution and dried over anhydrous magnesium sulfate. The solvent was removed under reduced pressure, and the resulting residue was dissolved in 20 ml of methanol. A solution of 172 mg of ammonium chloride in 0.69 ml of water was added to the methanol solution, and the mixture was heated under reflux for 6 hours. The reaction mixture was concentrated under reduced pressure, and the resulting residue was separated by octadesyl-bounded silica gel medium pressure liquid column chromatography (eluent: water-methanol). The desired fraction obtained was acidified with dilute hydrochloric acid, and the solvent was removed under reduced pressure. The resulting residue were dissolved in 5 ml of methanol. Water (10 ml), 5 ml of acetonitrile and 1.0 g of potassium carbonate were added to the solution, and the mixture was allowed to react at room temperature for 15 hours. The reaction mixture was acidified with dilute hydrochloric acid and the mixture was concentrated under reduced pressure. Methanol was added to the resulting residue, and the insoluble material was removed through Celite®. The solvent was removed under reduced pressure, and the resulting residue was dissolved in 10 ml of methanol. A small amount of a saturated hydrogen chloride methanol solution was added to the solution, and the mixture was allowed to react at room temperature for 8 hours. The reaction mixture was concentrated under reduced pressure, and the resulting residue was separated by octadesyl-bounded silica gel medium pressure liquid column chromatography (eluent: water-methanol). The desired fraction obtained was acidified by dilute hydrochloric acid, and the solvent was removed under reduced pressure to give 418 mg of the Compound 42.

### Compound 41

¹H-NMR(DMSO-d₆) δ ppm:
   1.71-1.96 (4H, m), 2.71-2.84 (1H, m), 2.90-3.02 (2H, m), 4.30-4.43 (4H, m), 4.72 (2H, s), 6.80-7.40 (11H, m), 7.46-7.61 (3H, m), 8.62-9.07 (4H, m), 9.27 (2H, br-s), 10.23 (1H, s)
MS(FAB, m/z): 515(M+H)

### Compound 42

¹H-NMR(DMSO-d₆) δ ppm:
   1.73-2.00 (4H, m), 2.72-2.85 (1H, m), 2.90-3.04 (2H, m), 3.66 (3H, s), 4.37 (2H, s), 4.77-4.88 (4H, m), 6.77-7.40 (10H, m), 7.52-7.70 (2H, m), 8.60-9.55 (6H, m), 10.30 (1H, s)
MS(FAB, m/z): 530(M+H)

### Example 5

### 2-[N-(3-Amidinophenyl)-N-(1-methoxycarbonylmethylimidazol-4-ylmethyl)amino]-N-(4-isopropylphenyl)acetamide hydrochloride (Compound 43)

To 90 mg of 2-[N-(1-tert-butoxycarbonylmethylimidazol-4-ylmethyl)-N-(3-cyanophenyl)amino]-N-(4-isopropylphenyl)acetamide was added 2 ml of a saturated hydrogen chloride methanol solution, and the mixture was sealed and allowed to react at 0 °C for 36 hours. The reaction mixture was concentrated under reduced pressure, and the resulting residue was alkalized with a saturated aqueous sodium hydrogen carbonate solution and extracted with ethyl acetate. The organic layer was dried over anhydrous magnesium sulfate, and the solvent was removed under reduced pressure. The resulting residue was dissolved in 5 ml of methanol, a solution of 21 mg of ammonium chloride in 81 µl of water was added to the solution, and the mixture was heated under reflux for 8 hours. The reaction mixture was concentrated under reduced pressure, and dichloromethane was added to the resulting residue. The insoluble material was removed by filtration, and the filtrate was concentrated under reduced pressure. The resulting residue was suspended in diethyl ether and a small amount of ethanol, and the resulting insoluble material was collected by filtration and washed with diethyl ether and a small amount of methanol to give 45 mg of 2-[N-(3-amidinophenyl)-N-(1-methoxycarbonylmethylimidazol-4-ylmethyl)amino]-N-(4-isopropylphenyl)acetamide hydrochloride.
¹H-NMR(DMSO-d₆) δ ppm:
   1.16 (6H, d, J=6.8Hz), 2.75-2.90 (1H, m), 3.69 (3H, s), 4.32 (2H, s), 4.68 (2H, s), 5.00 (2H, s), 6.87-7.40 (7H, m), 7.45-7.65 (2H, m), 7.70-7.94 (1H, br), 8.90 (2H, s), 9.23 (2H, s), 11.23 (1H, br-s)
MS(FAB, m/z): 463 (M+H)

### Example 6

### 2-[N-(3-Amidinophenyl)-N-(4-hydroxybutyl)amino]-N-[4-(4-piperidinyl)phenyl]acetamide dihydrochloride (Compound 44) 2-[N-(3-Amidinophenyl)-N-(4-benzyloxybutyl)amino]-N-[4-(4-piperidinyl)phenyl]acetamide dihydrochloride (Compound 45)

To 840 mg of 2-[N-(4-benzyloxybutyl)-N-(3-cyanophenyl)amino]-N-[4-(1-trifluoroacetylpiperidin-4-yl)phenyl]acetamide was added 50 ml of a saturated hydrogen chloride ethanol solution, and the mixture was sealed and allowed to react for 5 hours. The reaction mixture was concentrated under reduced pressure, and 50 ml of a saturated ammonia methanol solution was added to the resulting residue. After stirring for 12 hours, the reaction mixture was concentrated under reduced pressure, and the resulting residue was separated by octadesyl-bounded silica gel medium pressure liquid column chromatography (eluent: water-methanol). The former desired fraction was acidified with dilute hydrochloric acid, and the solvent was removed under reduced pressure to give 350 mg of the Compound 44. The latter one was acidified with dilute hydrochloric acid, and the solvent was removed under reduced pressure to give 330 mg of the Compound 45.

### Compound 44

¹H-NMR(DMSO-d₆) δ ppm;
   1.40-1.55 (2H, m), 1.60-1.70 (2H, m), 1.75-1.92 (4H, m), 2.70-2.85 (1H, m), 2.90-3.05 (2H, m), 3.28-3.50 (6H, m), 4.25 (2H, s), 6.90-7.07 (3H, m), 7.15 (2H, d, J=8.6Hz), 7.30-7.40 (1H, m), 7.56 (2H, d, J=8.6Hz), 8.65-9.00 (4H, m), 9.23 (2H, s), 10.20 (1H, s)

### Compound 45

¹H-NMR(DMSO-d₆) δ ppm:
   1.55-1.72 (4H, m), 1.73-1.95 (4H, m), 2.72-2.85 (1H, m), 2.90-3.03 (2H, m), 3.28-3.40 (2H, m), 3.41-3.52 (4H, m), 4.26 (2H, s), 4.46 (2H, s), 6.91-7.08 (3H, m), 7.15 (2H, d, J=8.6Hz), 7.21-7.40 (6H, m), 7.56 (2H, d, J=8.6Hz), 8.72-9.08 (4H, m), 9.28 (2H, s), 10.24 (1H, s)

### Example 7

### 2-[N-(3-Amidinophenyl)-N-(3-(2-hydroxyethyloxy)benzyl]amino]-N-[4-[1-(1-iminoethyl)piperidin-4-yl]phenyl]acetamide dihydrochloride (Compound 46)

2-[N-(3-Amidinophenyl)-N-[3-(2-hydroxyethyloxy)benzyl]amino]-N-[4-(4-piperidinyl)phenyl]acetamide dihydrochloride (130 mg) was dissolved in 2 ml of methanol, 58 mg of ethyl acetimidate hydrochloride and 0.126 ml of triethylamine were added to the solution, and the mixture was stirred at room temperature for 2 hours. The reaction mixture was concentrated under reduced pressure, and the resulting residue was separated by octadesyl-bounded silica gel medium pressure liquid column chromatography (eluent: water-methanol). The desired fraction obtained was acidified with dilute hydrochloric acid, and the solvent was removed under reduced pressure to give 115 mg of 2-[N-(3-amidinophenyl)-N-[3-(2-hydroxyethyloxy)benzyl]amino]-N-(4-[1-(1-iminoethyl)piperidin-4-yl]phenyl]acetamide dihydrochloride.
¹H-NMR(DMSO-d₆) δ ppm:
   1.55-1.92 (4H, m), 2.31 (3H, s), 2.79-2.91 (1H, m), 3.10-3.23 (1H, m), 3.63-3.72 (2H, m), 3.94 (2H, t, J=5.0Hz), 3.96-4.06 (1H, m), 4.16-4.28 (1H, m), 4.38 (2H, s), 4.72 (2H, s), 4.84 (1H, t, J=5.5Hz), 6.75-7.40 (10H, m), 7.56 (2H, d, J=8.6Hz), 8.67 (1H, br-s), 8.98 (2H, br-s), 9.15-9.45 (3H, m), 10.30 (1H, s)
MS(FAB, m/z): 543(M+H)

### Example 8

The following compounds were prepared using the same procedure as Example 7.

### 2-[N-(3-Amidinophenyl)-N-(3-carbamoylmethyloxybenzyl)amino]-N-[4-[1-(1-iminoethyl)piperidin-4-yl]phenyl]acetamide dihydrochloride (Compound 47)

¹H-NMR(DMSO-d₆) δ ppm:
   1.54-1.93 (4H, m), 2.31 (3H, s), 2.79-2.91 (1H, m), 3.09-3.35 (2H, m), 3.93-4.05 (1H, m), 4.18-4.31 (1H, m), 4.32-4.46 (4H, m), 4.73 (2H, s), 6.79- 7.40 (11H, m), 7.47-7.63 (3H, m), 8.72 (1H, s), 9.05 (2H, s), 9.22-9.40 (3H, m), 10.39 (1H, s)
MS(FAB, m/z): 556(M+H)

### 2-[N-(3-Amidinophenyl)-N-(3-methoxycarbonylmethyloxybenzyl)amino]-N-[4-[1-(1-iminoethyl)piperidin-4-yl]phenyl]acetamide dihydrochloride (Compound 48)

¹H-NMR(DMSO-d₆) δ ppm:
   1.54-1.94 (4H, m), 2.30 (3H, s), 2.79-2.92 (1H, m), 3.09-3.35 (2H, m), 3.66 (3H, s), 3.95-4.06 (1H, m), 4.17-4.28 (1H, m), 4.37 (2H, s), 4.67-4.79 (4H, m), 6.75-7.40 (10H, m), 7.56 (2H, d, J=8.5 Hz), 8.66 (1H, s), 8.96 (2H, s), 9.21 (1H, s), 9.28 (2H, s), 10.27 (1H, s)
MS(FAB, m/z): 570(M+H)

### 2-[N-(3-Amidinophenyl)-N-(5-quinoxalinylmethyl)amino]-N-[4-[1-(1-iminoethyl)piperidin-4-yl]phenyl]acetamide dihydrochloride (Compound 49)

¹H-NMR(DMSO-d₆) δ ppm:
   1.51-1.93 (4H, m), 2.30 (3H, s), 2.75-2.92 (1H, m), 3.92-4.16 (1H, m), 4.24-4.29 (1H, m), 4.48 (2H, s), 5.39 (2H, s), 6.90-7.24 (5H, m), 7.28-7.38 (1H, m), 7.49-7.72 (3H, m), 7.76-7.87 (1H, m), 7.97-8.08 (1H, m), 8.63 (1H, s), 8.84-9.06 (4H, m), 9.13-9.31 (3H, m), 10.28 (1H, s)

### 2-[N-(3-Amidinophenyl)-N-(3-chlorobenzyl)amino]-N-[4-[1-(1-iminoethyl)piperidin-4-yl]phenyl]acetamide dihydrochloride (Compound 50)

¹H-NMR(DMSO-d₆) δ ppm:
   1.55-1.95 (4H, m), 2.30 (3H, s), 2.80-2.90 (1H, m), 3.10-3.25 (1H, m), 3.95-4.05 (1H, m), 4.15-4.25 (1H, m), 4.39 (2H, s), 4.77 (2H, s), 6.94-6.96 (1H, m), 7.02-7.13 (2H, m), 7.20 (2H, d, J=8.6Hz), 7.25-7.42 (5H, m), 7.55 (2H, d, J=8.6Hz), 8.63 (1H, br-s), 8.93 (2H, br-s), 9.18 (1H, br-s), 9.28 (2H, br-s), 10.26 (1H, s)

### 2-[N-(3-Amidinophenyl)-N-(4-hydroxybutyl)amino]-N-[4-[1-(1-iminoethyl)piperidin-4-yl]phenyl]acetamide dihydrochloride (Compound 51)

¹H-NMR(DMSO-d₆) δ ppm:
   1.44-1.52 (2H, m), 1.58-1.80 (4H, m), 1.81-1.92 (2H, m), 2.30 (3H, s), 2.80-2.90 (1H, m), 3.10-3.22 (1H, m), 3.24-3.34 (1H, m), 3.40-3.50 (2H, m), 4.18-4.30 (3H, m), 6.91-7.08 (3H, m), 7.19 (2H, d, J=8.6Hz), 7.30-7.40 (1H, m), 7.55 (2H, d, J=8.6Hz), 8.66 (1H, s), 8.99 (2H, s), 9.22 (1H, s), 9.29 (2H, s), 10.25 (1H, s)

### 2-[N-(3-Amidinophenyl)-N-(3-hydroxybenzyl)amino]-N-[4-[1-(1-iminoethyl)piperidin-4-yl]phenyl]acetamide dihydrochloride (Compound 52)

¹H-NMR(DMSO-d₆) δ ppm:
   1.55-1.94 (4H, m), 2.30 (3H, s), 2.79-2.92 (1H, m), 3.11-3.32 (2H, m), 3.95-4.05 (1H, m), 4.15-4.28 (1H, m), 4.34 (2H, s), 4.66 (2H, s), 6.60-6.76 (3H, m), 6.95 (1H, dd, J=8.5Hz, 2.2Hz), 7.01-7.25 (5H, m), 7.30-7.39 (1H, m), 7.56 (2H, d, J=8.6Hz), 8.64 (1H, s), 8.94 (2H, s), 9.20 (1H, s), 9.28 (2H, s), 9.38 (1H, br-s) 10.24 (1H, s)

### 2-[N-(3-Amidinophenyl)-N-cyclopropylmethylaminol-N-[4-[1-(1-iminoethyl)piperidin-4-yl]phenyl]acetamide dihydrochloride (Compound 53)

¹H-NMR(DMSO-d₆) δ ppm:
   0.25-0.35 (2H, m), 0.45-0.55 (2H, m), 1.05-1.17 (1H, m), 1.55-1.92 (4H, m), 2.31 (3H, s), 2.80-2.90 (1H, m), 3.10-3.40 (4H, m), 3.95-4.05 (1H, m), 4.18-4.31 (1H, m), 4.36 (2H, s), 6.98-7.08 (2H, m), 7.11-7.22 (3H, m), 7.30-7.40 (1H, m), 7.55 (2H, d, J=8.6Hz), 8.50-9.50 (6H, m), 10.26 (1H, s)

### 2-[N-(3-Amidinophenyl)-N-benzylaminol)-N-[4-[1-(1-iminoethyl)piperidin-4-yl]phenyl]acetamide dihydrochloride (Compound 54)

¹H-NMR(DMSO-d₆) δ ppm:
   1.55-1.92 (4H, m), 2.31 (3H, s), 2.80-2.92 (1H, m), 3.10-3.22 (1H, m), 3.95-4.07 (1H, m), 4.16-4.28 (1H, m), 4.37 (2H, s), 4.76 (2H, s), 6.90-7.42 (11H, m), 7.56 (2H, d, J=8.4Hz), 8.67 (1H, br-s), 8.98 (2H, br-s), 9.15-9.40 (3H, m), 10.29 (1H, s)

### 2-[N-(3-Amidinophenyl)-N-(3-methyl-2-butenyl)amino]-N-[4-[1-(1-iminoethyl)piperidin-4-yl]phenyl]acetamide dihydrochloride (Compound 55)

¹H-NMR(DMSO-d₆) δ ppm:
   1.50-1.95 (10H, m), 2.30 (3H, s), 2.77-2.91 (1H, m), 3.10-3.35 (2H, m), 3.94-4.12 (3H, m), 4.16-4.30 (3H, m), 5.20-5.30 (1H, m), 6.96 (1H, dd, J=8.6Hz, 2.2Hz), 7.00-7.15 (2H, m), 7.19 (2H, d, J=8.6Hz), 7.30-7.40 (1H, m), 7.55 (2H, d, J=8.6Hz), 8.70 (1H, br-s), 9.04 (2H, br-s), 9.20-9.38 (3H, m), 10.27 (1H, br-s)

### 2-(5-Amidino-2-hydroxymethylphenylamino)-N-[4-[1-(1-iminoethyl)piperidin-4-yloxy]phenyl]acetamide dihydrochloride (Compound 56)

¹H-NMR(DMSO-d₆) δ ppm:
   1.62-1.83 (2H, m), 1.95-2.10 (2H, m), 2.28 (3H, s), 3.40-3.59 (2H, m), 3.67-3.83 (2H, m), 4.05 (2H, s), 4.50-4.68 (3H, m), 6.89-7.11 (4H, m), 7.36 (1H, d, J=7.8Hz), 7.54 (2H, d, J=9.0Hz), 8.67 (1H, s), 8.91 (2H, s), 9.18-9.35 (3H, m), 10.14 (1H, s)
MS(FAB, m/z): 439(M+H)

### 2-(5-Amidino-2-benzyloxyphenylamino)-N-[4-[1-(1-iminoethyl)piperidin-4-yloxy]phenyl]acetamide dihydrochloride (Compound 57)

¹H-NMR(DMSO-d₆) δ ppm:
   1.62-1.82 (2H, m), 1.95-2.10 (2H, m), 2.29 (3H, s), 3.45-3.60 (2H, m), 4.06 (2H, s), 4.57-4.69 (1H, m), 5.30 (2H, s), 6.95 (2H, d, J=9.1Hz), 7.02 (1H, s), 7.08-7.20 (2H, m), 7.30-7.60 (7H, m), 8.73 (1H, s), 8.85 (2H, s), 9.16 (2H, s), 9.58 (1H, s), 10.25 (1H, s)

### 2-[N-(3-Amidinophenyl)-N-benzylaminol-N-[4-[1-(1-imino-ethyl)piperidin-4-yloxy]phenyl]acetamide dihydrochloride (Compound 58)

¹H-NMR(DMSO-d₆) δ ppm:
   1.65-1.82 (2H, m), 1.95-2.12 (2H, m), 2.28 (3H, s), 3.46-3.57 (2H, m), 3.68-3.80 (2H, m), 4.31 (2H, s), 4.57-4.68 (1H, m), 4.75 (2H, s), 6.91-7.09 (5H, m), 7.22-7.38 (6H, m), 7.51 (2H, d, J=8.7Hz), 8.54-8.89 (3H, m), 9.10-9.26 (3H, m), 10.04 (1H, s)

### 2-(5-Amidino-2-methylphenylamino)-N-[4-[1-(1-iminoethyl)piperidin-4-yloxy]phenyl]acetamide dihydrochloride (Compound 59)

¹H-NMR(DMSO-d₆) δ ppm:
   1.65-1.80 (2H, m), 1.95-2.10 (2H, m), 2.24 (3H, s), 2.29 (3H, s), 3.45-3.61 (2H, m), 3.66-3.86 (2H, m), 4.07 (2H, s), 4.55-4.70 (1H, m), 6.88-7.07 (4H, m), 7.22 (1H, d, J=7.6Hz), 7.56 (2H, d, J=9.0Hz), 8.75 (1H, s), 9.00 (2H, s), 9.25 (2H, s), 9.31 (1H, s), 10.27 (1H, s)

### 2-(5-Amidino-2-chlorophenylamino)-N-[4-[1-(1-iminoethyl)piperidin-4-yloxy]phenyl]acetamide dihydrochloride (Compound 60)

¹H-NMR(DMSO-d₆) δ ppm:
   1.65-1.82 (2H, m), 1.95-2.10 (2H, m), 2.29 (3H, s), 3.65-3.85 (2H, m), 4.13 (2H, s), 4.58-4.69 (1H, m), 6.00-6.22 (1H, br), 6.95 (2H, d, J=9.0Hz), 7.00-7.14 (2H, m), 7.47-7.60 (3H, m), 8.70 (1H, s), 9.07 (2H, s), 9.25 (1H, s), 9.37 (2H, s), 10.26 (1H, s)

### Example 9

### 2-[N-(3-Amidinophenyl)-N-(3-methoxycarbonylmethyloxybenzyl)amino]-N-(4-isopropylphenyl)acetamide hydrochloride (Compound 61)

To 202 mg of 2-[N-(3-cyanophenyl)-N-(3-methoxycarbonylmethyloxybenzyl)amino]-N-(4-isopropylphenyl)acetamide was added 50 ml of a saturated hydrogen chloride methanol solution, and the mixture was sealed and stirred at room temperature for 5 hours. The reaction mixture was concentrated under reduced pressure, and the concentrate was neutralized with a saturated aqueous sodium hydrogen carbonate solution and extracted with ethyl acetate. The organic layer was dried over anhydrous sodium sulfate, and the solvent was removed under reduced pressure. Methanol (50 ml) and a solution of 57 mg of ammonium chloride in 2 ml of water were added to the resulting residue, and the mixture was heated under reflux for 12 hours. The reaction mixture was concentrated under reduced pressure, and the resulting residue was separated by aminopropylated silica gel column chromatography (eluent: methanol-dichloromethane). The desired fraction obtained was acidified with dilute hydrochloric acid, and the solvent was removed under reduced pressure to give 113 mg of 2-[N-(3-amidinophenyl)-N-(3-methoxycarbonylmethyloxybenzyl)amino]-N-(4-isopropylphenyl)acetamide hydrochloride.
¹H-NMR(DMSO-d₆) δ ppm:
   1.18 (6H, d, J=6.9Hz), 2.78-2.89 (1H, m), 3.67 (3H, s), 4.36 (2H, s), 4.73 (2H, s), 4.76 (2H, s), 6.77-7.40 (10H, m), 7.52 (2H, d, J=8.5Hz), 8.89 (2H, s), 9.26 (2H, s), 10.13 (1H, s)

### Example 10

### 2-[N-(3-Amidinophenyl)-N-(3-carboxymethyloxybenzyl)amino]-N-[4-[1-(1-iminoethyl)piperidin-4-yl]phenyl]acetamide dihydrochloride (Compound 62)

To 71 mg of 2-[N-(3-amidinophenyl)-N-(3-methoxycarbonylmethyloxybenzyl)amino]-N-[4-[1-(1-iminoethyl)piperidin-4-yl]phenyl]acetamide dihydrochloride were added 1 ml of 1 N hydrochloric acid and 1 ml of acetonitrile, and the mixture was allowed to react at 60 °C for 4 hours. The reaction mixture was concentrated under reduced pressure, and the resulting residue was separated by octadesyl-bounded silica gel medium pressure liquid column chromatography (eluent: water-acetonitrile). The desired fraction obtained was acidified with dilute hydrochloric acid, and the solvent was removed under reduced pressure to give 58 mg of 2-[N-(3-amidinophenyl)-N-(3-carboxymethyloxybenzyl)amino]-N-[4-[1-(1-iminoethyl)piperidin-4-yl]phenyl]acetamide dihydrochloride.
¹H-NMR(DMSO-d₆) δ ppm:
   1.52-1.93 (4H, m), 2.31 (3H, s), 2.77-2.92 (1H, m), 3.05-3.35 (2H, m), 3.94-4.07 (1H, m), 4.20-4.35 (1H, m), 4.41 (2H, s), 4.63 (2H, s), 4.73 (2H, s), 6.72-7.40 (10H, m), 7.59 (2H, d, J=8.6Hz), 8.80 (1H, s), 9.14 (2H, s), 9.25-9.50 (3H, m), 10.48 (1H, s)
MS(FAB, m/z): 557(M+H)

### Example 11

The following compound was prepared using the same procedure as Example 10.

### 2-[N-(3-Amidinophenyl)-N-(3-carboxymethyloxybenzyl)amino]-N-(4-isopropylphenyl)acetamide dihydrochloride (Compound 63)

¹H-NMR(DMSO-d₆) δ ppm:
   1.17 (6H, d, J=6.9Hz), 2.78-2.89 (1H, m), 4.35 (2H, s), 4.63 (2H, s), 4.72 (2H, s), 6.73-7.60 (12H, m), 8.89 (2H, br-s), 9.25 (2H, br-s), 10.11 (1H, br-s), 12.65-13.35 (1H, br)

### Example 12

### Ethyl 3-[2-[N-(3-amidinophenyl)-N-[N-(4-isopropylphenyl)carbamoylmethyl]aminomethyl]phenyl]acrylate hydrochloride (Compound 64)

2-[N-(2-Bromobenzyl)-N-(3-cyanophenyl)amino]-N-(4-isopropylphenyl)acetamide (800 mg), 10 mg of palladium acetate, 24 mg of triphenylphosphine, 0.55 ml of ethyl acrylate and 0.35 ml of triethylamine were suspended in 1.5 ml of N,N-dimethylformamide, and the suspension was allowed to react under an argon atmosphere at 100 °C for 18 hours. The insoluble material was removed by filtration, and the filtrate was concentrated under reduced pressure. The resulting residue was purified by silica gel column chromatography (eluent: ethyl acetate-hexane) to give 738 mg of a nitrile compound. To 360 mg of the nitrile compound was added 10 ml of a saturated hydrogen chloride ethanol solution, and the mixture was sealed and allowed to react at room temperature for 8 hours. The reaction mixture was concentrated under reduced pressure, and 10 ml of a saturated ammonia methanol solution was added to the resulting residue. After allowing to react at room temperature for 19 hours, the reaction mixture was concentrated under reduced pressure. The resulting residue was separated by aminopropylated silica gel column chromatography (eluent: 25 % aqueous ammonia solution-methanol-dichloromethane), and the desired fraction obtained was concentrated under reduced pressure. Methanol was added to the resulting residue, and the mixture was acidified with dilute hydrochloric acid. The solvent was removed under reduced pressure to give 230 mg of ethyl 3-[2-[N-(3-amidinophenyl)-N-[N-(4-isopropylphenyl)carbamoylmethyl]aminomethyl]phenyl]acrylate hydrochloride.
¹H-NMR(DMSO-d₆) δ ppm:
   1.17 (6H, d, J=6.9Hz), 1.24 (3H, t, J=7.1Hz), 2.76-2.90 (1H, m), 4.18 (2H, q, J=7.1Hz), 4.29 (2H, s), 4.94 (2H, s), 6.54 (1H, d, J=15.8Hz), 6.92-7.41 (9H, m), 7.50 (2H, d, J=8.5Hz), 7.75-7.84 (1H, m), 7.95 (1H, d, J=15.8Hz), 8.89 (2H, s), 9.25 (2H, s), 10.10 (1H, s)

### Example 13

### 2-[N-(3-Amidinophenyl)-N-(1-naphthylmethyl)amino]-N-[4-[1-(1-iminoethyl)piperidin-4-yl]phenyl]acetamide dihydrochloride (Compound 65)

To 670 mg of 2-[N-(3-cyanophenyl)-N-(1-naphthylmethyl)amino]-N-[4-(1-trifluoroacetylpiperidin-4-yl)phenyl]acetamide was added 25 ml of a saturated hydrogen chloride ethanol solution, and the mixture was sealed and allowed to react at room temperature for 5 hours. The reaction mixture was concentrated under reduced pressure, and 30 ml of a saturated ammonia methanol solution was added to the resulting residue. After being allowed to react for 15 hours, the reaction mixture was concentrated under reduced pressure. The resulting residue was purified by aminopropylated silica gel column chromatography (eluent: 25 % aqueous ammonia solution-methanol-dichloromethane) to give an amidine compound. The amidine compound obtained was dissolved in 4 ml of methanol, 377 mg of ethyl acetimidate hydrochloride and 0.425 ml of triethylamine were added to the solution, and the mixture was stirred at room temperature for 2 hours. The reaction mixture was concentrated under reduced pressure, and the resulting residue was separated by octadesyl-bounded silica gel medium pressure liquid column chromatography (eluent: water-methanol). The desired fraction obtained was acidified with dilute hydrochloric acid, and the solvent was removed under reduced pressure to give 275 mg of 2-[N-(3-amidinophenyl)-N-(1-naphthylmethyl)amino]-N-[4-[1-(1-iminoethylpiperidin-4-yl]phenyl]acetamide dihydrochloride.
¹H-NMR(DMSO-d₆) δ ppm:
   1.55-1.96 (4H, m), 2.31 (3H, s), 2.78-2.93 (1H, m), 3.10-3.36 (2H, m), 3.95-4.06 (1H, m), 4.16-4.28 (1H, m), 4.38 (2H, s), 5.24 (2H, s), 6.95-7.25 (5H, m), 7.29-7.64 (7H, m), 7.82-7.93 (1H, m), 7.96-8.10 (2H, m), 8.65 (1H, br-s), 8.94 (2H, br-s), 9.13-9.33 (3H, m), 10.21 (1H, br-s)

### Example 14

### 2-[N-(3-Amidinophenyl)-N-(5-methylimidazol-4-ylmethyl)amino]-N-(4-isopropylphenyl)acetamide hydrochloride (Compound 66)

To 1.20 g of 2-[N-(1-tert-butoxycarbonyl-5-methylimidazol-4-ylmethyl)-N-(3-cyanophenyl)amino]-N-(4-isopropylphenyl)acetamide was added 20 ml of a saturated hydrogen chloride ethanol solution, and the mixture was sealed and allowed to react at room temperature for 5 hours. The reaction mixture was concentrated under reduced pressure, and the resulting residue was suspended in diethyl ether. After the supernatant was decanted, the precipitates were dried under reduced pressure. A saturated ammonia methanol solution (25 ml) and 10 ml of dichloromethane were added to the resulting residue, and the mixture was allowed to react at room temperature for 24 hours. The reaction mixture was concentrated under reduced pressure, and the resulting residue was suspended in chloroform and a small amount of ethanol. The insoluble material was removed by filtration, and the filtrate was concentrated under reduced pressure. Methanol was added to the resulting residue, and the mixture was suspended in ethyl acetate. The insoluble material was collected by filtration, methanol was added to the insoluble material, and the mixture was suspended in ethyl acetate again. The insoluble material was collected by filtration and washed with ethyl acetate to give 900 mg of 2-[N-(3-amidinophenyl)-N-(5-methylimidazol-4-ylmethyl)amino]-N-(4-isopropylphenyl)acetamide hydrochloride.
¹H-NMR(DMSO-d₆) δ ppm:
   1.17 (6H, d, J=6.9Hz), 2.28 (3H, s), 2.74-2.90 (1H, m), 4.32 (2H, s), 4.67 (2H, s), 6.87-7.08 (3H, m), 7.16 (2H, d, J=8.4Hz), 7.29-7.41 (1H, m), 7.56 (2H, d, J=8.4Hz), 7.87-8.14 (1H, br), 8.95 (2H, s), 9.27 (2H, s), 11.15-11.65 (1H, br)

### Example 15

The following compounds were prepared using the same procedure as Example 14.

### 2-[N-(3-Amidinophenyl)-N-[1-(4-methylbenzyl)imidazol-4-yl-methyl]amino]-N-(4-isopropylphenyl)acetamide hydrochloride (Compound 67)

¹H-NMR(DMSO-d₆) δ ppm:
   1.17 (6H, d, J=6.9Hz), 2.28 (3H, s), 2.76-2.90 (1H, m), 4.31 (2H, s), 4.67 (2H, s), 5.16 (2H, s), 6.90-7.20 (9H, m), 7.23-7.38 (2H, m), 7.56 (2H, d, J=8.5Hz), 7.90-8.10 (1H, br), 8.95 (2H, s), 9.24 (2H, s), 11.28 (1H, br-s)

### 2-[N-(3-Amidinophenyl)-N-[1-(4-hydroxybenzyl)imidazol-4-ylmethyl]amino]-N-(4-isopropylphenyl)acetamide hydrochloride (Compound 68)

¹H-NMR(DMSO-d₆) δ ppm:
   1.16 (6H, d, J=6.9Hz), 2.75-2.89 (1H, m) 4.29 (2H, s), 4.63 (2H, s), 5.04 (2H, s), 6.73 (2H, d, J=8.4Hz), 6.85-7.36 (9H, m), 7.56 (2H, d, J=8.4Hz), 7.84 (1H, s), 8.70-9.70 (5H, m), 11.40 (1H, s)
MS(FAB, m/z): 497(M+H)

### Example 16

### 2-[N-(3-Amidinophenyl)-N-(4-hydroxybutyl)amino]-N-(4-isopropylphenyl)acetamide hydrochloride (Compound 69)

To 25 mg of 2-[N-(3-amidinophenyl)-N-(4-benzyloxybutyl)amino]-N-(4-isopropylphenyl)acetamide dihydrochloride were added 1.0 ml of ethanol and 5 mg of 10 % palladium on carbon, and the mixture was stirred at atmospheric pressure under a hydrogen atmosphere at room temperature for 2 hours. The insoluble material was removed by filtration, and the filtrate was concentrated under reduced pressure to give 18 mg of 2-[N-(3-amidinophenyl)-N-(4-hydroxybutyl)amino]-N-(4-isopropylphenyl)acetamide hydrochloride.
¹H-NMR(DMSO-d₆) δ ppm:
   1.17 (6H, d, J=6.9Hz), 1.41-1.52 (2H, m), 1.56-1.68 (2H, m), 2.78-2.90 (1H, m), 3.40-3.50 (4H, m), 4.23 (2H, s), 6.92-7.05 (3H, m), 7.16 (2H, d, J=8.5Hz), 7.30-7.38 (1H, m), 7.50 (2H, d, J=8.5Hz), 8.91 (2H, s), 9.25 (2H, s), 10.08 (1H, s)

### Example 17

The following compounds were prepared using the same procedure as Example 16.

### 2-[N-(3-Amidinophenyl)-N-(2-methylpropyl)amino]-N-(4-isopropoxyphenyl)acetamide hydrochloride (Compound 70)

¹H-NMR(DMSO-d₆) δ ppm:
   0.92 (6H, d, J=6.6Hz), 1.22 (6H, d, J=6.0Hz), 1.96-2.10 (1H, m), 3.28 (2H, d, J=7.6Hz), 4.23 (2H, s), 4.47-4.60 (1H, m), 6.84 (2H, d, J=9.0Hz), 6.92-7.01 (3H, m), 7.30-7.40 (1H, m), 7.45 (2H, d, J=9.0Hz), 8.81 (2H, s), 9.22 (2H, s), 9.94 (1H, s)

### 2-[N-(3-Amidinophenyl)-N-(3-methylbutyl)amino]-N-(4-isopropoxyphenyl)acetamide dihydrochloride (Compound 71)

¹H-NMR(DMSO-d₆) δ ppm:
   0.94 (6H, d, J=6.6Hz), 1.23 (6H, d, J=6.0Hz), 1.40-1.70 (3H, m), 3.36-3.50 (2H, m), 4.19 (2H, s), 4.45-4.59 (1H, m), 6.85 (2H, d, J=9.0Hz), 6.90-7.02 (3H, m), 7.30-7.40 (1H, m), 7.42 (2H, d, J=9.0Hz), 8.85 (2H, s), 9.24 (2H, s), 9.97 (1H, s)

### 2-[N-(3-Amidinophenyl)-N-propylamino]-N-(4-isopropoxyphenyl))acetamide dihydrochloride (Compound 72)

¹H-NMR(DMSO-d₆) δ ppm:
   0.91 (3H, t, J=7.4Hz), 1.23 (6H, d, J=6.0Hz), 1.50-1.70 (2H, m), 3.35-3.50 (2H, m), 4.21 (2H, s), 4.46-4.60 (1H, m), 6.84 (2H, d, J=9.0Hz), 6.90-7.06 (3H, m), 7.30-7.40 (1H, m), 7.48 (2H, d, J=9.0Hz), 8.89 (2H, s), 9.25 (2H, s), 9.99 (1H, s)

### 2-(5-Amidino-2-hydroxyphenylamino)-N-[4-[1-(1-iminoethyl)piperidin-4-yloxy]phenyl]acetamide dihydrochloride (Compound 73)

¹H-NMR(DMSO-d₆) δ ppm:
   1.62-1.85 (2H, m), 1.95-2.10 (2H, m), 2.29 (3H, s), 3.65-3.85 (2H, m), 4.01 (2H, s), 4.57-4.69 (1H, m), 5.20-5.70 (1H, m), 6.87 (1H, d, J=8.2Hz), 6.90-7.10 (4H, m), 7.55 (2H, d, J=9.0Hz), 8.63-8.80 (3H, m), 9.05 (2H, s), 9.28 (1H, s), 10.21 (1H, s), 10.75 (1H, s)

### Example 18

### 2-[N-(3-Amidinophenyl)-N-(5-methylimidazol-4-ylmethyl)amino]-N-[4-[1-(1-iminoethyl)piperidin-4-yl]phenyl]acetamide dihydrochloride (Compound 74)

To 3 ml of ethanol were added 138 mg of 2-[N-(3-amidinophenyl)-N-(5-methylimidazol-4-ylmethyl)amino]-N-[4-(4-piperidinyl)phenyl]acetamide trihydrochloride, 43 mg of ethyl acetimidate hydrochloride and 96 µl of triethylamine, and the mixture was allowed to react at room temperature for 18 hours. The reaction mixture was concentrated under reduced pressure, and to the resulting residue was added dichloromethane. The insoluble material was collected by filtration, a small amount of methanol was added to the insoluble material, and the mixture was suspended in ethyl acetate. The insoluble material was collected by filtration, and suspended with a small amount of ethanol and isopropanol. The insoluble material was removed by filtration, and the filtrate was concentrated under reduced pressure. To the resulting residue were added a small amount of methanol and diethyl ether, and the insoluble material was collected by filtration. To the resulting residue was added a small amount of ethanol, the mixture was suspended in diethyl ether, and the insoluble material was collected by filtration to give 105 mg of 2-[N-(3-amidinophenyl)-N-(5-methylimidazol-4-ylmethyl)amino]-N-[4-[1-(1-iminoethyl)piperidin-4-yl]phenyl]acetamide dihydrochloride.
¹H-NMR(DMSO-d₆) δ ppm:
   1.52-1.93 (4H, m), 2.20-2.36 (6H, m), 2.77-2.91 (1H, m), 3.07-3.12 (1H, m), 3.93-4.07 (1H, m), 4.14-4.38 (3H, m), 4.62 (2H, s), 6.80-7.07 (3H, m), 7.19 (2H, d, J=8.6Hz), 7.28-7.40 (1H, m), 7.50-7.77 (3H, m), 8.63 (1H, s), 8.94 (2H, br-s), 9.12-9.31 (3H, m), 11.70-12.22 (2H, m)

### Example 19

### Sodium salt of 2-[N-(3-amidinophenyl)-N-(1-carboxymethylimidazol-4-ylmethyl)amino]-N-(4-isopropylphenyl)acetamide (Compound 75)

2-[N-(3-Amidinophenyl)-N-(1-methoxycarbonylmethylimidazol-4-ylmethyl)amino]-N-(4-isopropylphenyl)acetamide hydrochloride (11 mg) was dissolved in 0.4 ml of methanol, 77 µl of 1 N sodium hydroxide methanol solution was added to the solution, and the mixture was heated under reflux for 2 hours. The reaction mixture was suspended in diethyl ether, and the supernatant was decanted. The resulting residue was dried under reduced pressure and dissolved in ethanol, and the remaining insoluble material was removed through Celite®. The filtrate was concentrated under reduced pressure, and the resulting residue was suspended in diethyl ether and a small amount of methanol. The supernatant was decanted and the residue was dried under reduced pressure to give 10 mg of sodium salt of 2-[N-(3-amidinophenyl)-N-(1-carboxymethylimidazol-4-ylmethyl)amino]-N-(4-isopropylphenyl)acetamide.
¹H-NMR(DMSO-d₆) δ ppm:
   1.16 (6H, d, J=6.9Hz), 2.73-2.90 (1H, m), 4.18 (2H, s), 4.22 (2H, s), 4.62 (2H, s), 6.60-7.20 (7H, m), 7.57-7.65 (3H, m), 11.92-12.01 (1H, br)
MS(FAB, m/z): 471(M+H)

### Example 20

### 2-[N-(3-Amidinophenyl)-N-(3-benzyloxybenzyl)amino]-N-(4-isopropoxyphenyl)acetamide hydrochloride (Compound 76)

### 2-[N-(3-Amidinophenyl)-N-(3-hydroxybenzyl)amino]-N-(4-isopropoxyphenyl)acetamide hydrochloride (Compound 77)

To 40 mg of 2-[N-(3-benzyloxybenzyl)-N-(3-cyanophenyl)amino]-N-(4-isopropoxyphenyl)acetamide was added 20 ml of a saturated hydrogen chloride ethanol solution, and the mixture was sealed and allowed to react at room temperature for 5 hours. The reaction mixture was concentrated under reduced pressure, 20 ml of a saturated ammonia methanol solution was added to the residue, and the mixture was stirred at room temperature for 12 hours. The solvent was removed under reduced pressure, and the resulting residue was separated by aminopropylated silica gel column chromatography (eluent: methanol-dichloromethane). Methanol was added to the former desired fraction, and the mixture was acidified with dilute hydrochloric acid and concentrated under reduced pressure to give 20 mg of the Compound 76. To the latter one was added methanol, and the mixture was acidified with dilute hydrochloric acid and concentrated under reduced pressure to give 15 mg of the Compound 77.

### Compound 76

¹H-NMR(DMSO-d₆) δ ppm:
   1.23 (6H, d, J=5.9Hz), 4.31 (2H, s), 4.45-4.85 (3H, m), 5.05 (2H, s), 6.80-7.55 (17H, m), 8.85 (2H, s), 9.24 (2H, s), 10.00 (1H, s)

### Compound 77

¹H-NMR(DMSO-d₆) δ ppm:
   1.23 (6H, d, J=5.9Hz), 4.31 (2H, s), 4.45-4.80 (3H, m), 6.80-7.55 (13H, m), 8.81 (2H, s), 9.22 (2H, s), 9.96 (1H, s)

### Example 21

### 2-(3-Amidinophenylamino)-N-(4-isopropoxyphenyl)acetamide hydrochloride (Compound 78)

2-[N-(3-Amidinophenyl)-N-benzylamino)-N-(4-isopropoxyphenyl)acetamide hydrochloride (50 mg) was dissolved in 3 ml of ethanol, 5 mg of 10 % palladium on carbon was added to the solution, and the mixture was stirred at atmospheric pressure under a hydrogen atmosphere at room temperature for 22 hours. The insoluble material was removed through Celite®, and the filtrate was concentrated under reduced pressure. The resulting residue was separated by aminopropylated silica gel column chromatography (eluent: methanol-dichloromethane). Methanol was added to the desired fraction obtained, and the mixture was acidified with dilute hydrochloric acid. The solvent was removed under reduced pressure to give 7 mg of 2-(3-amidinophenylamino)-N-(4-isopropoxyphenyl)acetamide hydrochloride.
¹H-NMR(DMSO-d₆) δ ppm:
   1.25 (6H, d, J=5.9Hz), 3.92 (2H, s), 4.45-4.55 (1H, m), 6.78 (2H, d, J=8.9Hz), 6.84-6.91 (3H, m), 7.18-7.28 (1H, m), 7.41 (2H, d, J=8.9Hz), 8.75 (2H, s), 9.10 (2H, s), 9.86 (1H, s)

### Example 22

### 2-(3-Amidinophenylamino)-N-[4-[1-(1-iminoethyl)piperidin-4-yloxy]phenyl]acetamide dihydrochloride (Compound 79)

2-(3-Amidinophenylamino)-N-[4-(4-piperidinyloxy)phenyl]acetamide dihydrochloride (70 mg) and 0.088 ml of triethylamine were dissolved in 5 ml of ethanol, 40 mg of ethyl acetimidate hydrochloride was added to the solution, and the mixture was stirred at room temperature overnight. The reaction mixture was concentrated under reduced pressure, and the resulting residue was washed with chloroform and dissolved in methanol. The solution was acidified with dilute hydrochloric acid, and the insoluble material was removed by filtration. The filtrate was concentrated under reduced pressure, and the resulting residue was washed with isopropanol to give 25 mg of 2-(3-amidinophenylamino)-N-[4-[1-(1-iminoethyl)piperidin-4-yloxy]phenyl]acetamide dihydrochloride.
¹H-NMR(DMSO-d₆) δ ppm:
   1.65-1.71 (2H, m), 1.96-2.09 (2H, m), 2.28 (3H, s), 3.46-3.57 (2H, m), 3.68-3.81 (2H, m), 3.96 (2H, d, J=6.1Hz), 4.58-4.68 (1H, m), 6.48-6.53 (1H, m), 6.91-7.00 (5H, m), 7.27-7.37 (1H, m), 7.54 (2H, d, J=9.1Hz), 8.60-9.35 (6H, m), 10.13 (1H, br-s)

### Example 23

The following compound was prepared using the same procedure as Example 10 except acidification with dilute hydrochloric acid.

### 2-[N-(3-Amidinophenyl)-N-(3-carboxymethyloxybenzyl)amino]-N-[4-[1-(1-iminoethyl)piperidin-4-yl]phenyl]acetamide hydrochloride (Compound 80)

¹H-NMR(DMSO-d₆) δ ppm:
   1.40-1.95 (4H, m), 2.30 (3H, s), 2.83 (1H, t, J=11.6Hz), 3.79-4.49 (6H, m), 4.69 (2H, br-s), 6.56-6.85 (3H, m), 6.87-7.40 (7H, m), 7.56 (2H, d, J=8.4Hz), 8.40-11.35 (6H, m)

### Example 24

### 2-[N-(3-Amidinophenyl)-N-(3-carboxymethyloxybenzyl)amino]-N-[4-[1-(1-iminoethyl)piperidin-4-yl]phenyl]acetamide sulfate dihydrate (Compound 81)

2-[N-(3-Amidinophenyl)-N-(3-carboxymethyloxybenzyl)amino]-N-[4-[1-(1-iminoethyl)piperidin-4-yl]phenyl]acetamide hydrochloride (4.4 g) was dissolved in 50 ml of 1 N aqueous sulfuric acid solution at 50 °C. After stirring at room temperature for 6 hours, the resulting precipitates were collected by filtration and washed successively with water and acetone to give 3.9 g of 2-[N-(3-amidinophenyl)-N-(3-carboxymethyloxybenzyl)amino]-N-[4-[1-(1-iminoethyl)piperidin-4-yl]phenyl]acetamide sulfate dihydrate.
¹H-NMR(DCOOD) δ ppm:
   1.69-1.99 (2H, m), 2.00-2.22 (2H, m), 2.48 (3H, s), 2.90-3.12 (1H, m), 3.28-3.60 (2H, m), 4.10-4.35 (2H, m), 4.79 (2H, s), 4.82 (2H, s), 5.00 (2H, s), 6.87-7.08 (3H, m), 7.31 (2H, d, J=8.5Hz), 7.32-7.43 (1H, m), 7.46 (2H, d, J=8.5Hz), 7.50-7.85 (4H, m)
Analysis Calcd for C₃₁H₃₆N₆O₄·H₂SO₄·2H₂O: C,53.90; H,6.13; N,12.17
Found: C,53.78; H,6.03; N,12.14

### Test Example 1

### Measurement of inhibitory activity for activated blood coagulation factor X

Five µl of dimethylsulfoxide solution of test compound, 375 µl of tris-hydrochloric acid buffer (pH 8.4) and 100 µl of 1 mM S-2222 (Daiichi Pure Chemicals) aqueous solution were mixed. Then 20 µl of 0.6 U/ml human activated blood coagulation factor X (Calbiochem Corporation) in gelatin-glycine buffer was added and the mixture was incubated for 10 minutes at 37 °C. The reaction was terminated with the addition of 100 µl of 60 % acetic acid and absorbance (405 nm) was measured.

The group without the test compound was defined as control and the group without human activated blood coagulation factor X was defined as blank. The concentration of the test compound that showed 50 % inhibition of control (IC₅₀) was obtained and this value was used as the index of inhibitory activity for activated blood coagulation factor X. Results are shown in Table 1.

### Test Example 2

### Measurement of inhibitory activity for thrombin

Five µl of dimethylsulfoxide solution of test compound, 375 µl of tris-hydrochloric acid buffer (pH 8.4) and 100 µl of 1 mM S-2238 (Daiichi Pure Chemicals) aqueous solution were mixed. Then 20 µl of 2.0 U/ml human thrombin (Sigma Chemical Company) in gelatin-glycine buffer was added and the mixture was incubated for 10 minutes at 37 °C. The reaction was terminated with the addition of 100 µl of 60 % acetic acid and absorbance (405 nm) was measured.

The group without the test compound was defined as control and the group without human thrombin was defined as blank. The concentration of the test compound that showed 50 % inhibition of control (IC₅₀) was obtained and this value was used as the index of inhibitory activity for thrombin. Results are shown in Table 1.

**Table 1**

| Compound | Inhibitory activity for activated blood coagulation factor X (nM) | Inhibitory activity for thrombin (µM) |
|---|---|---|
| 22 | 15 | 13 |
| 52 | 16 | 77 |
| 55 | 4 | 15 |
| 58 | 42 | 100 |
| 62 | 18 | 206 |
| 65 | 8 | 42 |
| 74 | 19 | 177 |
| 79 | 96 | 308 |

### Test Example 3

### Measurement of anticoagulation effects (plasma prothrombin time)

Two µl of dimethylsulfoxide solution of test compound which was put in a process tube was incubated at 37 °C, and 50 µl of normal human plasma (George King Bio-Medical Inc.) was mixed. One minute later, 100 µl of plasma prothrombin time reagent (Neoplastin Plus®, Boehringer Mannheim) was added into the mixture. Prothrombin time was measured with ST4 (Boehringer Mannheim).

The group without the test compound was defined as control. The concentration of test compound that prolonged the clotting time of control by 2 times (CT₂) was obtained and this value was used as the index of anticoagulation activity. Results are shown in Table 2.

**Table 2**

| Compound | Anticoagulation effect (µM) |
|---|---|
| 22 | 0.29 |
| 52 | 0.17 |
| 55 | 0.091 |
| 58 | 0.24 |
| 62 | 0.21 |
| 74 | 0.30 |

### Test Example 4

### Acute toxicity test

Male Wistar rats aged 7 weeks (SLC) were divided into several groups consisting of 5 rats. The test compound was dissolved at the concentration that became the administration volume as 2.5 ml/kg. The solution was administered through the tail vein at an infusion rate of 1 ml/minute. Observations were performed at constant intervals, and survival rate was judged for 24 hours. Results are shown in Table 3.

**Table 3**

| Compound | Administration dose (mg/kg) | Death case |
|---|---|---|
| 62 | 30 | 0/5 |

## Claims

1. A 3-amidinoaniline derivative represented by the general formula: 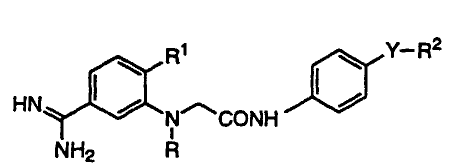 wherein R represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carbamoyl-substituted lower alkoxy group, a carboxy group, a lower alkoxycarbonyl group, a lower alkylsulfonyl group, a sulfamoyl group and a halogen atom, a group represented by the general formula: 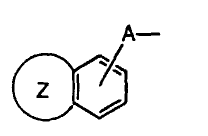 wherein A represents a lower alkylene group; and the ring Z fused with the benzene ring represents a 6-membered aromatic heterocyclic group which contains one or two nitrogen atoms in the ring; or a group represented by the general formula: 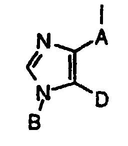 wherein A represents a lower alkylene group; B represents a hydrogen atom, a lower alkyl group, a carboxy-substituted lower alkyl group, a lower alkoxycarbonyl-substituted lower alkyl group, a carbamoyl-substituted lower alkyl group or an aryl-substituted lower alkyl group which may have a hydroxy group or a lower alkyl group as a substituent; and D represents a hydrogen atom or a lower alkyl group; R¹ represents a hydrogen atom, a hydroxy group, a lower alkyl group, a hydroxy-substituted lower alkyl group, a lower alkoxy group which may have an aryl group as a substituent or a halogen atom; Y represents a single bond or an oxygen atom; and R² represents a lower alkyl group or a group represented by the general formula: 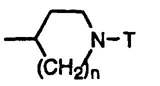 wherein n represents 1 or 2; and T represents a hydrogen atom or a group represented by the general formula:
-C(=NH)-W
wherein W represents a lower alkyl group; or a pharmaceutically acceptable salt thereof.

2. A 3-amidinoaniline derivative as claimed in claim 1, represented by the general formula: 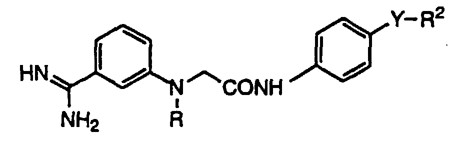 wherein R represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carbamoyl-substituted lower alkoxy group, a carboxy group, a lower alkoxycarbonyl group, a lower alkylsulfonyl group, a sulfamoyl group and a halogen atom, a group represented by the general formula: 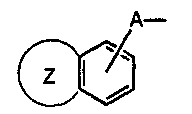 wherein A represents a lower alkylene group; and the ring Z fused with the benzene ring represents a 6-membered aromatic heterocyclic group which contains one or two nitrogen atoms in the ring; or a group represented by the general formula: 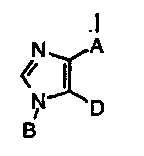 wherein A represents a lower alkylene group; B represents a hydrogen atom, a lower alkyl group, a carboxy-substituted lower alkyl group, a lower alkoxycarbonyl-substituted lower alkyl group, a carbamoyl-substituted lower alkyl group or an aryl-substituted lower alkyl group which may have a hydroxy group or a lower alkyl group as a substituent; and D represents a hydrogen atom or a lower alkyl group ; Y represents a single bond or an oxygen atom; and R² represents a lower alkyl group or a group represented by the general formula: 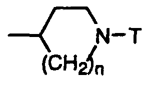 wherein n represents 1 or 2; and T represents a hydrogen atom or a group represented by the general formula:
-C(=NH)-W
wherein W represents a lower alkyl group; or a pharmaceutically acceptable salt thereof.

3. A 3-amidinoaniline derivative as claimed in claim 2, represented by the general formula: 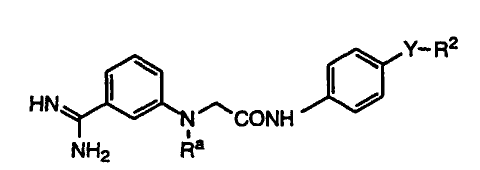 wherein R^{a} represents a lower alkenyl group, an aryl-substituted lower alkyl group which may have from one to three substituents selected from the group consisting of a hydroxy group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxycarbonyl-substituted lower alkenyl group, a carbamoyl-substituted lower alkoxy group, a carboxy group, a lower alkoxycarbonyl group, a lower alkylsulfonyl group, a sulfamoyl group and a halogen atom, a group represented by the general formula: 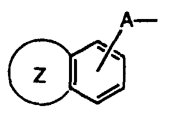 wherein A represents a lower alkylene group; and the ring Z fused with the benzene ring represents a 6-membered aromatic heterocyclic group which contains one or two nitrogen atoms in the ring; or a group represented by the general formula: 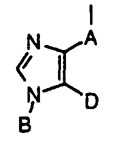 wherein A represents a lower alkylene group; B represents a hydrogen atom, a lower alkyl group, a carboxy-substituted lower alkyl group, a lower alkoxycarbonyl-substituted lower alkyl group, a carbamoyl-substituted lower alkyl group or an aryl-substituted lower alkyl group which may have a hydroxy group or a lower alkyl group as a substituent; and D represents a hydrogen atom or a lower alkyl group ; Y represents a single bond or an oxygen atom; and R² represents a lower alkyl group or a group represented by the general formula: 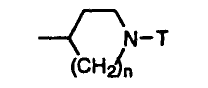 wherein n represents 1 or 2; and T represents a hydrogen atom or a group represented by the general formula:
-C(=NH)-W
wherein W represents a lower alkyl group; or a pharmaceutically acceptable salt thereof.

4. The 3-amidinoaniline derivative as claimed in claim 3, represented by the general formula: 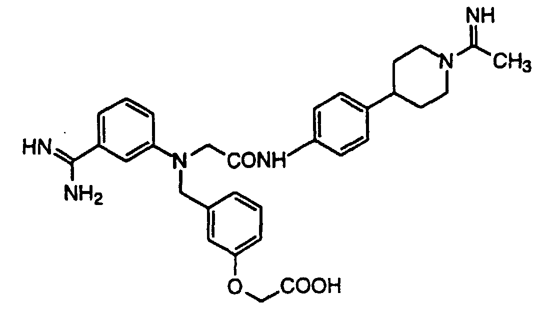 or pharmaceutically acceptable salt thereof.

5. A pharmaceutical composition comprising the 3-amidinoaniline derivative or pharmaceutically acceptable salt thereof as claimed in claims 1, 2, 3 or 4.

6. An activated blood coagulation factor X inhibitor which comprises, as an active ingredient, the 3-amidinoaniline derivative or pharmaceutically acceptable salt thereof as claimed in claims 1, 2, 3 or 4.

7. A method for the prevention or treatment of thromboembolic diseases which comprises administering the 3-amidinoaniline derivative or pharmaceutically acceptable salt thereof as claimed in claims 1, 2, 3 or 4.

8. A use of the 3-amidinoaniline derivative or pharmaceutically acceptable salt thereof as claimed in claims 1, 2, 3 or 4 for the manufacture of a pharmaceutical composition for the prevention or treatment of thromboembolic diseases.

9. A process for the manufacture of a pharmaceutical composition for the prevention or treatment of thromboembolic diseases, characterized in the use, as an essential constituent of said pharmaceutical composition, of the 3-amidinoaniline derivative or pharmaceutically acceptable salt thereof as claimed in claims 1, 2, 3 or 4.

10. A 3-cyanoaniline derivative represented by the general formula: 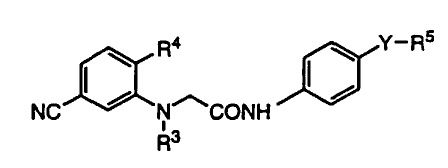 wherein R³ represents a hydrogen atom, a lower alkyl group, a lower alkenyl group, a cycloalkyl-substituted lower alkyl group, a hydroxy-substituted lower alkyl group, a lower alkoxy-substituted lower alkyl group which may have an aryl group as a substituent, a lower alkoxy-substituted lower alkenyl group which may have an aryl group as a substituent, an aryl-substituted lower alkyl group which may have from one to three substituents selected from the group consisting of a hydroxy group which may have a protective group, a lower alkyl group, a lower alkoxy group, a hydroxy-substituted lower alkoxy group, an aryl-substituted lower alkoxy group, a carboxy-substituted lower alkoxy group, a lower alkoxycarbonyl-substituted lower alkoxy group, a carboxy-substituted lower alkenyl group, a lower alkoxy-carbonyl-substituted lower alkenyl group, a carboxy group, a lower alkoxycarbonyl group, a lower alkylsulfonyl group, a sulfamoyl group and a halogen atom, a group represented by the general formula: 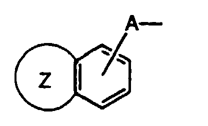 wherein A represents a lower alkylene group; and the ring Z fused with the benzene ring represents a 6-membered aromatic heterocyclic group which contains one or two nitrogen atoms in the ring or a group represented by the general formula: 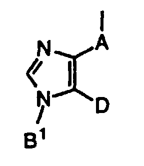 wherein A represents a lower alkylene group; B¹ represents a hydrogen atom, an amino-protective group, a lower alkyl group, a carboxy-substituted lower alkyl group, a lower alkoxy-carbonyl-substituted lower alkyl group or an aryl-substituted lower alkyl group which may have a hydroxy group or a lower alkyl group as a substituent; and D represents a hydrogen atom or a lower alkyl group; R⁴ represents a hydrogen atom, a lower alkyl group, a protected hydroxy-substituted lower alkyl group, a lower alkoxy group which may have an aryl group as a substituent or a halogen atom; Y represents a single bond or an oxygen atom; and R⁵ represents a lower alkyl group or a group represented by the general formula: 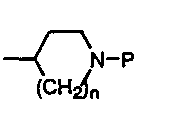 wherein n represents 1 or 2; and P represents a hydrogen atom or an amino-protective group ; or a salt thereof.
